# EUROPEAN PATENT APPLICATION

(11) **EP 4 818 158 A1**
(43) Date of publication of application: **07.10.2026**
(21) Application number: 23963233.4
(22) Date of filing: 29.12.2023
(51) Int. Cl.: C07D 213/40, A61K 31/50, A61K 31/4418, A61P 5/14, A61P 5/16, A61P 27/02, C07D 237/08

(54) **BIARYL COMPOUND**

(71) Applicant: Kissei Pharmaceutical Co., Ltd., Matsumoto-Shi, Nagano 399-8710 (JP)
(72) Inventor: MUTAI, Yosuke, Azumino-shi, Nagano 399-8304 (JP); MATSUMOTO, Kyohei, Azumino-shi, Nagano 399-8304 (JP); HIRASAWA, Hideaki, Azumino-shi, Nagano 399-8304 (JP); YOSHIDA, Masako, Azumino-shi, Nagano 399-8304 (JP); OKUTOMI, Naoto, Azumino-shi, Nagano 399-8304 (JP); NISHIMURA, Toshihiro, Azumino-shi, Nagano 399-8304 (JP); OHNO, Kohsuke, Azumino-shi, Nagano 399-8304 (JP); KIYOSAWA, Seina, Azumino-shi, Nagano 399-8304 (JP); ISHIKAWA, Takehiro, Azumino-shi, Nagano 399-8304 (JP)
(74) Representative: Isarpatent
(86) International application number: PCT/JP2023/047363
(87) International publication number: WO 2025/141891

(57) **Abstract**

An object of the present invention is to provide a novel compound which has thyroid-stimulating hormone receptor antagonist activity and is useful for treating thyroid-related diseases.

The present invention relates to a biaryl compound represented by the following formula (I) or a pharmacologically acceptable salt thereof. The compound of the present invention or the pharmacologically acceptable salt thereof has antagonist activity against thyroid-stimulating hormone receptors and is useful as a therapeutic agent for thyroid-related diseases (for example, hyperthyroidism, Graves' disease, thyroid eye disease, and thyroid cancer).

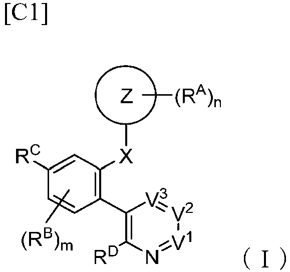

## Description

### [Technical Field]

The present invention relates to a biaryl compound useful as pharmaceuticals. In more detail, the present invention relates to a biaryl compound or a pharmacologically acceptable salt thereof that has antagonist activity against thyroid-stimulating hormone receptors (TSHRs) and is useful as a therapeutic agent for thyroid-related diseases.

### [Background Art]

Triiodothyronine (T3) and thyroxine (T4), which are thyroid hormones, play an important role in development, growth, and metabolism, and the synthesis and secretion thereof are tightly regulated by thyroid-stimulating hormones (TSHs) secreted from pituitary gland.

In hyperthyroidism, these thyroid hormones are excessively secreted for some reasons, and due to hormone actions thereof, various undesirable effects, such as goiter, tachycardia, hypertension, fatigue, weight loss, palpitation, sleep disorder, and menstrual irregularities come to appear in mind and body.

There are various causes of hyperthyroidism; however, among these, the highest percentage is Graves' disease (Basedow's disease). In Graves' disease, thyroid gland is recognized as a foreign body by autoimmune mechanisms, and autoantibodies against TSHR present on thyroid follicular cells, so-called TSHR antibodies (TRAb), are produced. It is considered that this TRAb acts as an agonist on TSHR, and TSHR is excessively stimulated, whereby more of thyroid hormones than necessary are produced, and hyperthyroidism develops.

In addition, as a disease associated with Graves' disease, thyroid eye disease is also known. Thyroid eye disease is an autoimmune inflammatory disease exhibiting various ocular symptoms and is considered to be mainly caused by the agonist action of TRAb on TSHR in orbital tissues. Thyroid eye disease often develops at roughly the same time as hyperthyroidism, but develops without thyroid dysfunction in some cases.

Currently, in drug therapy for Graves' disease, antithyroid drugs that inhibit biosynthesis of thyroid hormones such as thiamazole and propylthiouracil are in use, but there are problems of a low remission rate, a long treatment period taken until remission, a high frequency of side effects, and the like. Therefore, currently, there is a demand for a therapeutic agent for Graves' disease having a new action mechanism.

Blocking TSHR or inhibiting signal transduction induced through TSHR inhibits thyroid hormone secretion and thyroid cell proliferation. Therefore, TSHR antagonists are considered to have a therapeutic effect on Graves' disease and thyroid eye disease caused by the agonist action of TRAb on TSHR (PTL 1 and 2). NCGC00242364 is known as a TSHR antagonist and has been clarified to have a blood T4 concentration lowering effect in TSH-releasing hormone (TRH)-treated mice and thyroid stimulating antibody M22-treated mice (NPL 1).

Compounds having TSHR antagonist activity are described in PTL 1 to 3, and NPL 1. PTL 4 and 5 describe or exemplify compounds containing biaryl.

However, a biaryl compound of the present invention is described in none of PTL 1 to 5 and NPL 1.

### [Citation List]

### [Patent Literature]

[PTL 1] U.S. Patent Application Publication No. 2011/0172267
[PTL 2] U.S. Patent Application Publication No. 2012/0315217
[PTL 3] U.S. Patent Application Publication No. 2019/0134024
[PTL 4] U.S. Patent Application Publication No. 2014/0193906
[PTL 5] WO 2010/036316

### [Non Patent Literature]

[NPL 1] Susanne Neumann et al., "Endocrinology" 2014, Vol. 155, No. 1, pp. 310 to 314

### [Summary of Invention]

### [Technical Problem]

An object of the present invention is to provide a novel compound that has TSHR antagonist activity and is useful for the treatment of thyroid-related diseases.

### [Solution to Problem]

The present invention relates to a compound represented by the following formula (I) or a pharmacologically acceptable salt thereof.

That is, the present invention relates to [1] to [10] below and the like.
[1] A compound represented by formula (I): 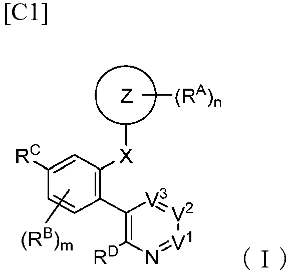 [wherein
   a ring Z is a C₆₋₁₀ aryl, a 5- or 6-membered heteroaryl, a C₃₋₈ cycloalkyl, or a 3- to 8-membered heterocycloalkyl;
   V¹ is =CR^{v1}-, or =N-;
   V² is =CR^{v2}-, or =N-; and
   V³ is =CR^{v3}-, or =N-;
   wherein R^{v1}, R^{v2}, and R^{v3} are each independently a hydrogen atom, a C₁₋₆ alkyl, or a hydroxy C₁₋₆ alkyl;
   X is -CR^{x}R^{x'}-, -O-, or -C(=O)-;
   R^{x} and R^{x'} are each independently a hydrogen atom or a C₁₋₆ alkyl;
      m and n are each independently an integer of 0 to 2;
   R^{A} is a halogen atom, a cyano group, a hydroxyl group, a C₁₋₆ alkyl, a halo C₁₋₆ alkyl, a C₁₋₆ alkoxy, a halo C₁₋₆ alkoxy, a C₆₋₁₀ aryl C₁₋₆ alkoxy, or a C₁₋₆ alkylsulfonyl;
   in a case where n is 2, the individual R^{A}'s are the same as or different from each other;
      R^{B} is a halogen atom or a C₁₋₆ alkyl;
   in a case where m is 2, the individual R^{B}'s are the same as or different from each other;
   R^{C} is -NHC(O)OR^{c1}, -NHC(O)R^{c2}, -NHC(O)NR^{c2}R^{c2'}, or -NHC(O)NR^{c2}(OR^{c2'}));
   R^{c1} is a group selected from the group consisting of the following (a) to (g):
      (a) a C₁₋₆ alkyl unsubstituted or substituted with 1 to 6 groups selected from a substituent group A,
      (b) a C₃₋₈ cycloalkyl unsubstituted or substituted with 1 to 6 groups selected from the substituent group A,
      (c) a C₃₋₈ cycloalkyl C₁₋₆ alkyl unsubstituted or substituted with 1 to 6 groups selected from the substituent group A,
      (d) a C₆₋₁₀ aryl C₁₋₆ alkyl unsubstituted or substituted with 1 to 3 groups selected from the substituent group A,
      (e) a 3- to 8-membered heterocycloalkyl unsubstituted or substituted with 1 to 3 groups selected from the substituent group A,
      (f) a 3- to 8-membered heterocycloalkyl C₁₋₆ alkyl unsubstituted or substituted with 1 to 3 groups selected from the substituent group A, and
      (g) a 5- or 6-membered heteroaryl C₁₋₆ alkyl unsubstituted or substituted with 1 to 3 groups selected from the substituent group A;
   the substituent group A is the group consisting of a halogen atom, a cyano group, a hydroxyl group, -N(C₁₋₆ alkyl)₂, a C₁₋₆ alkyl, a halo C₁₋₆ alkyl, a C₁₋₆ alkoxy, a C₆₋₁₀ aryl C₁₋₆ alkoxy, and a C₃₋₈ cycloalkyl;
   in the case of being substituted with 2 or more groups selected from the substituent group A, the individual groups may be the same as or different from each other;
   R^{c2} and R^{c2'} are each independently a group selected from the group consisting of the following (a) to (f):
      (a) a hydrogen atom,
      (b) a C₁₋₆ alkyl unsubstituted or substituted with 1 to 3 groups selected from a substituent group B,
      (c) a C₃₋₈ cycloalkyl unsubstituted or substituted with 1 to 3 groups selected from the substituent group B,
      (d) a C₃₋₈ cycloalkyl C₁₋₆ alkyl unsubstituted or substituted with 1 to 3 groups selected from the substituent group B,
      (e) a 3- to 8-membered heterocycloalkyl unsubstituted or substituted with 1 to 3 groups selected from the substituent group B, and
      (f) the following group unsubstituted or substituted with 1 to 3 groups selected from the substituent group B: 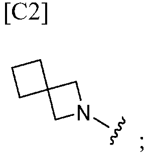
   the substituent group B is the group consisting of a halogen atom, a cyano group, a C₁₋₆ alkyl, a halo C₁₋₆ alkyl, a C₁₋₆ alkoxy, and a C₃₋₈ cycloalkyl;
   in the case of being substituted with 2 or 3 groups selected from the substituent group B, the individual groups may be the same or different from each other;
   R^{D} is a group selected from the group consisting of the following (a) to (e):
      (a) a C₁₋₆ alkyl,
      (b) a hydroxy C₁₋₆ alkyl, 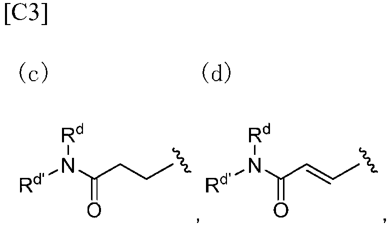 and 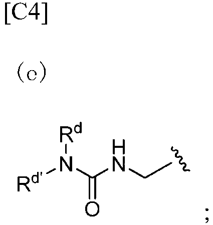 and
   R^{d} and R^{d'} are each independently a hydrogen atom or a C₁₋₆ alkyl], or
   a pharmacologically acceptable salt thereof.
[2] The compound according to the above [1], wherein
   V² is =CR^{v2}-;
   R^{v1}, R^{v2}, and R^{v3} are each independently a hydrogen atom or a C₁₋₆ alkyl;
   R^{A} is a halogen atom, a cyano group, a hydroxyl group, a C₁₋₆ alkyl, a halo C₁₋₆ alkyl, a C₁₋₆ alkoxy, a C₆₋₁₀ aryl C₁₋₆ alkoxy, or a C₁₋₆ alkylsulfonyl;
   R^{c1} is a group selected from the group consisting of the following (a) to (f):
      (a) a C₁₋₆ alkyl unsubstituted or substituted with 1 to 6 groups selected from the substituent group A,
      (b) a C₃₋₈ cycloalkyl unsubstituted or substituted with 1 to 6 groups selected from the substituent group A,
      (c) a C₃₋₈ cycloalkyl C₁₋₆ alkyl unsubstituted or substituted with 1 to 6 groups selected from the substituent group A,
      (d) a C₆₋₁₀ aryl C₁₋₆ alkyl unsubstituted or substituted with 1 to 3 groups selected from the substituent group A,
      (e) a 3- to 8-membered heterocycloalkyl unsubstituted or substituted with 1 to 3 groups selected from the substituent group A, and
      (f) a 3- to 8-membered heterocycloalkyl C₁₋₆ alkyl unsubstituted or substituted with 1 to 3 groups selected from the substituent group A;
   the substituent group A has the same meaning as in the above [1];
   R^{c2} and R^{c2'} are each independently a group selected from the group consisting of the following (a) to (e):
      (a) a hydrogen atom,
      (b) a C₁₋₆ alkyl unsubstituted or substituted with 1 to 3 groups selected from a substituent group B,
      (c) a C₃₋₈ cycloalkyl unsubstituted or substituted with 1 to 3 groups selected from the substituent group B,
      (d) a 3- to 8-membered heterocycloalkyl unsubstituted or substituted with 1 to 3 groups selected from the substituent group B, and
      (e) the following group unsubstituted or substituted with 1 to 3 groups selected from the substituent group B: 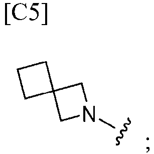
   the substituent group B is the group consisting of a halogen atom and a C₁₋₆ alkyl; and
   R^{d} and R^{d'} are hydrogen atoms, or a pharmacologically acceptable salt thereof.
[3] The compound according to the above [1] or [2], wherein
   the ring Z is a C₆₋₁₀ aryl or a 5- or 6-membered heteroaryl;
   V¹ is =CH- or =N-;
   V² and V³ are =CH-;
   R^{x} and R^{x'} are hydrogen atoms;
   m is 1;
   R^{B} is a halogen atom;
   R^{C} is -NHC(O)OR^{c1};
   R^{c1} is a group selected from the group consisting of the following (a) to (e):
      (a) a C₁₋₆ alkyl unsubstituted or substituted with 1 to 3 groups selected from the substituent group A,
      (b) a C₃₋₈ cycloalkyl unsubstituted or substituted with 1 to 3 groups selected from the substituent group A,
      (c) a C₃₋₈ cycloalkyl C₁₋₆ alkyl unsubstituted or substituted with 1 to 3 groups selected from the substituent group A,
      (d) a C₆₋₁₀ aryl C₁₋₆ alkyl unsubstituted or substituted with 1 to 3 groups selected from the substituent group A, and
      (e) a 3- to 8-membered heterocycloalkyl unsubstituted or substituted with 1 to 3 groups selected from the substituent group A;
   the substituent group A has the same meaning as in the above [1];
   R^{D} is a group represented by the following (a) or (b): 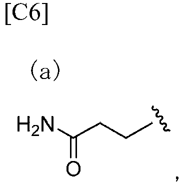 or 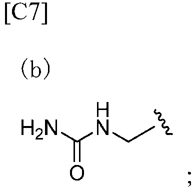 or a pharmacologically acceptable salt thereof.
[4] The compound according to any one of the above [1] to [3], wherein the compound is represented by
   formula (II): 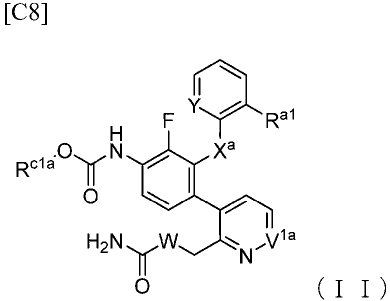 [wherein
   V^{1a} is =CH- or =N-;
   W is -CH₂- or -NH-;
   X^{a} is -CH₂-, -O-, or -C(=O)-;
   Y is =CR^{a2}- or =N-;
   R^{a1} and R^{a2} are each independently a hydrogen atom, a halogen atom, a cyano group, a hydroxyl group, a C₁₋₆ alkyl, a halo C₁₋₆ alkyl, a C₁₋₆ alkoxy, a C₆₋₁₀ aryl C₁₋₆ alkoxy, or a C₁₋₆ alkylsulfonyl;
   R^{c1a} is a group selected from the group consisting of the following (a) to (e):
      (a) a C₁₋₆ alkyl unsubstituted or substituted with 1 to 3 groups selected from a substituent group A^{a},
      (b) a C₃₋₈ cycloalkyl unsubstituted or substituted with 1 to 3 groups selected from the substituent group A^{a},
      (c) a C₃₋₈ cycloalkyl C₁₋₆ alkyl unsubstituted or substituted with 1 to 3 groups selected from the substituent group A^{a},
      (d) a C₆₋₁₀ aryl C₁₋₆ alkyl unsubstituted or substituted with 1 to 3 groups selected from the substituent group A^{a}, and
      (e) a 3- to 8-membered heterocycloalkyl unsubstituted or substituted with 1 to 3 groups selected from the substituent group A^{a};
   the substituent group A^{a} is a group consisting of a halogen atom, a cyano group, a hydroxyl group, -N(C₁₋₆ alkyl)₂, a C₁₋₆ alkyl, a halo C₁₋₆ alkyl, a C₁₋₆ alkoxy, a C₆₋₁₀ aryl C₁₋₆ alkoxy, and a C₃₋₈ cycloalkyl; and
   in the case of being substituted with 2 or 3 groups selected from the substituent group A^{a}, the individual groups may be the same or different from each other], or
   a pharmacologically acceptable salt thereof.
[5] The compound according to any one of the above [1] to [4], wherein the compound is represented by
   formula (III): 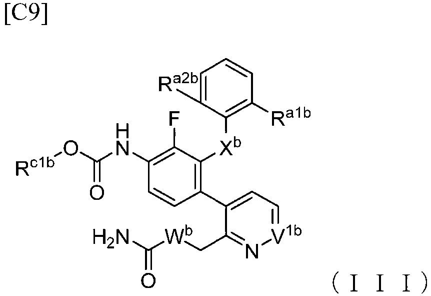 [wherein
   V^{1b} is =CH- or =N-;
   W^{b} is -CH₂- or -NH-;
   X^{b} is -CH₂- or -O-;
   R^{a1b} and R^{a2b} are each independently a hydrogen atom, a halogen atom, a cyano group, a hydroxyl group, a C₁₋₆ alkyl, a halo C₁₋₆ alkyl, a C₁₋₆ alkoxy, a C₆₋₁₀ aryl C₁₋₆ alkoxy, or a C₁₋₆ alkylsulfonyl;
   R^{c1b} is a group selected from the group consisting of the following (a) to (e):
      (a) a C₁₋₆ alkyl unsubstituted or substituted with 1 to 3 groups selected from a substituent group A^{b},
      (b) a C₃₋₈ cycloalkyl unsubstituted or substituted with 1 to 3 groups selected from the substituent group A^{b},
      (c) a C₃₋₈ cycloalkyl C₁₋₆ alkyl unsubstituted or substituted with 1 to 3 groups selected from the substituent group A^{b},
      (d) a C₆₋₁₀ aryl C₁₋₆ alkyl unsubstituted or substituted with 1 to 3 groups selected from the substituent group A^{b}, and
      (e) a 3- to 8-membered heterocycloalkyl unsubstituted or substituted with 1 to 3 groups selected from the substituent group A^{b};
   the substituent group A^{b} is a group consisting of a halogen atom, a cyano group, a hydroxyl group, -N(C₁₋₆ alkyl)₂, a C₁₋₆ alkyl, a halo C₁₋₆ alkyl, a C₁₋₆ alkoxy, a C₆₋₁₀ aryl C₁₋₆ alkoxy, and a C₃₋₈ cycloalkyl; and
   in the case of being substituted with 2 or 3 groups selected from the substituent group A^{b}, the individual groups may be the same or different from each other], or
   a pharmacologically acceptable salt thereof.
[6] The compound according to the above [5], wherein
   R^{a1b} and R^{a2b} are each independently a hydrogen atom, a halogen atom, or a cyano group;
   R^{c1b} is a group selected from the group consisting of the following (a) to (c):
      (a) a C₁₋₆ alkyl unsubstituted or substituted with 1 to 3 groups selected from the substituent group A^{b},
      (b) a C₃₋₈ cycloalkyl unsubstituted or substituted with 1 to 3 groups selected from the substituent group A^{b}, and
      (c) a C₃₋₈ cycloalkyl C₁₋₆ alkyl unsubstituted or substituted with 1 to 3 groups selected from the substituent group A^{b}; and
   the substituent group A^{b} is a halogen atom, a cyano group, a C₁₋₆ alkyl, or a halo C₁₋₆ alkyl, or a pharmacologically acceptable salt thereof.
[7] The compound according to any one of the above [1] to [6], wherein the compound is selected from the group consisting of the following compounds: 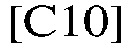 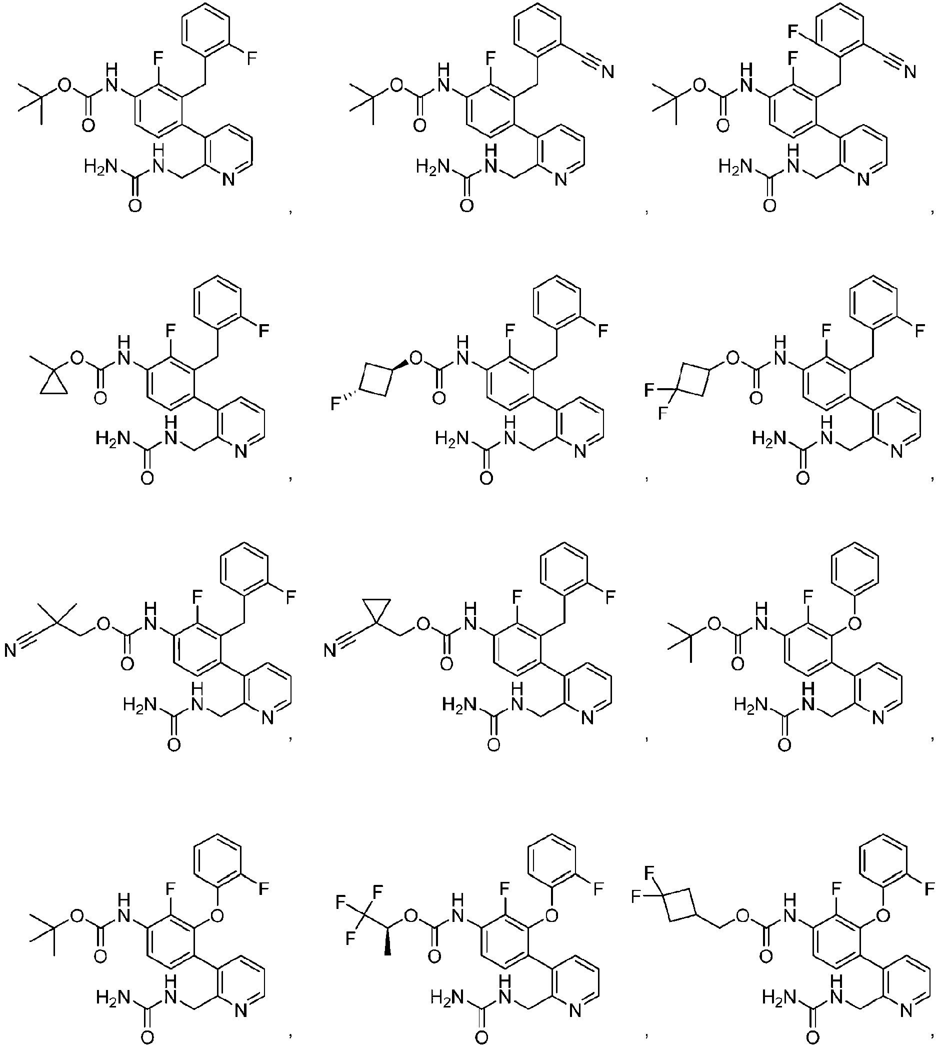 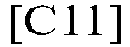 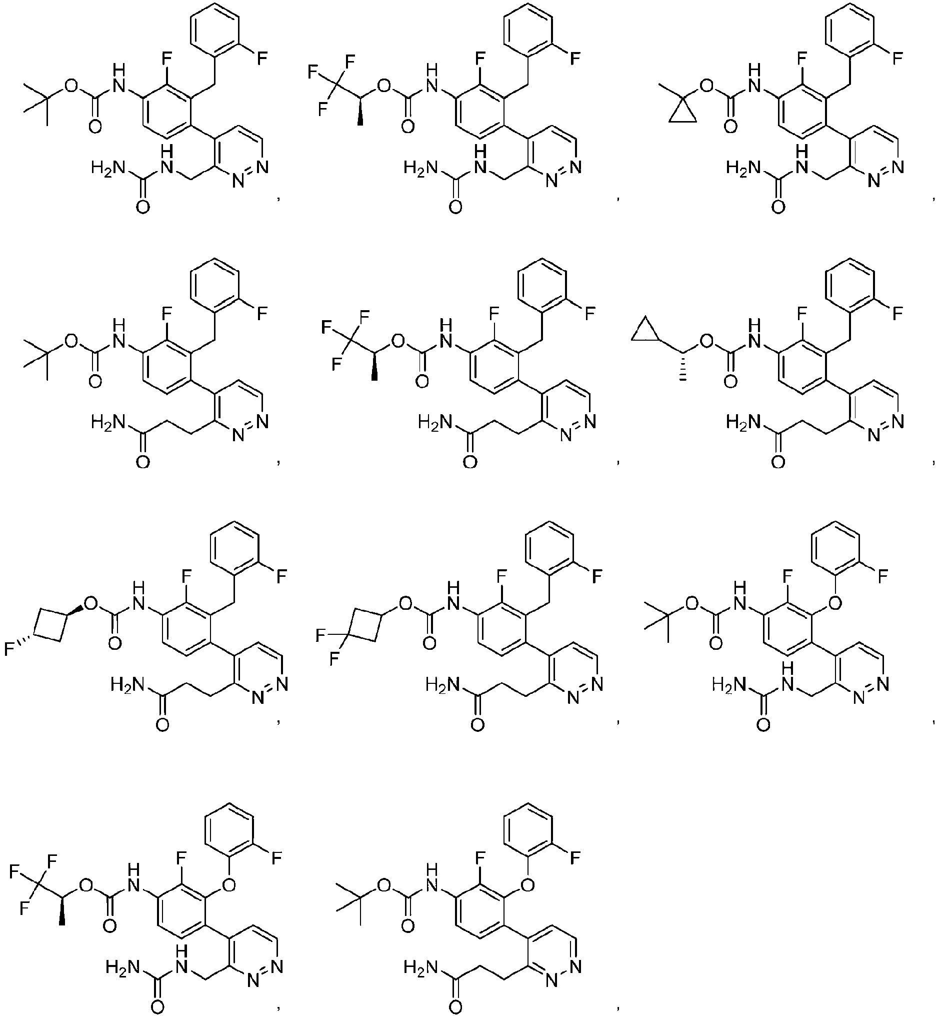 and 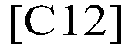 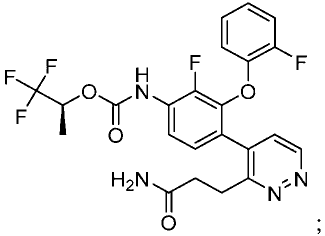 or a pharmacologically acceptable salt thereof.
[8] A pharmaceutical composition comprising the compound according to any one of the above [1] to [7] or a pharmacologically acceptable salt thereof, and a pharmaceutical additive.
[9] The pharmaceutical composition according to the above [8], wherein the composition is a pharmaceutical composition for treating thyroid-related diseases.
[10] The pharmaceutical composition according to the above [9], wherein the thyroid-related diseases are hyperthyroidism, Graves' disease, or thyroid eye disease.

As one embodiment, the present invention relates to a method for treating thyroid-related diseases including administering a necessary amount of the pharmaceutical composition according to the above [8] to a patient.

As one embodiment, the present invention relates to a use of the compound according to any one of the above [1] to [7] or a pharmacologically acceptable salt thereof for manufacturing a pharmaceutical composition for treating thyroid-related diseases.

### [Advantageous Effects of Invention]

The compound of the present invention has excellent TSHR antagonist activity. Therefore, the compound of the present invention or a pharmacologically acceptable salt thereof is useful as a therapeutic agent for thyroid-related diseases.

### [Description of Embodiments]

Hereinafter, embodiments of the present invention will be described in more detail.

In the present invention, unless particularly otherwise described, respective terms have the following meanings.

"Halogen atom" means a fluorine atom, a chlorine atom, a bromine atom, or an iodine atom.

"C₁₋₆ alkyl" means a linear or branched alkyl group having 1 to 6 carbon atoms. Examples thereof include methyl, ethyl, propyl, isopropyl, butyl, isobutyl, sec-butyl, tert-butyl, and the like.

"C₁₋₆ alkoxy" means a linear or branched alkoxy group having 1 to 6 carbon atoms. Examples thereof include methoxy, ethoxy, propoxy, isopropoxy, and the like.

"Halo C₁₋₆ alkyl" means a C₁₋₆ alkyl substituted with 1 to 5 identical or different halogen atoms. Examples thereof include monofluoromethyl, 2-fluoroethyl, difluoromethyl, trifluoromethyl, 1,1-difluoroethyl, 2,2,2-trifluoroethyl, 3,3,3-trifluoropropyl, 4,4,4-trifluorobutyl, pentafluoroethyl, and the like.

"Hydroxy C₁₋₆ alkyl" means a C₁₋₆ alkyl substituted with 1 or 2 hydroxyl groups. Examples thereof include hydroxymethyl, 1-hydroxyethyl, 2-hydroxyethyl, 3-hydroxypropyl, 2-hydroxypropan-2-yl, and the like.

"Halo C₁₋₆ alkoxy" means a C₁₋₆ alkoxy substituted with 1 to 5 identical or different halogen atoms. Examples thereof include monofluoromethoxy, difluoromethoxy, trifluoromethoxy, 1,1,2,2-tetrafluoroethoxy, pentafluoroethoxy, and the like.

"C₁₋₆ alkylsulfonyl" means a group represented by (C₁₋₆ alkyl)-SO₂-. Examples thereof include methylsulfonyl, ethylsulfonyl, and the like.

"C₆₋₁₀ aryl" means a phenyl group or a naphthyl group.

"5- or 6-membered heteroaryl" means a 5- or 6-membered aromatic heterocyclic group having any 1 to 4 heteroatoms selected from an oxygen atom, a nitrogen atom, and a sulfur atom in a ring. Examples thereof include pyridyl, furyl, pyrrolyl, thienyl, imidazolyl, pyrazolyl, 1,2,4-triazolyl, isothiazolyl, isoxazolyl, oxazolyl, thiazolyl, 1,3,4-oxadiazolyl, 1,2,4-oxadiazolyl, and the like.

"C₃₋₈ cycloalkyl" means a saturated hydrocarbon group of a 3- to 8-membered ring and also includes such saturated hydrocarbon groups having a partially crosslinked structure. Examples thereof include cyclopropyl, cyclobutyl, cyclopentyl, cyclohexyl, bicyclo[1.1.1]pentyl, bicyclo[2.2.2]octyl, and the like.

"3- to 8-membered heterocycloalkyl" means a C₃₋₈ cycloalkyl group in which carbon atoms in a ring have been substituted with 1 or 2 heteroatoms selected from an oxygen atom, a nitrogen atom, and a sulfur atom and also includes such cycloalkyl groups having a partially crosslinked structure. Examples thereof include aziridinyl, azetidinyl, pyrrolidinyl, piperidinyl, piperazinyl, morpholinyl, thiomorpholinyl, azepanyl, azabicyclo[2.2.2]octyl, 2-oxa-5-azabicyclo[2.2.1]heptanyl, 6-oxa-3-azabicyclo[2.2.1]heptanyl, 8-oxa-3-azabicyclo[3.2.1]octanyl, 3-oxa-8-azabicyclo[3.2.1]octanyl, 2-azaspiro[3.3]heptanyl, 2-oxa-6-azaspiro[3.3]heptanyl, pyrrolidonyl, piperidonyl, oxiranyl, oxetyl, tetrahydrofuranyl, tetrahydropyranyl, and the like. Preferable examples thereof include 1-pyrrolidinyl, 1-piperidinyl, and 4-morpholinyl.

"C₆₋₁₀ aryl C₁₋₆ alkoxy" means a C₁₋₆ alkoxy substituted with one C₆₋₁₀ aryl. Examples thereof include benzyloxy.

"C₆₋₁₀ aryl C₁₋₆ alkyl" means a C₁₋₆ alkyl substituted with one C₆₋₁₀ aryl. Examples thereof include benzyl.

"5- or 6-membered heteroaryl C₁₋₆ alkyl" means a C₁₋₆ alkyl substituted with one 5-or 6-membered heteroaryl. Examples thereof include 2-pyridylmethyl, 3-pyridylmethyl, 2-thienylmethyl, 3-thienylmethyl, 2-thiazolylmethyl, and the like.

"C₃₋₈ cycloalkyl C₁₋₆ alkyl" means a C₁₋₆ alkyl substituted with one C₃₋₈ cycloalkyl. Examples thereof include cyclopropylmethyl, cyclopropylethan-1-yl, cyclobutylmethyl, cyclopentylmethyl, cyclohexylmethyl, and the like.

"3- to 8-membered heterocycloalkyl C₁₋₆ alkyl" means a C₁₋₆ alkyl substituted with one 3- to 8-membered heterocycloalkyl. Examples thereof include tetrahydrofuranylmethyl.

In a case where a group including an acyclic structure and a cyclic structure is substituted with a group selected from the substituent group A, either or both of the acyclic structure and the cyclic structure may be substituted. Examples of the C₃₋₈ cycloalkyl C₁₋₆ alkyl substituted with 1 to 3 groups selected from the substituent group A include (1-cyanocyclopropyl)methyl, (1-methylcyclopropyl)methyl, (2-fluorocyclopropyl)methyl, (2,2-difluorocyclopropyl)methyl, (1-(fluoromethyl)cyclopropyl)methyl, (1-(difluoromethyl)cyclopropyl)methyl, (2,2-difluoro-1-methylcyclopropyl)methyl, (2,2-difluorocyclobutyl)methyl, (3,3-difluorocyclobutyl)methyl, 1-cyclopropyl-2,2,2-trifluoroethan-1-yl, and the like.

The following abbreviations in the main text, drawings, and tables have the following respective meanings.
Boc₂O: di-tert-Butyl dicarbonate
CDI: 1,1'-Carbonyldiimidazole
DCE: 1,2-Dichloroethane
DCM: Dichloromethane
DIPEA: N,N-Diisopropylethylamine
DMAP: 4-Dimethylaminopyridine
DME: 1,2-Dimethoxyethane
DMF: N,N-Dimethylformamide
DMT-MM: 4-(4,6-Dimethoxy-1,3,5-triazin-2-yl)-4-methylmorpholinium chloride
EDC-HCl: 1-(3-Dimethylaminopropyl)-3-ethylcarbodiimide hydrochloride
Et₂O: Diethyl ether
HOBt: 1-Hydroxybenzotriazole
HOBt hydrate: HOBt monohydrate
KHMDS: Potassium bis(trimethylsilyl)amide
LiHMDS: Lithium bis(trimethylsilyl)amide
MeCN: Acetonitrile
MTBE: Methyl tert-butyl ether
NaHMDS: Sodium bis(trimethylsilyl)amide
NBS: N-Bromosuccinimide
Pd(amphos)₂Cl₂: Bis[di-tert-butyl(4-dimethylaminophenyl)phosphine]dichloropalladium (II)
Pd(dppf)Cl₂·DCM: [1,1'-Bis(diphenylphosphino)ferrocene]palladium(II) dichloride-dichloromethane complex (1:1)
Pd(PPh₃)₄: Tetrakis(triphenylphosphine)palladium (0)
T3P (registered trademark): Propylphosphonic acid anhydride (cyclic trimer)
TBAF: Tetrabutylammonium fluoride
TEA: Triethylamine
TEMPO: 2,2,6,6-Tetramethylpiperidine 1-oxyl free radical
TFA: Trifluoroacetic acid
THF: Tetrahydrofuran
Xantphos: 4,5-Bis(diphenylphosphino)-9,9-dimethylxanthene
10% Pd/C: 10% Palladium on carbon (wetted with about 55% of water)
Amino silica gel: Aminopropylated silica gel
Diamine silica gel: CHROMATOREX Q-PACK DNH60 (Fuji Silysia Chemical Ltd.)
ODS: Octadecylsilylated silica gel
Method A: Column chromatography in which a silica gel column is connected to the lower portion of an aminopropylated silica gel column
Method B: Column chromatography in which an aminopropylated silica gel column is connected to the lower portion of a silica gel column
Process: Step
Scheme: Scheme
Ref. No.: Reference Example number
Str.: Structural formula
Ex. No.: Example number
Phys. data: Physical property value
IC₅₀: 50% inhibitory concentration
(Numerals with "*" in the tables indicate inhibition rates at a compound concentration of 30 µM.)
¹H-NMR: Proton nuclear magnetic resonance spectrum
DMSO: Dimethyl sulfoxide
DMSO-d₆: Dimethyl sulfoxide-d6
CDCl₃: Chloroform-d1
MS: Mass spectrometry
(MS values in the tables were measured by multi-ionization using electrospray ionization and atmospheric pressure chemical ionization.)
cAMP: Adenosine 3',5'-cyclic monophosphate
CHO: Chinese hamster ovary
FBS: Fetal bovine serum
HEPES: 2-(4-(2-Hydroxyethyl)-1-piperazinyl)ethanesulfonic acid
IBMX: 3-Isobutyl-1-methylxanthine

In a case where there are one or more asymmetric carbon atoms in the compound represented by the formula (I), the present invention also includes compounds having each asymmetric atom in an R configuration, compounds having each asymmetric atom in an S configuration, and compounds obtained by combining the above-described compounds in an arbitrary manner. In addition, racemic compounds, racemic mixtures, single enantiomers, and diastereomeric mixtures thereof are also included in the scope of the present invention.

In a case where there are cis-trans isomers for the compound represented by the formula (I), all of the cis-trans isomers are included in the present invention.

In a case where there are tautomers for the compound represented by the formula (I), the present invention also includes any of the tautomers thereof.

In the present invention, stereochemistry can also be determined by a method known in the art.

The compound represented by the formula (I) can also be made into a pharmacologically acceptable salt thereof according to a normal method as necessary. Examples of such a salt include acid addition salts and salts with bases.

Examples of the acid addition salts include acid addition salts with mineral acids such as hydrochloric acid, hydrobromic acid, hydroiodic acid, sulfuric acid, nitric acid, and phosphoric acid, and acid addition salts with organic acids such as formic acid, acetic acid, TFA, methanesulfonic acid, benzenesulfonic acid, p-toluenesulfonic acid, propionic acid, citric acid, succinic acid, tartaric acid, fumaric acid, butyric acid, oxalic acid, malonic acid, maleic acid, lactic acid, malic acid, carbonic acid, benzoic acid, glutamic acid, and aspartic acid.

Examples of the salts with bases include salts with inorganic bases such as lithium salts, sodium salts, potassium salts, calcium salts, and magnesium salts, and salts with organic bases such as N-methyl-D-glucamine, N,N'-dibenzylethylenediamine, TEA, piperidine, morpholine, pyrrolidine, arginine, lysine, and choline.

Unless otherwise specified, suffixes to chemical names or structural formulas regarding salts, for example, "hydrochloride" or "HCl" are not stoichiometric descriptions, but simply mean salt forms.

In a case where the compound represented by the formula (I) or the pharmacologically acceptable salt thereof is present as, for example, a crystal, the present invention also includes all crystal forms. For example, the pharmacologically acceptable salt also includes solvates with pharmacologically acceptable solvents such as water or ethanol, cocrystals with appropriate coformers, and the like.

In the compound represented by the formula (I), some of the individual atoms may be replaced with isotopes corresponding to the individual atoms. The present invention also includes compounds in which an atom has been replaced with an isotope. Examples of the isotopes include isotopes of a hydrogen atom, a carbon atom, a chlorine atom, a fluorine atom, an iodine atom, a nitrogen atom, an oxygen atom, and a sulfur atom represented by ²H, ³H, ¹¹C, ¹³C, ¹⁴C, ³⁶Cl, ¹⁸F, ¹²³I, ¹²⁵I, ¹³N, ¹⁵N, ¹⁵O, ¹⁷O, ¹⁸O, and ³⁵S, respectively. Examples of one embodiment include compounds in which some of the hydrogen atoms in the compound represented by the formula (I) have been replaced with ²H (D: deuterium atom).

Compounds obtained by replacing some of the atoms with isotopes in the compound represented by the formula (I) can be manufactured by the same method as a manufacture method to be described below using commercially available building blocks into which the isotopes have been introduced. For example, a compound obtained by replacing some of the hydrogen atoms in the compound represented by the formula (I) with deuterium atoms can also be manufactured using the above-described method and a method described in a publication (for example, refer to Journal of Synthetic Organic Synthesis, Vol. 65, No. 12, pp. 1179 to 1190, 2007). In addition, for example, a compound obtained by replacing some of the carbon atoms in the compound represented by the formula (I) with ¹³C can also be manufactured using the above-described method and a method described in a publication (for example, refer to RADIOISOTOPES, Vol. 56, No. 11, pp. 741 to 750, 2007).

The compound represented by the formula (I) of the present invention can be manufactured by, for example, methods shown in Schemes 1 to 12 or similar methods or methods described in publications or similar methods. In the Schemes, the compound represented by the formula (I) corresponds to compounds represented by compounds (Ia) to (In) and a compound (I').

The compound represented by the formula (I) of the present invention can be manufactured by the following method, but the following manufacture method is simply an example of general manufacture methods and does not limit the manufacture method.

In a reaction of each step, in a case where a raw material or a reagent is commercially available, a commercially available product can be used.

In the reaction of each step, the reaction time varies depending on a raw material to be used, a solvent, a reaction temperature, and the like, but is normally 30 minutes to 3 days unless particularly otherwise specified.

In the reaction of each step, the reaction temperature varies depending on a raw material to be used, a solvent, or the like, but is normally -78°C to a reflux temperature unless particularly otherwise specified.

In the reaction of each step, the pressure varies depending on a raw material to be used, a solvent, the reaction temperature, and the like, but is normally 1 to 20 atm unless particularly otherwise specified.

In the reaction of each step, a microwave reaction device such as Initiator manufactured by Biotage AB can be used. In the case of performing the reaction using the microwave reaction device, the reaction can be performed under conditions, while varying depending on a raw material to be used, a solvent, a model, or the like, of a pressure range: 1 to 30 bar, power range: 1 to 400 W, reaction temperature: room temperature to 300°C, and reaction time: 1 minute to 1 day.

Unless particularly otherwise specified, the reaction in each step is performed in the absence of a solvent or using an appropriate solvent. Examples of the appropriate solvent include solvents inert to the reaction. Specific examples of usable solvents include solvents described in reference examples and examples corresponding to each step and the following solvents. Two or more of the following solvents may be mixed together at an appropriate ratio and used.
Alcohols: Methanol, ethanol, tert-butyl alcohol, 2-propanol, and the like;
Ethers: Et₂O, THF, DME, 1,4-dioxane, cyclopentyl methyl ether, and the like;
Aromatic hydrocarbons: Benzene, chlorobenzene, 1,2-dichlorobenzene, toluene, xylene, and the like;
Saturated hydrocarbons: Cyclohexane, n-hexane, and the like;
Amides: DMF, N,N-dimethylacetamide, N-methylpyrrolidone, and the like;
Halogenated hydrocarbons: DCM, DCE, carbon tetrachloride, and the like;
Nitriles: MeCN and the like;
Sulfoxides: DMSO and the like;
Aromatic organic bases: Pyridine and the like;
Acid anhydrides: Acetic anhydride and the like;
Organic acids: Formic acid, acetic acid, TFA, and the like;
Esters: Ethyl acetate, methyl acetate, isopropyl acetate, and the like;
Ketones: Acetone, methyl ethyl ketone, and the like;
Water.

In the reaction of each step, in a case where a base is used, the reaction is performed using a base suitable for the reaction. Specific examples of usable bases include bases described in reference examples and examples corresponding to each step and the following bases.
Inorganic bases: Sodium hydroxide, lithium hydroxide, potassium hydroxide, and the like; Basic salts: Sodium carbonate, sodium bicarbonate, potassium carbonate, cesium carbonate, cesium fluoride, and the like;
Organic bases: TEA, DIPEA, diethylamine, pyridine, DMAP, 2,6-lutidine, 1,8-diazabicyclo[5.4.0]-7-undecene, imidazole, piperidine, and the like;
Metal alkoxides: Sodium ethoxide, sodium methoxide, sodium tert-butoxide, potassium tert-butoxide, and the like;
Alkali metal hydrides: Sodium hydride and the like;
Metal amides: Sodium amide, lithium diisopropylamide, LiHMDS, NaHMDS, KHMDS, and the like;
Organomagnesiums: Isopropylmagnesium chloride and the like;
Organolithiums: n-Butyllithium, sec-butyllithium, tert-butyllithium, and the like.

In a case where an acid or an acidic catalyst is used in the reaction of each step, the reaction is performed using an acid or acid catalyst suitable for the reaction. Specific examples of usable acids or acidic catalysts include acids or acidic catalysts described in reference examples and examples corresponding to each step or the following acids or acidic catalysts.
Inorganic acids: Hydrochloric acid, sulfuric acid, nitric acid, hydrobromic acid, phosphoric acid, and the like;
Organic acids: Acetic acid, TFA, citric acid, methanesulfonic acid, p-toluenesulfonic acid, 10-camphorsulfonic acid, and the like;
Lewis acids: BF₃-Et₂O complex, zinc iodide, aluminum chloride, zinc chloride, titanium(IV) chloride, and the like.

In the reaction of each step, in a case where a condensing agent is used, the reaction is performed using a condensing agent suitable for the reaction. Specific examples of usable condensing agents include condensing agents described in reference examples and examples corresponding to each step and the following condensing agents.
Carbodiimides: EDC-HCl, N,N'-dicyclohexylcarbodiimide, and the like;
Imidazoles: CDI and the like;
Uronium salts and phosphonium salts: O-(7-azabenzotriazol-1-yl)-N,N,N',N'-tetramethyluronium hexafluorophosphate, 1H-benzotriazol-1-yl
oxytris(dimethylamino)phosphonium hexafluorophosphate, and the like;
Triazines: DMT-MM and the like;
Other: T3P and the like.

In the reaction of each step, in a case where a reducing agent is used, the reaction is performed using a reducing agent suitable for the reaction. Specific examples of usable reducing agents include reducing agents described in reference examples and examples corresponding to each step and the following reducing agents.
Metal hydrides: Lithium aluminum hydride, lithium borohydride, sodium borohydride, sodium triacetoxyborohydride, sodium cyanoborohydride, diisobutylaluminum hydride, and the like;
Boranes: BH₃-THF complex, picoline borane complex, decaborane, and the like.

In the reaction of each step, in a case where an oxidizing agent is used, the reaction is performed using an oxidizing agent suitable for the reaction. Specific examples of usable oxidizing agents include oxidizing agents described in reference examples and examples corresponding to each step and the following oxidizing agents.
Peracids: m-Chloroperbenzoic acid, hydrogen peroxide, tert-butyl hydroperoxide, and the like;
Chlorates: Sodium hypochlorite, sodium chlorite, and the like;
Iodates: Sodium periodate and the like;
Hypervalent iodine reagents: Iodobenzene diacetate, Dess-Martin periodinane, and the like; Reagents with chromium: Pyridinium dicromate, Jones reagent, and the like;
Reagents with manganese: Manganese dioxide, potassium permanganate, and the like.

In the reaction of each step, in a case where a carbonyl group introduction reagent is used, the reaction is performed using a carbonyl group introduction reagent suitable for the reaction. Specific examples of usable carbonyl group introduction reagents include carbonyl group introduction reagents described in reference examples and examples corresponding to each step and the following carbonyl group introduction reagents.
Phosgenes: Phosgene, diphosgene, triphosgene, and the like;
Chloroformate esters: 4-Nitrophenyl chloroformate and the like;
Imidazoles: CDI and the like.

In a case where a protective group is required depending on the type of a functional group in each step, it is also possible to perform an introduction operation and a removal operation in an appropriate combination according to a normal method. Regarding the type of the protective group, protection, and deprotection, for example, methods described in "Greene's Protective Groups in Organic Synthesis," fifth edition, Wiley-Interscience, 2014 edited by Peter G. M. Wuts may be referred to.

In a case where a hydrolysis reaction is performed in each step, the reaction can be performed in the presence of an acid or a base. Examples of usable acids and bases include the above-described examples.

In a case where a catalytic reduction reaction is performed in each step, the reaction can be performed in the presence of hydrogen and a catalyst. Examples of usable catalysts include palladium on carbon powder, Perlman's catalyst, platinum carbon powder, RANEY nickel, and the like. An acid may be used in the reaction as necessary.

In a case where a metal reduction reaction is performed in each step, the reaction can be performed in the presence of a metal or the like. Examples of usable metals or the like include an iron powder, a zinc powder, tin chloride, titanium trichloride, and the like. An acid may be used in the reaction as necessary.

In a case where a reduction reaction is performed in each step, the reaction can be performed in the presence of a reducing agent. Examples of usable reducing agents include the above-described examples.

In a case where an oxidation reaction is performed in each step, the reaction can be performed in the presence of an oxidizing agent. Examples of usable oxidizing agents include the above-described examples. In a case where a nitroxylradical oxidation catalyst is used, the reaction can be performed in the presence of a re-oxidizing agent. Examples of usable nitroxylradical oxidation catalysts include TEMPO, 2-azaadamantane-N-oxyl, and the like. Examples of usable re-oxidizing agents include sodium hypochlorite, iodobenzene diacetate, and the like. An additive such as tetrabutylammonium hydrogen sulfate or potassium bromide may be added as necessary for the reaction.

In a case where an amidation reaction is performed in each step, the reaction can be performed using a condensing agent in the presence or absence of a base. Examples of usable condensing agents and bases include the above-described examples. In a case where a carbodiimide is used as a condensing agent, an additive such as HOBt or DMAP may be added as necessary for the reaction. In addition, the reaction can be performed using an acyl halide or an acid anhydride.

In a case where a carbamate formation reaction or a urea formation reaction is performed in each step, the reaction can be performed using a carbonyl group introduction reagent in the presence or absence of a base. Examples of usable carbonyl group introduction reagents and bases include the above-described examples.

In a case where a Staudinger reaction is performed in each step, the reaction can be performed in the presence of a phosphine compound. Examples of usable phosphine compounds include triphenylphosphine and the like.

In a case where a Suzuki-Miyaura cross coupling reaction is performed in each step, the reaction can be performed in the presence of a palladium catalyst and a base. Examples of usable palladium catalysts include Pd(amphos)₂Cl₂, Pd(PPh₃)₄, and the like. Examples of usable bases include the above-described examples.

In a case where a Migita-Kosugi-Stille cross coupling reaction is performed in each step, the reaction can be performed in the presence of a palladium catalyst. Examples of usable palladium catalysts include Pd(PPh₃)₄ and the like.

In a case where a bromination reaction is performed in each step, the reaction can be performed in the presence of a bromination reagent. Examples of usable bromination reagents include NBS and the like.

In a case where an aromatic nucleophilic substitution reaction is performed in each step, the reaction can be performed in the presence of a base. Examples of usable bases include the above-described examples.

In a case where a Horner-Wadsworth-Emmons reaction is performed in each step, the reaction can be performed in the presence of a base. Examples of usable bases include the above-described examples.

In a case where an Appel reaction is performed in each step, the reaction can be performed in the presence of a halogen source and a phosphine compound. Examples of usable halogen sources include carbon tetrachloride, carbon tetrabromide, iodine, and the like. Examples of usable phosphine compounds include triphenylphosphine and the like.

In a case where a Curtius rearrangement reaction is performed in each step, the reaction can be performed in the presence of an azide source. Examples of usable azide sources include sodium azide, diphenylphosphoryl azide, and the like.

In a case where a diazotization reaction and a subsequent Sandmeyer reaction are performed in each step, the reaction can be performed in the presence of a nitrite ester and a copper salt. Examples of usable nitrite esters include tert-butyl nitrite and the like. Examples of usable copper salts include copper (II) bromide and the like.

The compound represented by the formula (I) can be manufactured according to, for example, a method described in Scheme 1.

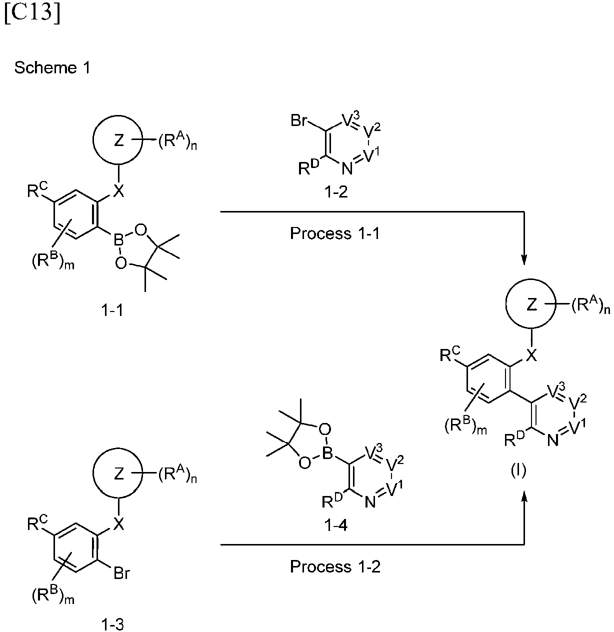

Symbols in the formula have the same meanings as described above.

### Process 1-1

A compound (I) can also be manufactured by a Suzuki-Miyaura cross coupling reaction of a compound (1-1) and a compound (1-2).

### Process 1-2

The compound (I) can also be manufactured by a Suzuki-Miyaura cross coupling reaction of a compound (1-3) and a compound (1-4).

Compounds represented by formula (Ia), formula (Ib), formula (Ic), and formula (Id) can be manufactured according to, for example, a method described in Scheme 2.

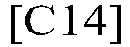

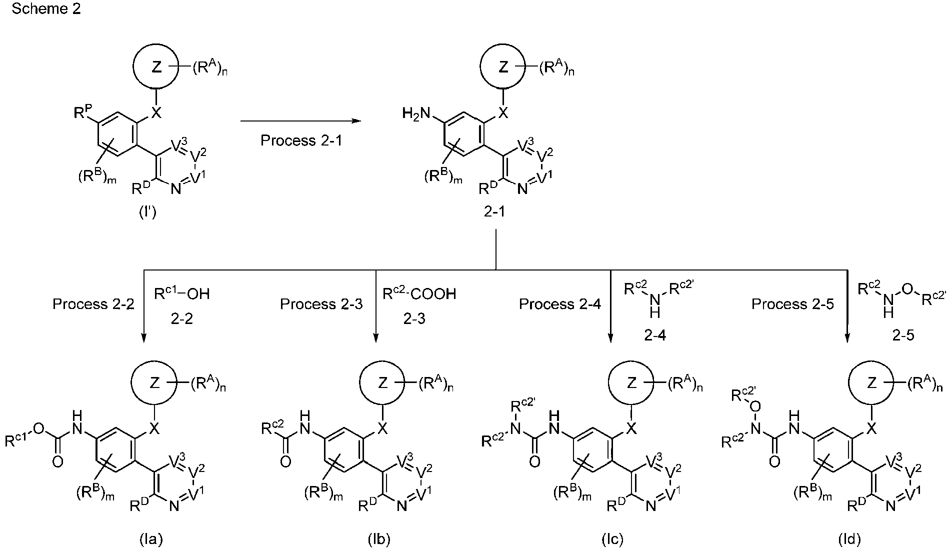

Symbols in the formula have the same meanings as described above. R^{P} is a protected amino group.

### Process 2-1

A compound (2-1) can also be manufactured by removing a protective group in the compound (I').

### Process 2-2

The compound (Ia) can also be manufactured by a carbamate formation reaction between the compound (2-1) and a compound (2-2).

### Process 2-3

The compound (Ib) can also be manufactured by an amidation reaction between the compound (2-1) and the compound (2-3).

### Process 2-4

The compound (Ic) can also be manufactured by a urea formation reaction between the compound (2-1) and a compound (2-4).

### Process 2-5

The compound (Id) can also be manufactured by a urea formation reaction between the compound (2-1) and a compound (2-5).

Compounds represented by formula (Ie) and formula (If) can be manufactured according to, for example, a method described in Scheme 3.

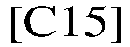

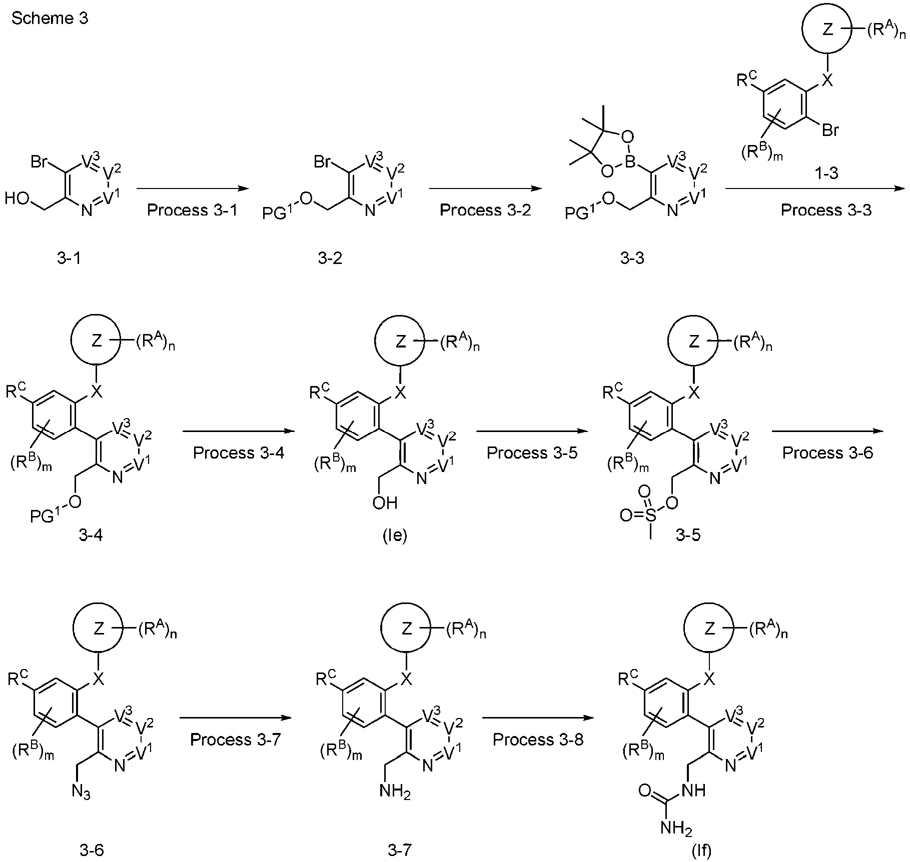

Symbols in the formula have the same meanings as described above. PG¹ is a protective group.

### Process 3-1

A compound (3-2) can also be manufactured by introducing a protective group into a hydroxyl group of a compound (3-1).

### Process 3-2

A compound (3-3) can also be manufactured by reacting the compound (3-2) and bis(pinacolato)diboron in the presence of a palladium catalyst.

### Process 3-3

A compound (3-4) can also be manufactured by a Suzuki-Miyaura cross coupling reaction of the compound (3-3) and the compound (1-3).

### Process 3-4

A compound (Ie) can also be manufactured by removing a protective group in the compound (3-4).

### Process 3-5

A compound (3-5) can also be manufactured by reacting the compound (Ie) and methanesulfonyl chloride in the presence of a base.

### Process 3-6

A compound (3-6) can also be manufactured by reacting the compound (3-5) and sodium azide.

### Process 3-7

A compound (3-7) can also be manufactured by a Staudinger reaction of the compound (3-6).

### Process 3-8

A compound (If) can also be manufactured by reacting the compound (3-7) and trimethylsilyl isocyanate. As another method, the compound (If) can also be manufactured by reacting the compound (3-7) and potassium cyanate, sodium cyanate, or the like in the presence of an acid.

Compounds represented by formula (Ig) and formula (Ih) can be manufactured according to, for example, a method described in Scheme 4. 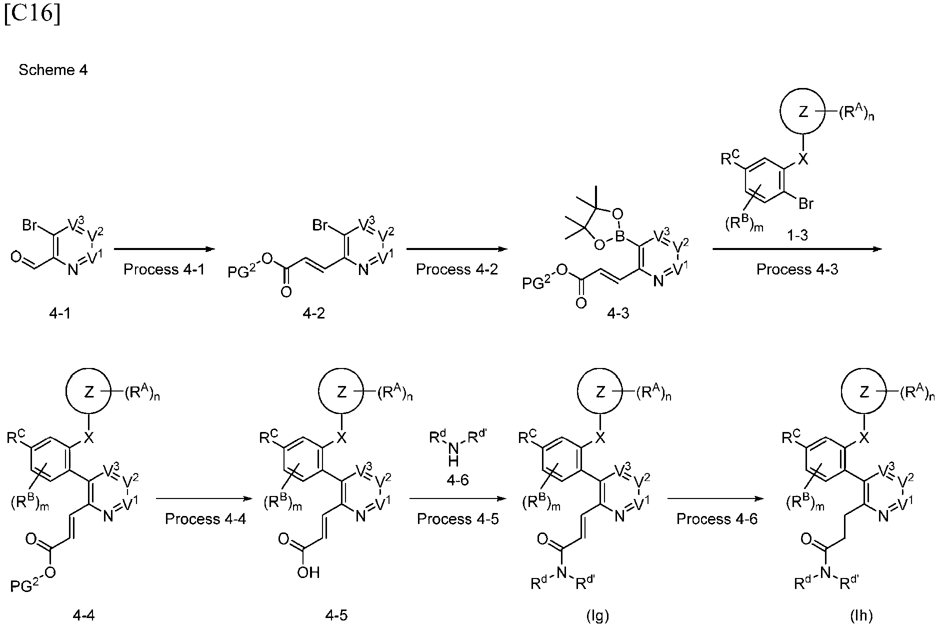 Symbols in the formula have the same meanings as described above. PG² is a protective group.

### Process 4-1

A compound (4-2) can also be manufactured by a Horner-Wadsworth-Emmons reaction of the compound (4-1).

### Process 4-2

A compound (4-3) can also be manufactured by reacting the compound (4-2) and bis(pinacolato)diboron in the presence of a palladium catalyst.

### Process 4-3

A compound (4-4) can also be manufactured by a Suzuki-Miyaura cross coupling reaction of the compound (4-3) and the compound (1-3).

### Process 4-4

A compound (4-5) can also be manufactured by removing a protective group in the compound (4-4).

### Process 4-5

The compound (Ig) can also be manufactured by an amidation reaction between the compound (4-5) and a compound (4-6).

### Process 4-6

A compound (Ih) can also be manufactured by a catalytic reduction reaction of the compound (Ig).

A compound represented by formula (Ii) can be manufactured according to, for example, a method described in Scheme 5.

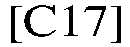

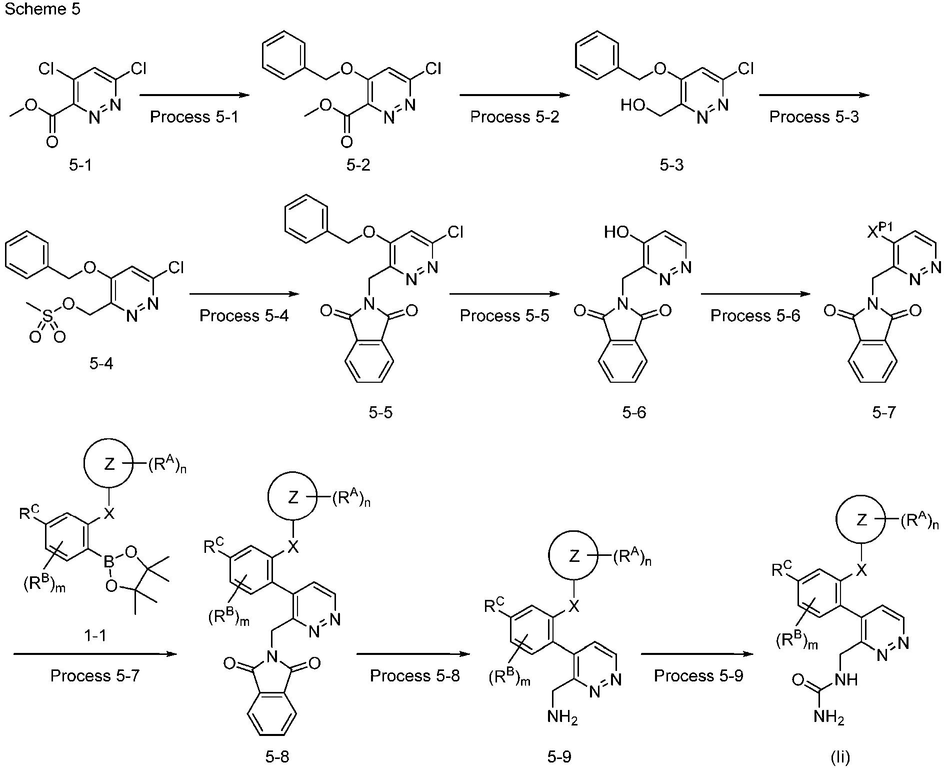

Symbols in the formula have the same meanings as described above. X^{P1} is a chlorine atom or a bromine atom.

### Process 5-1

A compound (5-2) can also be manufactured by an aromatic nucleophilic substitution reaction of the compound (5-1).

### Process 5-2

A compound (5-3) can also be manufactured by a reduction reaction of the compound (5-2).

### Process 5-3

A compound (5-4) can also be manufactured by reacting the compound (5-3) and methanesulfonyl chloride in the presence of a base.

### Process 5-4

A compound (5-5) can also be manufactured by reacting the compound (5-4) and phthalimide in the presence of a base.

### Process 5-5

A compound (5-6) can also be manufactured by a catalytic reduction reaction of the compound (5-5).

### Process 5-6

A compound (5-7) can also be manufactured by reacting the compound (5-6) and phosphorus oxychloride or phosphorus oxybromide.

### Process 5-7

A compound (5-8) can also be manufactured by a Suzuki-Miyaura cross coupling reaction of the compound (5-7) and the compound (1-1).

### Process 5-8

A compound (5-9) can also be manufactured by removing a phthaloyl group in the compound (5-8).

### Process 5-9

A compound (Ii) can also be manufactured by reacting the compound (5-9) and trimethylsilyl isocyanate. As another method, the compound (Ii) can also be manufactured by reacting the compound (5-9) and potassium cyanate, sodium cyanate, or the like in the presence of an acid.

Compounds represented by formula (Ij), formula (Ik), and formula (Il) can be manufactured according to, for example, a method described in Scheme 6.

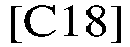

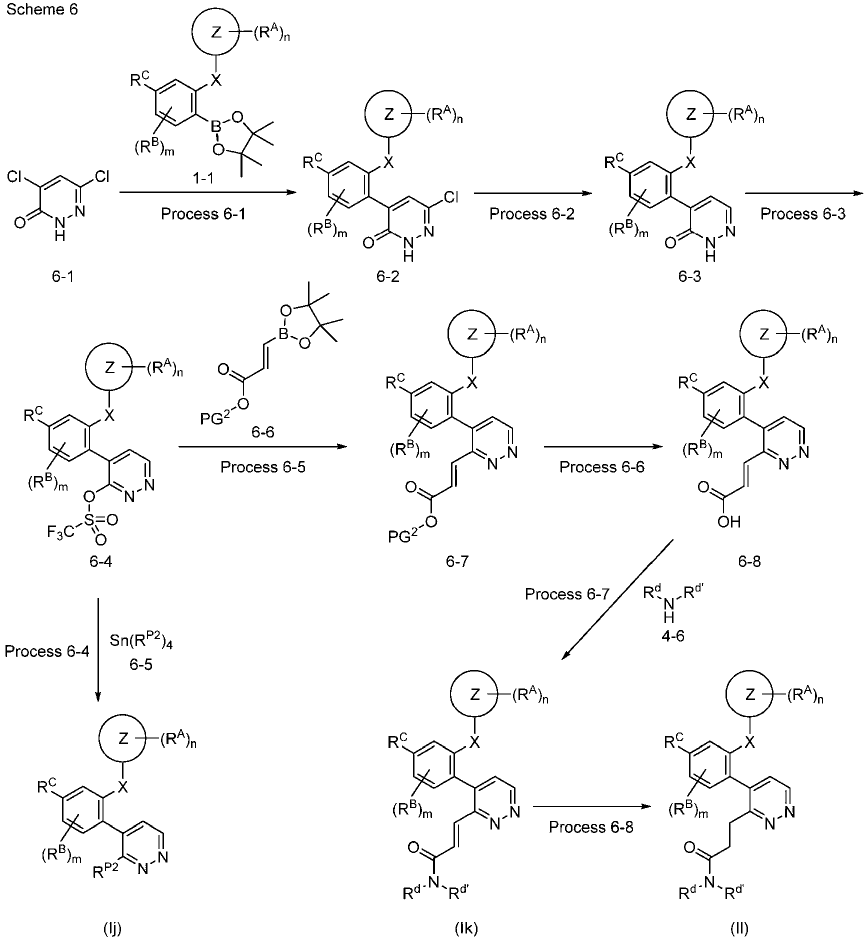

Symbols in the formula have the same meanings as described above. R^{P2} is a C₁₋₆ alkyl.

### Process 6-1

A compound (6-2) can also be manufactured by a Suzuki-Miyaura cross coupling reaction of a compound (6-1) and the compound (1-1).

### Process 6-2

A compound (6-3) can also be manufactured by a catalytic reduction reaction of the compound (6-2).

### Process 6-3

A compound (6-4) can also be manufactured by reacting the compound (6-3) and N-Phenylbis(trifluoromethanesulfonimide).

### Process 6-4

A compound (Ij) can also be manufactured by a Migita-Kosugi-Stille cross coupling reaction between the compound (6-4) and the compound (6-5).

### Process 6-5

A compound (6-7) can also be manufactured by a Suzuki-Miyaura cross coupling reaction of the compound (6-4) and a compound (6-6).

### Process 6-6

A compound (6-8) can also be manufactured by removing a protective group in the compound (6-7).

### Process 6-7

The compound (Ik) can also be manufactured by an amidation reaction between the compound (6-8) and the compound (4-6).

### Process 6-8

A compound (Il) can also be manufactured by a catalytic reduction reaction of the compound (Ik).

Compounds represented by formula (Im) and formula (In) can be manufactured according to, for example, a method described in Scheme 7.

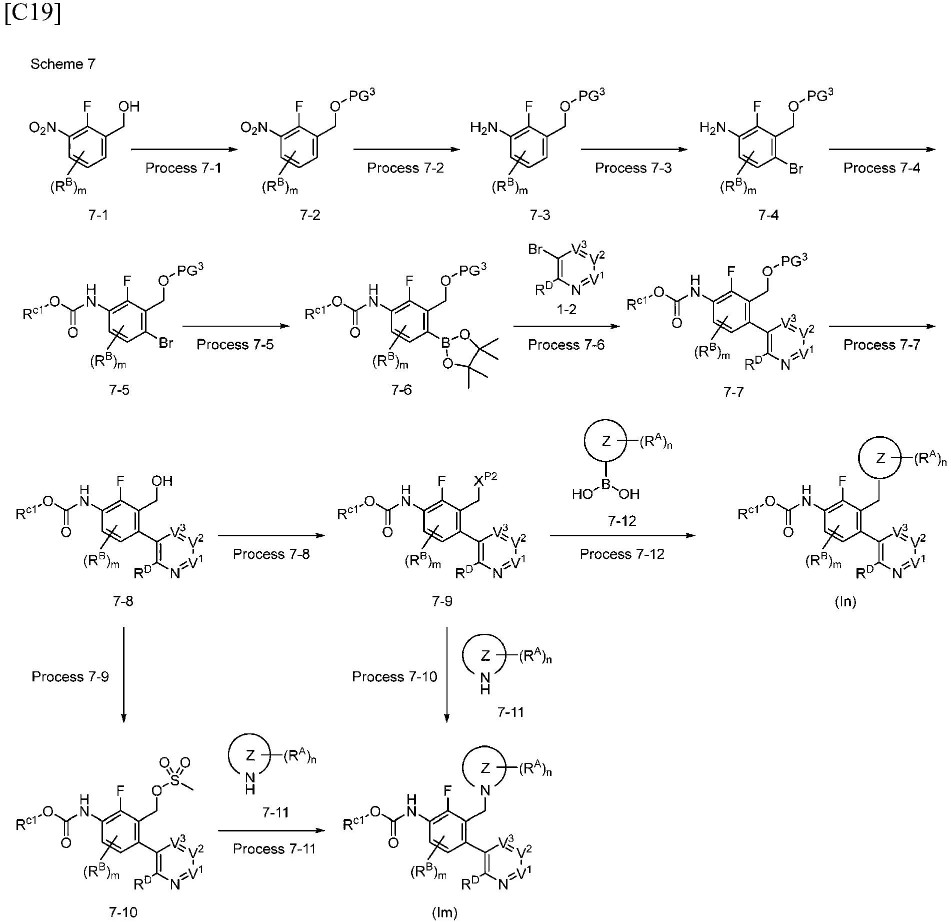

Symbols in the formula have the same meanings as described above. PG³ is a protective group. X^{P2} is a chlorine atom, a bromine atom, or an iodine atom.

### Process 7-1

A compound (7-2) can also be manufactured by introducing a protective group into a hydroxyl group of a compound (7-1).

### Process 7-2

A compound (7-3) can also be manufactured by a catalytic reduction reaction or metal reduction reaction of the compound (7-2).

### Process 7-3

A compound (7-4) can also be manufactured by a bromination reaction of the compound (7-3).

### Process 7-4

The compound (7-5) can also be manufactured by a carbamate formation reaction of the compound (7-4).

### Process 7-5

A compound (7-6) can also be manufactured by reacting the compound (7-5) and bis(pinacolato)diboron in the presence of a palladium catalyst.

### Process 7-6

A compound (7-7) can also be manufactured by a Suzuki-Miyaura cross coupling reaction of the compound (7-6) and the compound (1-2).

### Process 7-7

A compound (7-8) can also be manufactured by removing a protective group in the compound (7-7).

### Process 7-8

A compound (7-9) can also be manufactured by an Appel reaction of the compound (7-8).

### Process 7-9

A compound (7-10) can also be manufactured by reacting the compound (7-8) and methanesulfonyl chloride in the presence of a base.

### Process 7-10

A compound (Im) can also be manufactured by reacting the compound (7-9) and a compound (7-11).

### Process 7-11

A compound (Im) can also be manufactured by reacting the compound (7-10) and the compound (7-11) in the presence of a base.

### Process 7-12

A compound (In) can also be manufactured by a Suzuki-Miyaura cross coupling reaction of the compound (7-9) and a compound (7-12).

A compound (1-2a) and a compound (1-2b) can be manufactured according to, for example, a method described in Scheme 8.

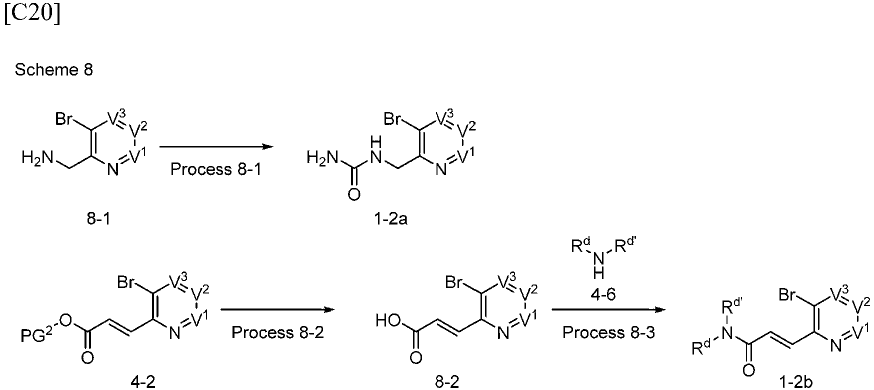

Symbols in the formula have the same meanings as described above.

### Process 8-1

A compound (1-2a) can also be manufactured by reacting the compound (8-1) and trimethylsilyl isocyanate. As another method, the compound (1-2a) can also be manufactured by reacting the compound (8-1) and potassium cyanate, sodium cyanate, or the like in the presence of an acid.

### Process 8-2

A compound (8-2) can also be manufactured by removing a protective group in the compound (4-2).

### Process 8-3

The compound (1-2b) can also be manufactured by an amidation reaction between the compound (8-2) and the compound (4-6).

A compound (1-3a) and a compound (1-1a) can be manufactured according to, for example, a method described in Scheme 9.

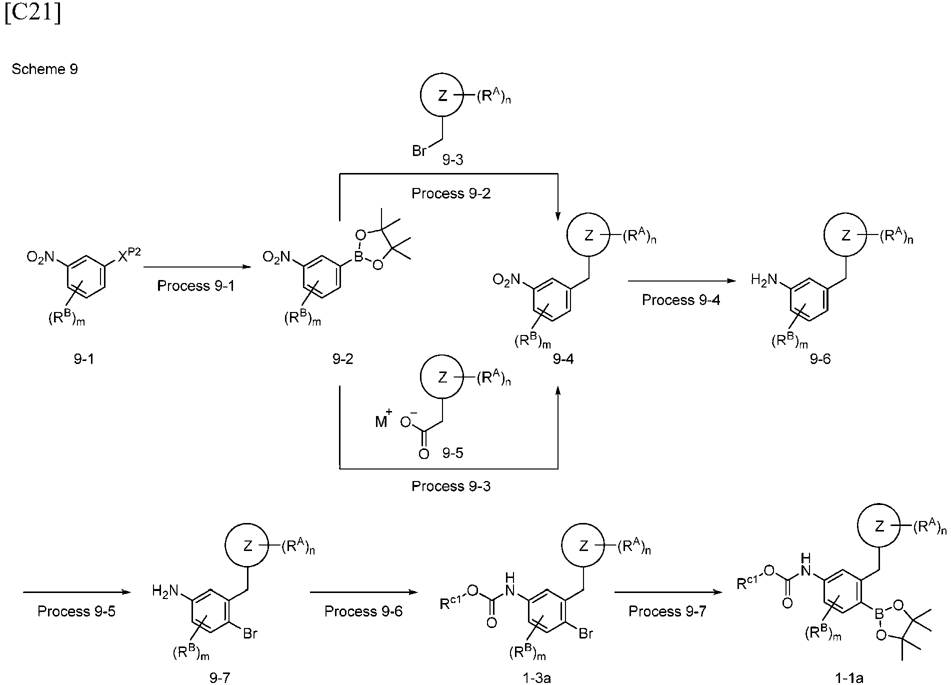

Symbols in the formula have the same meanings as described above. M is a lithium atom, a sodium atom, or a potassium atom.

### Process 9-1

A compound (9-2) can also be manufactured by reacting a compound (9-1) and bis(pinacolato)diboron in the presence of a palladium catalyst.

### Process 9-2

A compound (9-4) can also be manufactured by a Suzuki-Miyaura cross coupling reaction of the compound (9-2) and a compound (9-3).

### Process 9-3

The compound (9-4) can also be manufactured by reacting the compound (9-2) and a compound (9-5) in the presence of a palladium catalyst.

### Process 9-4

A compound (9-6) can also be manufactured by a catalytic reduction reaction or metal reduction reaction of the compound (9-4).

### Process 9-5

A compound (9-7) can also be manufactured by a bromination reaction of the compound (9-6).

### Process 9-6

The compound (1-3a) can also be manufactured by a carbamate formation reaction of the compound (9-7).

### Process 9-7

The compound (1-1a) can also be manufactured by reacting the compound (1-3a) and bis(pinacolato)diboron in the presence of a palladium catalyst.

A compound (1-3b) and a compound (1-1b) can be manufactured according to, for example, a method described in Scheme 10.

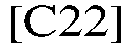

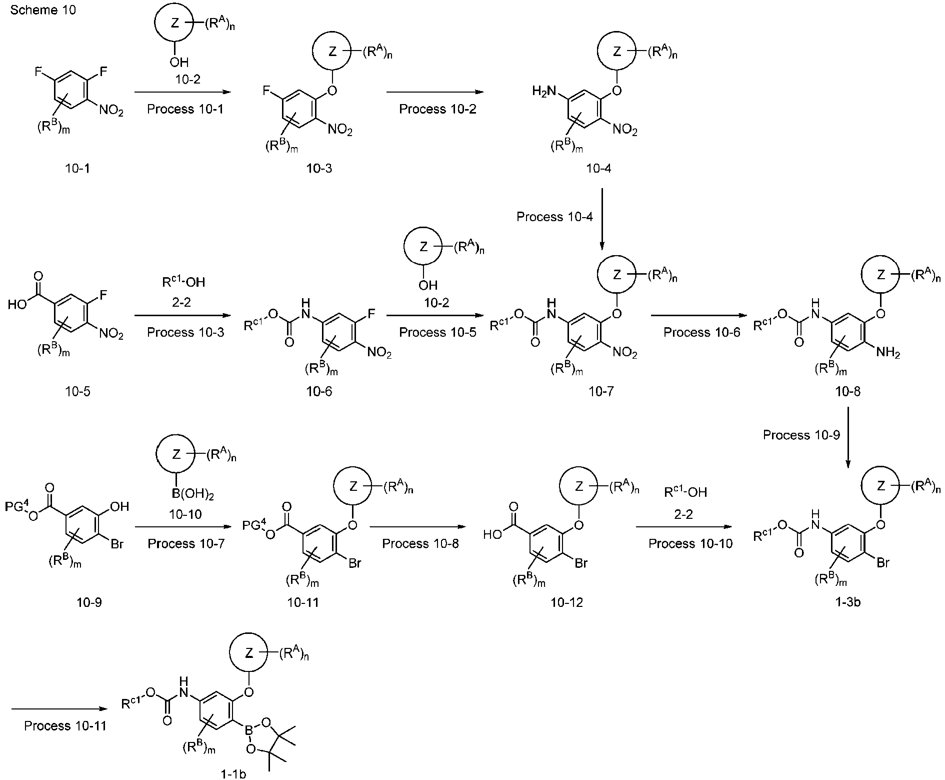

Symbols in the formula have the same meanings as described above. PG⁴ is a protective group.

### Process 10-1

A compound (10-3) can also be manufactured by an aromatic nucleophilic substitution reaction between the compound (10-1) and the compound (10-2).

### Process 10-2

A compound (10-4) can also be manufactured by reacting the compound (10-3) and ammonia water.

### Process 10-3

A compound (10-6) can also be manufactured by a Curtius rearrangement reaction between a compound (10-5) and the compound (2-2).

### Process 10-4

A compound (10-7) can also be manufactured by a carbamate formation reaction of the compound (10-4).

### Process 10-5

The compound (10-7) can also be manufactured by an aromatic nucleophilic substitution reaction between the compound (10-6) and the compound (10-2).

### Process 10-6

A compound (10-8) can also be manufactured by a catalytic reduction reaction or metal reduction reaction of the compound (10-7).

### Process 10-7

A compound (10-11) can also be manufactured by reacting a compound (10-9) and a compound (10-10) in the presence of a copper salt, for example, copper (II) acetate or the like.

### Process 10-8

A compound (10-12) can also be manufactured by removing a protective group in the compound (10-11).

### Process 10-9

The compound (1-3b) can also be manufactured by a diazotization reaction and a subsequent Sandmeyer reaction of the compound (10-8).

### Process 10-10

The compound (1-3b) can also be manufactured by a Curtius rearrangement reaction between the compound (10-12) and the compound (2-2).

### Process 10-11

A compound (1-1b) can also be manufactured by reacting the compound (1-3b) and bis(pinacolato)diboron in the presence of a palladium catalyst.

A compound (1-3c), a compound (1-1c), and a compound (1-1d) can be manufactured according to, for example, a method described in Scheme 11.

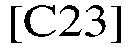

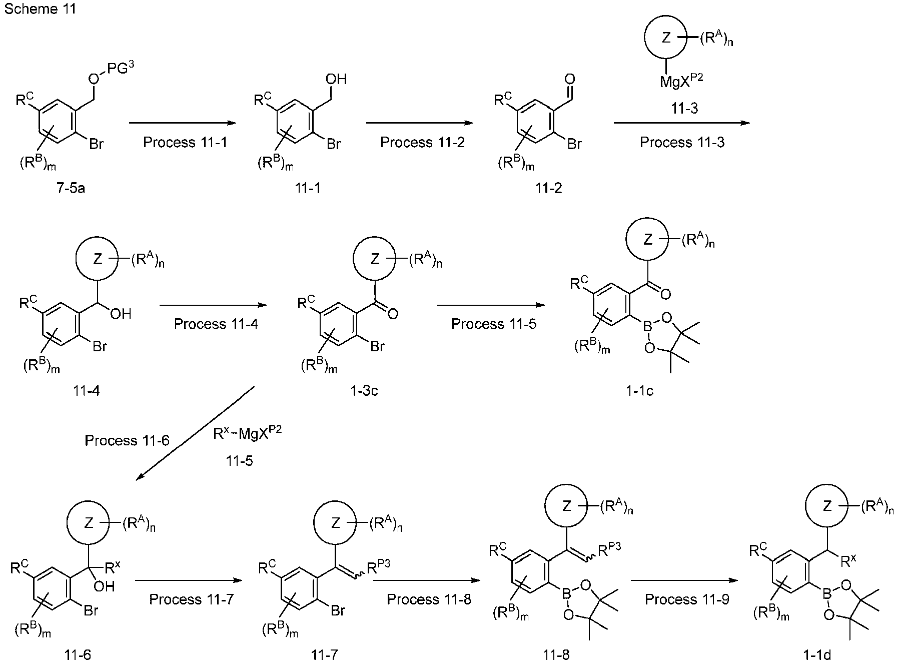

Symbols in the formula have the same meanings as described above. Here, R^{x} is a C₁₋₆ alkyl. R^{P3} is a hydrogen atom or an alkyl.

### Process 11-1

A compound (11-1) can also be manufactured by removing a protective group in the compound (7-5a).

### Process 11-2

A compound (11-2) can also be manufactured by an oxidation reaction of the compound (11-1).

### Process 11-3

A compound (11-4) can also be manufactured by reacting the compound (11-2) and a Grignard reagent (11-3).

### Process 11-4

The compound (1-3c) can also be manufactured by an oxidation reaction of the compound (11-4).

### Process 11-5

A compound (1-1c) can also be manufactured by reacting the compound (1-3c) and bis(pinacolato)diboron in the presence of a palladium catalyst.

### Process 11-6

A compound (11-6) can also be manufactured by reacting the compound (1-3c) and a Grignard reagent (11-5).

### Process 11-7

A compound (11-7) can also be manufactured by reacting the compound (11-6) and triethylsilane in the presence of an acid.

### Process 11-8

A compound (11-8) can also be manufactured by reacting the compound (11-7) and bis(pinacolato)diboron in the presence of a palladium catalyst.

### Process 11-9

A compound (1-1d) can also be manufactured by a catalytic reduction reaction of the compound (11-8).

A compound (9-4a) and a compound (1-3e) can be manufactured according to, for example, a method described in Scheme 12.

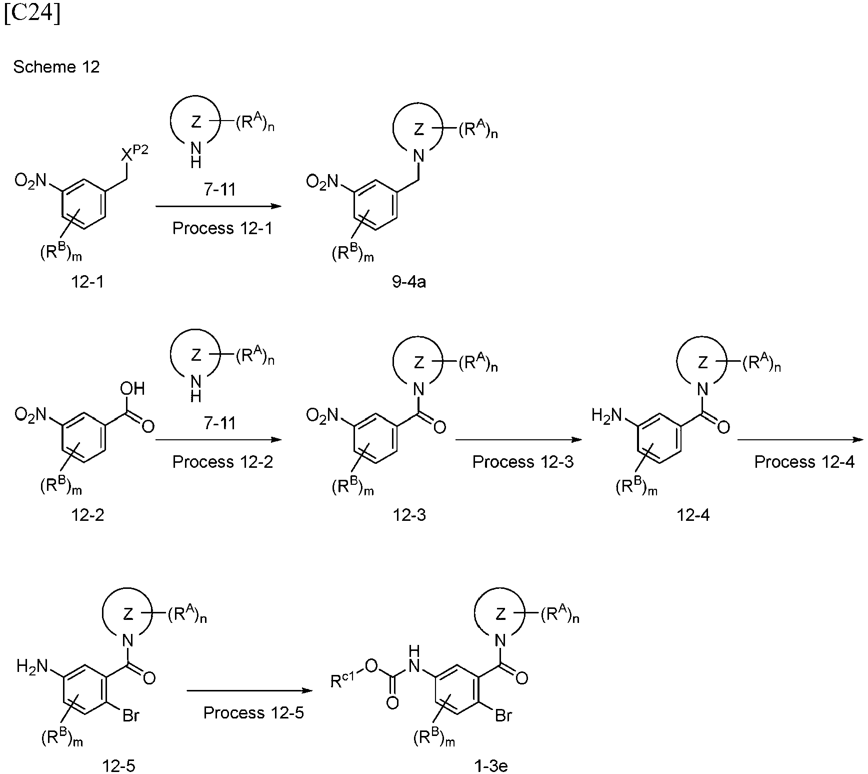

Symbols in the formula have the same meanings as described above.

### Process 12-1

A compound (9-4a) can also be manufactured by reacting a compound (12-1) and the compound (7-11).

### Process 12-2

A compound (12-3) can also be manufactured by an amidation reaction between a compound (12-2) and the compound (7-11).

### Process 12-3

A compound (12-4) can also be manufactured by a catalytic reduction reaction or metal reduction reaction of the compound (12-3).

### Process 12-4

A compound (12-5) can also be manufactured by a bromination reaction of the compound (12-4).

### Process 12-5

The compound (1-3e) can also be manufactured by a carbamate formation reaction of the compound (12-5).

Schemes described above are examples of the method for manufacturing the compound represented by the formula (I) or manufacture intermediates thereof. Schemes described above can be modified in various ways to Schemes that persons skilled in the art can easily understand.

The compound represented by the formula (I) and the manufacture intermediates thereof can also be isolated and purified by solvent extraction, crystallization, recrystallization, chromatography or preparative high performance liquid chromatography, which are isolation and purification means well known to persons skilled in the art, as necessary.

The compound of the present invention has excellent TSHR antagonist activity and can be thus used as a therapeutic agent for thyroid-related diseases. In the present invention, the thyroid-related diseases include, for example, hyperthyroidism, Graves' disease, thyroid eye disease, and thyroid cancer. Preferably, the compound of the present invention can be used as a therapeutic agent for hyperthyroidism, Graves' disease, or thyroid eye disease (refer to Endocrinology, 2014, 155 (1), pp. 310 to 314). More preferably, the compound of the present invention can be used as a therapeutic agent for hyperthyroidism or Graves' disease.

In addition, in one embodiment, hyperthyroidism includes, for example, hyperthyroidism caused by any one of Graves' disease, thyroiditis, Plummer's disease, toxic multinodular goiter, TSH-secreting pituitary adenoma, hyperemesis gravidarum, ovarian goiter, gestational trophoblastic tumor, and germ cell tumor. Preferably, the compound of the present invention can be used as a therapeutic agent for hyperthyroidism caused by Graves' disease.

In addition, in one embodiment, thyroid-related diseases are diseases and symptoms associated with abnormal thyroid hormone levels. The diseases and symptoms associated with abnormal thyroid hormone levels include, for example, diseases and symptoms caused by TRAb.

In the present invention, "treatment" includes the meaning of "prevention." For example, the treatment of hyperthyroidism, Graves' disease, or thyroid eye disease includes the meaning of "prevention of relapse/recurrence" and "maintenance of remission." In addition, in one embodiment, the compound of the present invention can be used to prevent the development of thyroid eye disease in patients with Graves' disease.

In the present invention, "antagonist" refers to a drug that inhibits or blocks the function of a target protein regardless of a binding site thereof. For example, "antagonist" includes the meaning of "allosteric antagonist" and "negative allosteric modulator (NAM)."

The therapeutic effect of the compound of the present invention on thyroid-related diseases can be confirmed according to a known method in the related art. Examples of the method for confirming the effect on hyperthyroidism or Graves' disease in model animals include methods described in Endocrinology 2007, 148 (5), pp. 2335 to 2344 and the like or similar methods.

A pharmaceutical composition of the present invention may be used in various dosage forms depending on usage. Examples of such dosage include powders, granules, fine granules, dry syrups, tablets, capsules, injections, liquid preparations, ointments, suppositories, patches, eye drops, and enema.

The pharmaceutical composition of the present invention contains the compound represented by the formula (I) or a pharmacologically acceptable salt thereof as an active ingredient.

The pharmaceutical composition of the present invention is prepared using the compound represented by the formula (I) or a pharmacologically acceptable salt thereof and at least one pharmaceutical additive. These pharmaceutical compositions can also be prepared by being appropriately mixed or diluted with or dissolved in an appropriate pharmaceutical additives such as an excipient, a disintegrant, a binder, a lubricant, a diluent, a buffer, an isotonic agent, a preservative, a wetting agent, an emulsifier, a dispersant, a stabilizer, or a solubilizing agent by a pharmaceutically known method depending on the dosage form.

In a case where the pharmaceutical composition of the present invention is used in treatments, the dosage of the compound represented by the formula (I) or a pharmacologically acceptable salt thereof is appropriately determined depending on the age, gender, body weight, degree of disorders and treatment, or the like of a patient. The daily dosage may be administered once or twice, three times, or four times separately.

In the case of oral administration, the dosage for adults can be determined within, for example, a range of 0.1 to 5,000 mg/day. In one embodiment, the oral dosage can also be determined within a range of 1 to 1500 mg/day and is preferably within a range of 1 to 500 mg/day and more preferably within a range of 10 to 500 mg/day.

In the case of parenteral administration, the dosage for adults can be determined as, for example, 0.01 to 5000 mg/day. In one embodiment, the parenteral dosage can also be determined within a range of 0.1 to 1500 mg/day and is preferably within a range of 0.1 to 500 mg/day and more preferably within a range of 1 to 500 mg/day.

In one embodiment, the pharmaceutical composition of the present invention can also be used in combination with a medicine other than TSHR antagonists. Examples of other medicines that can be used in combination in the treatment of thyroid-related diseases include antithyroid drugs (for example, thiamazole, propylthiouracil, and the like), inorganic iodine, lithium carbonate, thyroid hormone formulations, and the like.

In the case of using the compound represented by the formula (I) or a pharmacologically acceptable salt thereof in combination with a different medicine, it is possible to administer a formulation containing these active ingredients together or formulations obtained by separately formulating these active ingredients. In a case where the active ingredients are separately formulated, the formulations can be administered separately or simultaneously. In addition, the dosage of the compound represented by the formula (I) or a pharmacologically acceptable salt thereof may be reduced as appropriate depending on the dosage of the different medicine used in combination.

The compound represented by the formula (I) may be used after being appropriately converted into a prodrug. For example, the prodrug of the compound represented by the formula (I) can be manufactured by introducing a group configuring the prodrug using a prodrug-forming reagent such as a corresponding halide and performing purification. Examples of the group configuring the prodrug include groups described in "Pharmaceutical research and development" (Hirokawa Shoten, 1990), Vol. 7, pp. 163 to 198.

### [Examples]

Hereinafter, the present invention will be described in more detail based on reference examples, examples, and test examples, but the present invention is not limited to contents thereof.

The names of compounds described in the following reference examples or examples were named using ChemDraw Professional (PerkinElmer), MarvinSketch (ChemAxon), and the like except for commercially available reagents.

### Reference Example A-1

### 2-Fluoro-1-(2-fluorobenzyl)-3-nitrobenzene

Pd(PPh₃)₄ (0.065 g) was added to a mixture of 2-(2-fluoro-3-nitrophenyl)-4,4,5,5-tetramethyl-1,3,2-dioxaborolane (0.300 g), 2-fluorobenzyl bromide (0.234 g), a 2 mol/L sodium carbonate aqueous solution (1.12 mL), and DME (2 mL) at room temperature under an argon atmosphere. The reaction mixture was stirred at 100°C for one hour under microwave irradiation. A saturated ammonium chloride aqueous solution was added to the reaction mixture, and the mixture was extracted with DCM. The extract was concentrated under reduced pressure. The residue was purified by silica gel column chromatography (eluent: n-hexane/ethyl acetate = 95/5 to 70/30), and the title compound (0.240 g) was obtained.

### Reference Example A-2

### 2-Fluoro-3-(2-fluorobenzyl)aniline

A mixture of Reference Example A-1 (0.240 g), 10% Pd/C (0.050 g), methanol (2 mL), and THF (2 mL) was stirred at room temperature for two hours under a hydrogen atmosphere. The reaction mixture was filtered through Celite, and the filtrate was concentrated under reduced pressure. The residue was purified by silica gel column chromatography (eluent: n-hexane/ethyl acetate = 90/10 to 60/40), and the title compound (0.210 g) was obtained.

### Reference Example A-3

### 4-Bromo-2-fluoro-3-(2-fluorobenzyl)aniline

NBS (0.188 g) was added to a mixture of Reference Example A-2 (0.210 g) and DCM (3 mL) under ice cooling. The reaction mixture was stirred under ice cooling for one hour. A saturated sodium bicarbonate aqueous solution and a 1 mol/L sodium thiosulfate aqueous solution were added to the reaction mixture, and the mixture was extracted with DCM. The extract was concentrated under reduced pressure. The residue was purified by silica gel column chromatography (eluent: n-hexane/ethyl acetate = 95/5 to 70/30), and the title compound (0.210 g) was obtained.
¹H-NMR (CDCl₃) δ ppm : 2.64-4.63 (4H, m), 6.62 (1H, t, J=8.9 Hz), 6.84-7.10 (3H, m), 7.11-7.22 (2H, m)

### Reference Example A-4

### tert-Butyl (4-bromo-2-fluoro-3-(2-fluorobenzyl)phenyl)carbamate

Boc₂O (0.338 g) was added to a mixture of Reference Example A-3 (0.210 g), TEA (0.214 g), DMAP (0.017 g), and THF (2 mL) under water cooling. The reaction mixture was stirred at room temperature for two hours. A saturated ammonium chloride aqueous solution and water were added to the reaction mixture, and the mixture was extracted with ethyl acetate. The extract was dried over anhydrous magnesium sulfate and concentrated under reduced pressure.

A mixture of the residue, potassium carbonate (0.292 g), and methanol (2 mL) was stirred at 50°C for 30 minutes. The reaction mixture was cooled to room temperature, water was added thereto, and the mixture was extracted with DCM. The extract was concentrated under reduced pressure. The residue was purified by silica gel column chromatography (eluent: n-hexane/ethyl acetate = 95/5 to 70/30), and the title compound (0.170 g) was obtained.

### Reference Example A-5

### tert-Butyl (2-fluoro-3-(2-fluorobenzyl)-4-(4,4,5,5-tetramethyl-1,3,2-dioxaborolan-2-yl)phenyl)carbamate

Pd(dppf)Cl₂·DCM (0.031 g) was added to a mixture of Reference Example A-4 (0.170 g), bis(pinacolato)diboron (0.141 g), potassium acetate (0.126 g), and 1,4-dioxane (2 mL) at room temperature. The reaction mixture was refluxed for three hours. The reaction mixture was cooled to room temperature, a saturated ammonium chloride aqueous solution and water were added thereto, and the mixture was extracted with DCM. The extract was concentrated under reduced pressure. The residue was purified by silica gel column chromatography (eluent: n-hexane/ethyl acetate = 98/2 to 80/20), and the title compound (0.160 g) was obtained.

### Reference Example A-6

### 2-(2-Fluoro-3-nitrobenzyl)benzonitrile

Pd(PPh₃)₄ (0.065 g) was added to a mixture of 2-(2-fluoro-3-nitrophenyl)-4,4,5,5-tetramethyl-1,3,2-dioxaborolane (0.300 g), 2-(bromomethyl)benzonitrile (0.242 g), a 2 mol/L sodium carbonate aqueous solution (1.12 mL), and DME (2 mL) at room temperature under an argon atmosphere. The reaction mixture was stirred at 100°C for one hour under microwave irradiation. A saturated ammonium chloride aqueous solution was added to the reaction mixture, and the mixture was extracted with DCM. The extract was concentrated under reduced pressure. The residue was purified by Method A (eluent: n-hexane/ethyl acetate = 95/5 to 70/30), and the title compound (0.240 g) was obtained.

### Reference Example A-7

### 2-(3-Amino-2-fluorobenzyl)benzonitrile

A mixture of Reference Example A-6 (0.240 g), 10% Pd/C (0.050 g), methanol (2 mL), and THF (2 mL) was stirred at room temperature for two hours under a hydrogen atmosphere. The reaction mixture was filtered through Celite, and the filtrate was concentrated under reduced pressure. The residue was purified by Method A (eluent: n-hexane/ethyl acetate = 90/10 to 60/40), and the title compound (0.210 g) was obtained.

### Reference Example A-8

### 2-(3-Amino-6-bromo-2-fluorobenzyl)benzonitrile

NBS (0.198 g) was added to a mixture of Reference Example A-7 (0.210 g) and DCM (3 mL). The reaction mixture was stirred under ice cooling for one hour. A saturated sodium bicarbonate aqueous solution and a 1 mol/L sodium thiosulfate aqueous solution were added to the reaction mixture, and the mixture was extracted with DCM. The extract was concentrated under reduced pressure. The residue was purified by Method A (eluent: n-hexane/ethyl acetate = 95/5 to 70/30), and the title compound (0.180 g) was obtained.
¹H-NMR (CDCl₃) δ ppm : 3.78 (2H, br), 4.35-4.44 (2H, m), 6.61-6.70 (1H, m), 7.02 (1H, d, J=7.8 Hz), 7.19 (1H, dd, J=1.5, 8.5 Hz), 7.25-7.35 (1H, m), 7.40-7.49 (1H, m), 7.61-7.72 (1H, m)

### Reference Example A-9

### tert-Butyl (4-bromo-3-(2-cyanobenzyl)-2-fluorophenyl)carbamate

Boc₂O (0.283 g) was added to a mixture of Reference Example A-8 (0.180 g), TEA (0.179 g), DMAP (0.014 g), and THF (2 mL) under water cooling. The reaction mixture was stirred at room temperature for two hours. A saturated ammonium chloride aqueous solution and water were added to the reaction mixture, and the mixture was extracted with ethyl acetate. The extract was washed with 1 mol/L hydrochloric acid and a saturated saline solution, then, dried over anhydrous sodium sulfate, and concentrated under reduced pressure.

A mixture of the residue, potassium carbonate (0.245 g), and methanol (2 mL) was stirred at room temperature for one hour and then at 50°C for 30 minutes. The reaction mixture was cooled to room temperature, water was added thereto, and the mixture was extracted with DCM. The extract was concentrated under reduced pressure. The residue was purified by Method A (eluent: n-hexane/ethyl acetate = 95/5 to 70/30), and the title compound (0.180 g) was obtained.

Reference Example A-10 was synthesized by the same method as in Reference Example A-5 except that Reference Example A-9 was used in place of Reference Example A-4.

### Reference Example A-11

### 3-Fluoro-2-(2-fluoro-3-nitrobenzyl)benzonitrile

Pd(PPh₃)₄ (0.087 g) was added to a mixture of 2-(2-fluoro-3-nitrophenyl)-4,4,5,5-tetramethyl-1,3,2-dioxaborolane (0.400 g), 2-(bromomethyl)-3-fluorobenzonitrile (0.321 g), sodium carbonate (0.317 g), water (3 mL), and DME (8 mL) at room temperature. The reaction mixture was stirred at 100°C for one hour under microwave irradiation. The reaction mixture was refluxed overnight. The reaction mixture was cooled to room temperature, a saturated ammonium chloride aqueous solution was added thereto, and the mixture was extracted with ethyl acetate. The extract was washed with a saturated saline solution, then, dried over anhydrous sodium sulfate, and concentrated under reduced pressure. The residue was purified by Method A (eluent: n-hexane/ethyl acetate = 100/0 to 85/15), and the title compound (0.083 g) was obtained.

### Reference Example A-12

### 2-(3-Amino-2-fluorobenzyl)-3-fluorobenzonitrile

A mixture of Reference Example A-11 (0.276 g), 10% Pd/C (0.083 g), methanol (1.5 mL), and THF (1.5 mL) was stirred at room temperature for seven hours under a hydrogen atmosphere. The reaction mixture was filtered through Celite, and the filtrate was concentrated under reduced pressure. The residue was purified by silica gel column chromatography (eluent: n-hexane/ethyl acetate = 100/0 to 80/20), and the title compound (0.234 g) was obtained.

### Reference Example A-13

### 2-(3-Amino-6-bromo-2-fluorobenzyl)-3-fluorobenzonitrile

NBS (0.170 g) was added to a mixture of Reference Example A-12 (0.233 g) and DCM (6 mL) at -45°C. The reaction mixture was stirred for two hours while being slowly warmed up to room temperature. A saturated sodium bicarbonate aqueous solution and a 1 mol/L sodium thiosulfate aqueous solution were added to the reaction mixture, and the mixture was extracted with DCM. The extract was concentrated under reduced pressure. The residue was purified by silica gel column chromatography (eluent: n-hexane/ethyl acetate = 100/0 to 80/20), and the title compound (0.261 g) was obtained.
¹H-NMR (CDCl₃) δ ppm : 3.71 (2H, br), 4.37 (2H, s), 6.56-6.65 (1H, m), 7.11-7.17 (1H, m), 7.19-7.25 (1H, m), 7.28-7.37 (1H, m), 7.43-7.49 (1H, m)

### Reference Example A-14

### tert-Butyl (4-bromo-3-(2-cyano-6-fluorobenzyl)-2-fluorophenyl)carbamate

Boc₂O (0.423 g) was added to a mixture of Reference Example A-13 (0.261 g), TEA (0.245 g), DMAP (0.020 g), and THF (4 mL). The reaction mixture was stirred at the same temperature for four hours. DMAP (0.049 g) and Boc₂O (0.176 g) were added to the reaction mixture at room temperature. The reaction mixture was stirred at 50°C for one hour. The reaction mixture was cooled to room temperature, a saturated ammonium chloride aqueous solution and water were added to the reaction mixture, and the mixture was extracted with ethyl acetate. The extract was washed with 1 mol/L hydrochloric acid and a saturated saline solution, then, dried over anhydrous sodium sulfate and concentrated under reduced pressure.

A mixture of the residue, potassium carbonate (0.335 g), and methanol (4 mL) was stirred at 50°C for 30 minutes. The reaction mixture was cooled to room temperature, water was added thereto, and the mixture was extracted with DCM. The extract was concentrated under reduced pressure. The residue was purified by silica gel column chromatography (eluent: n-hexane/ethyl acetate = 100/0 to 90/10), and the title compound (0.270 g) was obtained.

### Reference Example A-15

### 1-Methylcyclopropyl (4-bromo-2-fluoro-3-(2-fluorobenzyl)phenyl)carbamate

NaHMDS (1 mol/L in THF) (1.62 mL) was slowly added to a mixture of Reference Example A-3 (0.161 g), Reference Example I-1 (0.385 g), and THF (5 mL) under ice cooling. The reaction mixture was stirred under ice cooling for one hour. A saturated saline solution and water were added to the reaction mixture, and the mixture was extracted with ethyl acetate. The extract was dried over anhydrous sodium sulfate and concentrated under reduced pressure. The residue was purified by silica gel column chromatography (eluent: n-hexane/ethyl acetate = 100/0 to 70/30), and the title compound (0.136 g) was obtained.

### Reference Example A-16

### 1-Methylcyclopropyl (2-fluoro-3-(2-fluorobenzyl)-4-(4,4,5,5-tetramethyl-1,3,2-dioxaborolan-2-yl)phenyl)carbamate

Pd(dppf)Cl₂·DCM (0.028 g) was added to a mixture of Reference Example A-15 (0.136 g), bis(pinacolato)diboron (0.130 g), potassium acetate (0.101 g), and 1,4-dioxane (5 mL) at room temperature. The reaction mixture was refluxed for five hours. The reaction mixture was cooled to room temperature, a saturated sodium bicarbonate aqueous solution and water were added thereto, and the mixture was extracted with ethyl acetate. The extract was washed with a saturated saline solution, then, dried over anhydrous sodium sulfate, and concentrated under reduced pressure. The residue was purified by silica gel column chromatography (eluent: n-hexane/ethyl acetate = 98/2 to 85/15), and the title compound (0.109 g) was obtained.

### Reference Example A-17

### 3-Benzyl-2-fluoroaniline

Pd(dppf)Cl₂·DCM (0.645 g) was added to a mixture of 3-bromo-2-fluoroanilne (3.00 g), potassium benzyltrifluoroborate (6.25 g), cesium carbonate (10.3 g), water (6 mL), and 1,4-dioxane (60 mL) at room temperature under an argon atmosphere. The reaction mixture was stirred at 100°C for three hours. The reaction mixture was cooled to room temperature, a saturated ammonium chloride aqueous solution and water were added thereto, and the mixture was extracted with DCM. The extract was concentrated under reduced pressure. The residue was purified by silica gel column chromatography (eluent: n-hexane/ethyl acetate = 95/5 to 70/30), and the title compound (2.44 g) was obtained.

Reference Example A-18 was synthesized in the same method as in Reference Example A-3 except that Reference Example A-17 was used in place of Reference Example A-2.
¹H-NMR (CDCl₃) δ ppm : 3.72 (2H, br), 4.14 (2H, d, J=2.8 Hz), 6.48-6.68 (1H, m), 6.90-7.54 (6H, m)

### Reference Example A-19

### tert-Butyl (3-benzyl-4-bromo-2-fluorophenyl)carbamate

Boc₂O (3.09 g) was added to a mixture of Reference Example A-18 (1.80 g), TEA (1.95 g), DMAP (0.157 g), and DCM (20 mL) under water cooling. The reaction mixture was stirred at room temperature for one hour. A saturated ammonium chloride aqueous solution and water were added to the reaction mixture, and the mixture was extracted with DCM. The extract was concentrated under reduced pressure.

A mixture of the residue, potassium carbonate (2.66 g), and methanol (10 mL) was stirred at 50°C for five hours. The reaction mixture was cooled to room temperature, water was added thereto, and the mixture was extracted with ethyl acetate. The extract was washed with a saturated saline solution, then, dried over anhydrous sodium sulfate, and concentrated under reduced pressure. The residue was purified by silica gel column chromatography (eluent: n-hexane/ethyl acetate = 98/2 to 85/15), and the title compound (1.60 g) was obtained.

### Reference Example A-20

### tert-Butyl (3-benzyl-2-fluoro-4-(4,4,5,5-tetramethyl-1,3,2-dioxaborolan-2-yl)phenyl)carbamate

Pd(dppf)Cl₂·DCM (0.308 g) was added to a mixture of Reference Example A-19 (1.60 g), bis(pinacolato)diboron (1.60 g), potassium acetate (1.24 g), and 1,4-dioxane (10 mL) at room temperature. The reaction mixture was refluxed for three hours. The reaction mixture was cooled to room temperature, and a saturated ammonium chloride aqueous solution, water, and ethyl acetate were added thereto. The mixture was stirred at the same temperature for 10 minutes and then filtered through Celite. The filtrate was extracted with ethyl acetate. The extract was washed with a saturated saline solution, then, dried over anhydrous sodium sulfate, and concentrated under reduced pressure. The residue was purified by silica gel column chromatography (eluent: n-hexane/ethyl acetate = 98/2 to 85/15), and the title compound (1.58 g) was obtained.

### Reference Example A-21

### tert-Butyl (3-benzoyl-4-bromophenyl)carbamate

Phenylmagnesium bromide (3 mol/L in Et₂O) (4.78 mL) was added to a mixture of 2-bromo-5-nitrobenzaldehyde (3.00 g) and THF (60 mL) under ice cooling. The reaction mixture was stirred for two hours under ice cooling. A saturated ammonium chloride aqueous solution and water were added to the reaction mixture, and the mixture was extracted with ethyl acetate. The extract was washed with a saturated saline solution, then, dried over anhydrous sodium sulfate, and concentrated under reduced pressure. The residue was purified by silica gel column chromatography (eluent: n-hexane/ethyl acetate = 90/10 to 60/40), and (2-bromo-5-nitrophenyl)(phenyl)methanol was obtained.

Dess-Martin periodinane (4.09 g) was added to a mixture of the obtained compound and DCM (30 mL) under ice cooling. The reaction mixture was stirred at room temperature for one hour. A saturated sodium bicarbonate aqueous solution and a 1 mol/L sodium thiosulfate aqueous solution were added to the reaction mixture, and the mixture was extracted with DCM. The extract was concentrated under reduced pressure. The residue was purified by Method A (eluent: n-hexane/ethyl acetate = 90/10 to 60/40), and (2-bromo-5-nitrophenyl)(phenyl)methanone was obtained.

An iron powder (1.78 g) and 2 mol/L hydrochloric acid (23.9 mL) were added to a mixture of the obtained compound and methanol (20 mL) under ice cooling. The reaction mixture was stirred at 85°C for three hours. The reaction mixture was cooled to room temperature and filtered through Celite. A 2 mol/L sodium hydroxide aqueous solution (25 mL) and ethyl acetate were added to the filtrate, and the mixture was filtered through Celite. The filtrate was extracted with ethyl acetate. The extract was washed with a saturated saline solution, then, dried over anhydrous sodium sulfate, and concentrated under reduced pressure. The residue was purified by silica gel column chromatography (eluent: n-hexane/ethyl acetate = 90/10 to 60/40), and (5-amino-2-bromophenyl)(phenyl)methanone was obtained.

Boc₂O (1.90 g) was added to a mixture of the obtained compound, TEA (1.47 g), DMAP (0.088 g), and DCM (20 mL) at room temperature. The reaction mixture was stirred at the same temperature for 15 hours. A saturated ammonium chloride aqueous solution and water were added to the reaction mixture, and the mixture was extracted with DCM. The extract was concentrated under reduced pressure.

A mixture of the residue, potassium carbonate (1.00 g), and methanol (20 mL) was stirred at 50°C for two hours. The reaction mixture was cooled to room temperature, and insoluble matter was collected. The filtrate was concentrated under reduced pressure. The residue was purified by silica gel column chromatography (eluent: n-hexane/ethyl acetate = 90/10 to 70/30), and the title compound (2.00 g) was obtained.

### Reference Example A-22

### tert-Butyl (4-bromo-3-(1-hydroxy-1-phenylethyl)phenyl)carbamate

Methylmagnesium bromide (3 mol/L in Et₂O) (1.40 mL) was added to a mixture of Reference Example A-21 (1.05 g) and THF (10 mL) under ice cooling. The reaction mixture was stirred at room temperature for six hours. Methylmagnesium bromide (3 mol/L in Et₂O) (1.40 mL) was added to the reaction mixture under ice cooling. The reaction mixture was stirred at 40°C for one hour. A saturated ammonium chloride aqueous solution and water were added to the reaction mixture under ice cooling, and the mixture was extracted with ethyl acetate. The extract was washed with a saturated saline solution, then, dried over anhydrous sodium sulfate, and concentrated under reduced pressure. The residue was purified by silica gel column chromatography (eluent: n-hexane/ethyl acetate = 90/10 to 60/40), and the title compound (1.15 g) was obtained.

### Reference Example A-23

### tert-Butyl (4-bromo-3-(1-phenylvinyl)phenyl)carbamate

Triethylsilane (0.971 g) and TFA (0.381 g) were added to a mixture of Reference Example A-22 (1.15 g) and DCM (10 mL) under ice cooling. The reaction mixture was stirred at room temperature for five days. A saturated sodium bicarbonate aqueous solution was added to the reaction mixture, and the mixture was extracted with DCM. The extract was concentrated under reduced pressure. The residue was purified by silica gel column chromatography (eluent: n-hexane/ethyl acetate = 95/5 to 60/40), and the title compound (0.640 g) was obtained.

### Reference Example A-24

### tert-Butyl (3-(1-phenylvinyl)-4-(4,4,5,5-tetramethyl-1,3,2-dioxaborolan-2-yl)phenyl)carbamate

Pd(PPh₃)₄ (0.031 g) was added to a mixture of Reference Example A-23 (0.200 g), bis(pinacolato)diboron (0.156 g), cesium fluoride (0.191 g), and THF (20 mL) at room temperature under an argon atmosphere. The reaction mixture was stirred at 110°C for three hours under microwave irradiation. The reaction mixture was poured into water, and the mixture was extracted with Et₂O. The extract was washed with a saturated saline solution, then, dried over anhydrous sodium sulfate, and concentrated under reduced pressure. The residue was purified by silica gel column chromatography (eluent: n-hexane/ethyl acetate = 100/0 to 90/10), and the title compound (0.088 g) was obtained.

### Reference Example A-25

### tert-Butyl (3-(1-phenylethyl)-4-(4,4,5,5-tetramethyl-1,3,2-dioxaborolan-2-yl)phenyl)carbamate

10% Pd/C (0.016 g) was added to a mixture of Reference Example A-24 (0.035 g) and ethyl acetate (1 mL) at room temperature under an argon atmosphere. The reaction mixture was stirred at room temperature for one hour under a hydrogen atmosphere. The reaction mixture was filtered through Celite, and the filtrate was concentrated under reduced pressure. The residue was purified by amino silica gel column chromatography (eluent: n-hexane/ethyl acetate = 100/0 to 85/15), and the title compound (0.022 g) was obtained.

### Reference Example A-26

### 1-(2-Fluorobenzyl)-2-methyl-3-nitrobenzene

Pd(PPh₃)₄ (0.132 g) was added to a mixture of 4,4,5,5-tetramethyl-2-(2-methyl-3-nitrophenyl)-1,3,2-dioxaborolane (0.600 g), 1-(bromomethyl)-2-fluorobenzene (0.474 g), a 2 mol/L sodium carbonate aqueous solution (2.28 mL), and DME (6 mL) at room temperature under an argon atmosphere. The reaction mixture was stirred at 100°C for one hour under microwave irradiation. A saturated ammonium chloride aqueous solution and water were added to the reaction mixture, and the mixture was extracted with ethyl acetate. The extract was washed with a saturated saline solution, then, dried over anhydrous sodium sulfate, and concentrated under reduced pressure. The residue was purified by Method A (eluent: n-hexane/ethyl acetate = 95/5 to 70/30), and the title compound (0.590 g) was obtained.

### Reference Example A-27

### 3-(2-Fluorobenzyl)-2-methylaniline

A mixture of Reference Example A-26 (0.590 g), 10% Pd/C (0.050 g), methanol (5 mL), and THF (5 mL) was stirred at room temperature for four hours and then at 50°C for four hours under a hydrogen atmosphere. The reaction mixture was cooled to room temperature and left to stand for 62 hours. The reaction mixture was filtered through Celite, the filtrate was concentrated under reduced pressure. The residue was purified by Method A (eluent: n-hexane/ethyl acetate = 95/5 to 70/30), and the title compound (0.450 g) was obtained.

Reference Example A-28 was synthesized by the same method as in Reference Example A-8 except that Reference Example A-27 was used in place of Reference Example A-7.
¹H-NMR (CDCl₃) δ ppm : 2.02 (3H, s), 3.62 (2H, br), 4.24 (2H, s), 6.56 (1H, d, J=8.4 Hz), 6.68-6.79 (1H, m), 6.92-6.99 (1H, m), 7.01-7.09 (1H, m), 7.12-7.21 (1H, m), 7.29 (1H, d, J=8.4 Hz)

### Reference Example A-29

### tert-Butyl (4-bromo-3-(2-fluorobenzyl)-2-methylphenyl)carbamate

Boc₂O (0.816 g) was added to a mixture of Reference Example A-28 (0.500 g), TEA (0.516 g), DMAP (0.021 g), and THF (3 mL) under water cooling. The reaction mixture was stirred at room temperature for 1,5 hours. A saturated ammonium chloride aqueous solution and water were added to the reaction mixture, and the mixture was extracted with ethyl acetate. The extract was washed with 0.5 mol/L hydrochloric acid and a saturated saline solution, then, dried over anhydrous sodium sulfate, and concentrated under reduced pressure.

A mixture of the residue, potassium carbonate (0.705 g), and methanol (3 mL) was stirred at 50°C for one hour. The reaction mixture was cooled to room temperature, a saturated ammonium chloride aqueous solution and water were added thereto, and the mixture was extracted with DCM. The extract was concentrated under reduced pressure. The residue was purified by Method A (eluent: n-hexane/ethyl acetate = 98/2 to 80/20), and the title compound (0.530 g) was obtained.

### Reference Example A-30

### tert-Butyl (3-(2-fluorobenzyl)-2-methyl-4-(4,4,5,5-tetramethyl-1,3,2-dioxaborolan-2-yl)phenyl)carbamate

Pd(dppf)Cl₂·DCM (0.220 g) was added to a mixture of Reference Example A-29 (0.530 g), bis(pinacolato)diboron (0.512 g), potassium acetate (0.396 g), and 1,4-dioxane (3 mL) at room temperature. The reaction mixture was refluxed for two hours. The reaction mixture was cooled to room temperature, and a saturated sodium bicarbonate aqueous solution and ethyl acetate were added thereto. The mixture was stirred at the same temperature for 10 minutes and then filtered through Celite. The filtrate was extracted with ethyl acetate. The extract was washed with a saturated saline solution, then, dried over anhydrous sodium sulfate, and concentrated under reduced pressure. The residue was purified by silica gel column chromatography (eluent: n-hexane/ethyl acetate = 98/2 to 85/15), and the title compound (0.260 g) was obtained.

### Reference Example A-31

### 2-(2,4-Difluoro-3-nitrophenyl)-4,4,5,5-tetramethyl-1,3,2-dioxaborolane

Pd(dppf)Cl₂·DCM (0.854 g) was added to a mixture of 1-bromo-2,4-difluoro-3-nitrobenzene (2.49 g), bis(pinacolato)diboron (3.99 g), potassium acetate (3.08 g), and 1,4-dioxane (30 mL) at room temperature. The reaction mixture was refluxed for 2.5 hours. The reaction mixture was cooled to room temperature, a saturated sodium bicarbonate aqueous solution, water, and ethyl acetate were added thereto. The mixture was stirred at the same temperature for 10 minutes and then filtered through Celite. The filtrate was extracted with ethyl acetate. The extract was washed with a saturated saline solution, then, dried over anhydrous sodium sulfate, and concentrated under reduced pressure. The residue was purified by silica gel column chromatography (eluent: n-hexane/ethyl acetate = 90/10 to 40/60), and the title compound (0.860 g) was obtained.

Reference Example A-32 was synthesized by the same method as in Reference Example A-6 except that Reference Example A-31 and 1-(bromomethyl)-2-fluorobenzene were used in place of 2-(2-fluoro-3-nitrophenyl)-4,4,5,5-tetramethyl-1,3,2-dioxaborolane and 2-(bromomethyl)benzonitrile.

Reference Example A-33 was synthesized by the same method as in Reference Example A-7 except that Reference Example A-32 was used in place of Reference Example A-6.

Reference Example A-34 was synthesized by the same method as in Reference Example A-8 except that Reference Example A-33 was used in place of Reference Example A-7.
¹H-NMR (CDCl₃) δ ppm : 3.75 (2H, br), 4.07-4.22 (2H, m), 6.87-6.96 (1H, m), 6.97-7.08 (2H, m), 7.12 (1H, dd, J=2.1, 9.9 Hz), 7.15-7.23 (1H, m)

### Reference Example A-35

### tert-Butyl (4-bromo-2,6-difluoro-3-(2-fluorobenzyl)phenyl)carbamate

Boc₂O (0.349 g) was added to a mixture of Reference Example A-34 (0.230 g), TEA (0.221 g), DMAP (0.009 g), and THF (3 mL) under water cooling. The reaction mixture was stirred at room temperature for one hour and then at 50°C for four hours. The reaction mixture was cooled to room temperature and stirred for 12 hours. A saturated ammonium chloride aqueous solution and water were added to the reaction mixture, and the mixture was extracted with ethyl acetate. The extract was washed with 0.5 mol/L hydrochloric acid and a saturated saline solution, then, dried over anhydrous sodium sulfate, and concentrated under reduced pressure.

A mixture of the residue, potassium carbonate (0.302 g), and methanol (3 mL) was stirred at 50°C for one hour. The reaction mixture was cooled to room temperature, a saturated ammonium chloride aqueous solution and water were added thereto, and the mixture was extracted with DCM. The extract was concentrated under reduced pressure. The residue was purified by Method A (eluent: n-hexane/ethyl acetate = 95/5 to 70/30), and the title compound (0.280 g) was obtained.

### Reference Example A-36

### tert-Butyl (2,6-difluoro-3-(2-fluorobenzyl)-4-(4,4,5,5-tetramethyl-1,3,2-dioxaborolan-2-yl)phenyl)carbamate

Pd(dppf)Cl₂·DCM (0.110 g) was added to a mixture of Reference Example A-35 (0.280 g), bis(pinacolato)diboron (0.256 g), potassium acetate (0.198 g), and 1,4-dioxane (2 mL) at room temperature. The reaction mixture was refluxed for two hours. The reaction mixture was cooled to room temperature, a saturated sodium hydrogen aqueous solution and water were added thereto, and the mixture was extracted with DCM. The extract was concentrated under reduced pressure. The residue was purified by silica gel column chromatography (eluent: n-hexane/ethyl acetate = 98/2 to 85/15), and the title compound (0.270 g) was obtained.

Reference Example A-37 was synthesized by the same method as in Reference Example A-1 except that 3-fluorobenzyl bromide was used in place of 2 fluorobenzyl bromide.

Reference Example A-38 was synthesized by the same method as in Reference Example A-2 except that Reference Example A-37 was used in place of Reference Example A-1.

Reference Example A-39 was synthesized by the same method as in Reference Example A-3 except that Reference Example A-38 was used in place of Reference Example A-2.
¹H-NMR (CDCl₃) δ ppm : 2.80-4.56 (4H, m), 6.54-6.67 (1H, m), 6.83-7.10 (3H, m), 7.15 (1H, dd, J=1.8, 8.5 Hz), 7.18-7.34 (1H, m)

Reference Example A-40 was synthesized by the same method as in Reference Example A-4 except that Reference Example A-39 was used in place of Reference Example A-3.

Reference Example A-41 was synthesized by the same method as in Reference Example A-5 except that Reference Example A-40 was used in place of Reference Example A-4.

Reference Example A-42 was synthesized by the same method as in Reference Example A-1 except that 4-fluorobenzyl bromide was used in place of 2 fluorobenzyl bromide.

Reference Example A-43 was synthesized by the same method as in Reference Example A-2 except that Reference Example A-42 was used in place of Reference Example A-1.

Reference Example A-44 was synthesized by the same method as in Reference Example A-3 except that Reference Example A-43 was used in place of Reference Example A-2.
¹H-NMR (CDCl₃) δ ppm : 2.94-4.27 (4H, m), 6.54-6.64 (1H, m), 6.90-7.02 (2H, m), 7.14 (1H, dd, J=1.6, 8.7 Hz), 7.18-7.29 (2H, m)

Reference Example A-45 was synthesized by the same method as in Reference Example A-4 except that Reference Example A-44 was used in place of Reference Example A-3.

Reference Example A-46 was synthesized by the same method as in Reference Example A-5 except that Reference Example A-45 was used in place of Reference Example A-4.

### Reference Example A-47

### 3-(((tert-Butyldimethylsilyl)oxy)methyl)-2-fluoroaniline

tert-Butyldimethylsilyl chloride (0.851 g) was added to a mixture of (2-fluoro-3-nitrophenyl)methanol (0.920 g), imidazole (0.439 g), and DCM (10 mL) at room temperature. The reaction mixture was stirred at the same temperature for one hour. The reaction mixture was poured into a saturated ammonium chloride aqueous solution, and the mixture was extracted with ethyl acetate. The extract was washed with water and a saturated saline solution, then, dried over anhydrous magnesium sulfate, and concentrated under reduced pressure.

A mixture of the residue, 10% Pd/C (0.200 g), methanol (5 mL), and THF (5 mL) was stirred at room temperature for four hours under a hydrogen atmosphere. The reaction mixture was filtered through Celite, and the filtrate was concentrated under reduced pressure. The residue was purified by silica gel column chromatography (eluent: n-hexane/ethyl acetate = 98/2 to 80/20), and the title compound (1.20 g) was obtained.

### Reference Example A-48

### tert-Butyl (4-bromo-3-(((tert-butyldimethylsilyl)oxy)methyl)-2-fluorophenyl)carbamate

NBS (0.753 g) was added to a mixture of Reference Example A-47 (1.20 g) and DCM (10 mL) at -20°C. The reaction mixture was stirred at the same temperature for 20 minutes. A saturated sodium bicarbonate aqueous solution and a 1 mol/L sodium thiosulfate aqueous solution were added to the reaction mixture, and the mixture was extracted with DCM. The extract was concentrated under reduced pressure.

Boc₂O (2.26 g) was added to a mixture of the residue, TEA (1.43 g), DMAP (0.057 g), and THF (10 mL) under water cooling. The reaction mixture was stirred at room temperature for 13 hours. A saturated ammonium chloride aqueous solution and water were added to the reaction mixture, and the mixture was extracted with ethyl acetate. The extract was dried over anhydrous magnesium sulfate and concentrated under reduced pressure.

A mixture of the residue, potassium carbonate (1.95 g), and methanol (5 mL) was stirred at room temperature for five hours. Water was added to the reaction mixture, and the mixture was extracted with DCM. The extract was concentrated under reduced pressure. The residue was purified by silica gel column chromatography (eluent: n-hexane/ethyl acetate = 90/10 to 75/25), and the title compound (1.31 g) was obtained.
¹H-NMR (CDCl₃) δ ppm : 0.11 (6H, s), 0.91 (9H, s), 1.52 (9H, s), 4.81 (2H, d, J=2.5 Hz), 6.69 (1H, br), 7.30 (1H, dd, J=1.6, 8.9 Hz), 7.85-8.04 (1H, m)

### Reference Example A-49

### tert-Butyl (3-(((tert-butyldimethylsilyl)oxy)methyl)-2-fluoro-4-(4,4,5,5-tetramethyl-1,3,2-dioxaborolan-2-yl)phenyl)carbamate

Potassium acetate (0.339 g) was added to a mixture of Reference Example A-48 (0.500 g), bis(pinacolato)diboron (0.380 g), Pd(dppf)Cl₂·DCM (0.042 g), and 1,4-dioxane (2 mL) at room temperature. The reaction mixture was refluxed for three hours. The reaction mixture was cooled to room temperature, a saturated ammonium chloride aqueous solution and water were added thereto, and the mixture was extracted with DCM. The extract was concentrated under reduced pressure. The residue was purified by silica gel column chromatography (eluent: n-hexane/ethyl acetate = 98/2 to 90/10), and the title compound (0.400 g) was obtained.

### Reference Example A-50

### 1-(Benzyloxy)-2-methylpropan-2-yl (4-bromo-2-fluoro-3-(2-fluorobenzyl)phenyl)carbamate

NaHMDS (1 mol/L in THF) (0.791 mL) was slowly added to a mixture of Reference Example A-3 (0.118 g), Reference Example I-2 (0.151 g), and THF (4 mL) under ice cooling. The reaction mixture was stirred under ice cooling for one hour. A saturated saline solution and water were added to the reaction mixture, and the mixture was extracted with ethyl acetate. The extract was dried over anhydrous sodium sulfate and concentrated under reduced pressure. The residue was purified by Method A (eluent: n-hexane/ethyl acetate = 100/0 to 86/14), and the title compound (0.200 g) was obtained.

### Reference Example A-51

### 1-(Benzyloxy)-2-methylpropan-2-yl (2-fluoro-3-(2-fluorobenzyl)-4-(4,4,5,5-tetramethyl-1,3,2-dioxaborolan-2-yl)phenyl)carbamate

A mixture of Reference Example A-50 (0.102 g), bis(pinacolato)diboron (0.065 g), potassium acetate (0.058 g), Pd(dppf)Cl₂·DCM (0.016 g), and 1,4-dioxane (1 mL) was stirred at 120°C for 1.5 hours under microwave irradiation. A saturated ammonium chloride aqueous solution and water were added the reaction mixture, and the mixture was extracted with DCM. The extract was dried over anhydrous sodium sulfate and concentrated under reduced pressure. The residue was purified by silica gel column chromatography (eluent: n-hexane/ethyl acetate = 100/0 to 89/11), and the title compound (0.078 g) was obtained.

### Reference Example A-52

### 2-Fluoro-1-(2-(methylsulfonyl)benzyl)-3-nitrobenzene

1-(bromomethyl)-2-(methylsulfonyl)benzene (0.089 g) was added to a mixture of 4,4,5,5-tetramethyl-2-(2-fluoro-3-nitrophenyl)-1,3,2-dioxaborolane (0.095 g), Pd(PPh₃)₄ (0.021 g), a 2 mol/L sodium carbonate aqueous solution (0.356 mL), and DME (2 mL) at room temperature under an argon atmosphere. The reaction mixture was stirred at 110°C for one hour under microwave irradiation. A saturated ammonium chloride aqueous solution was added to the reaction mixture, and the mixture was extracted with DCM. The extract was concentrated under reduced pressure. The residue was purified by silica gel column chromatography (eluent: n-hexane/ethyl acetate = 90/10 to 40/60), and the title compound (0.095 g) was obtained.

### Reference Example A-53

### 4-Bromo-2-fluoro-3-(2-(methylsulfonyl)benzyl)aniline

A mixture of Reference Example A-52 (0.095 g), 10% Pd/C (0.020 g), methanol (1 mL), and THF (1 mL) was stirred at room temperature for one hour under a hydrogen atmosphere. The reaction mixture was filtered through Celite, and the filtrate was concentrated under reduced pressure.

NBS (0.046 g) was added to a mixture of the residue, THF (0.5 mL), and DCM (1 mL) at -20°C. The reaction mixture was stirred for 30 minutes at the same temperature. A saturated sodium bicarbonate aqueous solution and a 1 mol/L sodium thiosulfate aqueous solution were added to the reaction mixture, and the mixture was extracted with DCM. The extract was concentrated under reduced pressure. The residue was purified by silica gel column chromatography (eluent: n-hexane/ethyl acetate = 90/10 to 45/55), and the title compound (0.072 g) was obtained.
¹H-NMR (CDCl₃) δ ppm : 3.24 (3H, s), 3.77 (2H, br), 4.60 (2H, d, J=2.3 Hz), 6.65-6.74 (1H, m), 6.84-6.94 (1H, m), 7.22 (1H, dd, J=1.5, 8.5 Hz), 7.37-7.55 (2H, m), 8.11 (1H, dd, J=1.6, 7.7 Hz)

### Reference Example A-54

### tert-Butyl (4-bromo-2-fluoro-3-(2-(methylsulfonyl)benzyl)phenyl)carbamate

Boc₂O (0.097 g) was added to a mixture of Reference Example A-53 (0.072 g), TEA (0.061 g), DMAP (0.003 g), and THF (1 mL) under water cooling. The reaction mixture was stirred at room temperature for 16 hours. A saturated ammonium chloride aqueous solution and water were added to the reaction mixture, and the mixture was extracted with ethyl acetate. The extract was dried over anhydrous magnesium sulfate and concentrated under reduced pressure.

A mixture of the residue, potassium carbonate (0.083 g), and methanol (1 mL) was stirred at room temperature for two hours. Water was added to the reaction mixture, and the mixture was extracted with DCM. The extract was concentrated under reduced pressure. The residue was purified by silica gel column chromatography (eluent: n-hexane/ethyl acetate = 90/10 to 70/30), and the title compound (0.045 g) was obtained.

### Reference Example A-55

### tert-Butyl (2-fluoro-3-(2-(methylsulfonyl)benzyl)-4-(4,4,5,5-tetramethyl-1,3,2-dioxaborolan-2-yl)phenyl)carbamate

Potassium acetate (0.029 g) was added to a mixture of Reference Example A-54 (0.045 g), bis(pinacolato)diboron (0.037 g), Pd(dppf)Cl₂·DCM (0.004 g), and 1,4-dioxane (2 mL) at room temperature. The reaction mixture was stirred at 120°C for one hour under microwave irradiation. A saturated ammonium chloride aqueous solution and water were added to the reaction mixture, and the mixture was extracted with DCM. The extract was concentrated under reduced pressure. The residue was purified by silica gel column chromatography (eluent: n-hexane/ethyl acetate = 90/10 to 60/40), and the title compound (0.027 g) was obtained.

### Reference Example A-56

### [1,1'-Bi(cyclopropane)]-1-yl (4-bromo-2-fluoro-3-(2-fluorobenzyl)phenyl)carbamate

NaHMDS (1 mol/L in THF) (1.34 mL) was slowly added to a mixture of Reference Example A-3 (0.200 g), Reference Example I-6 (0.229 g), and THF (5 mL) under ice cooling. The reaction mixture was stirred under ice cooling for one hour. Reference Example I-6 (0.050 g) and NaHMDS (1 mol/L in THF) (1.34 mL) were added to the reaction mixture under ice cooling. The reaction mixture was stirred for one hour under ice cooling. A saturated saline solution and water were added to the reaction mixture, and the mixture was extracted with ethyl acetate. The extract was dried over anhydrous sodium sulfate and concentrated under reduced pressure. The residue was purified by silica gel column chromatography (eluent: n-hexane/ethyl acetate = 100/0 to 70/30), and the title compound (0.193 g) was obtained.

Reference Example A-57 was synthesized by the same method as in Reference Example A-16 except that Reference Example A-56 was used in place of Reference Example A-15.

### Reference Example A-58

### tert-Butyl (4-bromo-2-fluoro-3-(hydroxymethyl)phenyl)carbamate

TBAF (1 mol/L in THF) (9.75 mL) was slowly added to a mixture of Reference Example A-48 (3.85 g) and THF (20 mL) under ice cooling. The reaction mixture was stirred at room temperature for one hour. A saturated ammonium chloride aqueous solution and water were added to the reaction mixture, and the mixture was extracted with ethyl acetate. The extract was washed with water and a saturated saline solution, then, dried over anhydrous sodium sulfate, and concentrated under reduced pressure. The residue was purified by silica gel column chromatography (eluent: n-hexane/ethyl acetate = 95/5 to 70/30), and the title compound (2.42 g) was obtained.

### Reference Example A-59

### tert-Butyl (4-bromo-2-fluoro-3-formylphenyl)carbamate

Dess-Martin periodinane (0.795 g) was added to a mixture of Reference Example A-58 (0.500 g) and DCM (5 mL) under ice cooling. The reaction mixture was stirred at room temperature for 1.5 hours. A saturated sodium bicarbonate aqueous solution and a 1 mol/L sodium thiosulfate aqueous solution were added to the reaction mixture, and the mixture was extracted with DCM. The extract was concentrated under reduced pressure. The residue was purified by silica gel column chromatography (eluent: n-hexane/ethyl acetate = 95/5 to 70/30), and the title compound (0.470 g) was obtained.

### Reference Example A-60

### tert-Butyl (4-bromo-2-fluoro-3-(hydroxy(o-tolyl)methyl)phenyl)carbamate

Isopropylmagnesium chloride (2 mol/L in THF) (0.377 mL) was added to a mixture of 1-iodo-2-methylbenzene (0.181 g), and THF (1 mL) at -40°C. The reaction mixture was stirred under ice cooling for 30 minutes. A THF (1 mL) solution of Reference Example A-59 (0.150 g) was added to the reaction mixture at -40°C. The reaction mixture was stirred at the same temperature for 10 minutes and then for two hours under ice cooling. A saturated ammonium chloride aqueous solution and water were added to the reaction mixture, and the mixture was extracted with DCM. The extract was concentrated under reduced pressure. The residue was purified by Method A (eluent: n-hexane/ethyl acetate = 95/5 to 60/40), and the title compound (0.090 g) was obtained.

### Reference Example A-61

### tert-Butyl (4-bromo-2-fluoro-3-(2-methylbenzoyl)phenyl)carbamate

Dess-Martin periodinane (0.112 g) was added to a mixture of Reference Example A-60 (0.090 g) and DCM (1 mL) under ice cooling. The reaction mixture was stirred at room temperature for one hour. Dess-Martin periodinane (0.056 g) was added to the reaction mixture at room temperature. The reaction mixture was stirred at the same temperature for 30 minutes. A saturated sodium bicarbonate aqueous solution and a 1 mol/L sodium thiosulfate aqueous solution were added to the reaction mixture, and the mixture was extracted with DCM. The extract was concentrated under reduced pressure. The residue was purified by Method A (eluent: n-hexane/ethyl acetate = 98/2 to 85/15), and the title compound (0.040 g) was obtained.

Reference Example A-62 was synthesized by the same method as in Reference Example A-36 except that Reference Example A-61 was used in place of Reference Example A-35.

Reference Example A-63 was synthesized by the same method as in Reference Example A-60 except that 1-iodo-2-(trifluoromethyl)benzene was used in place of 1-iodo-2-methylbenzene.

### Reference Example A-64

### tert-Butyl (4-bromo-2-fluoro-3-(2-(trifluoromethyl)benzoyl)phenyl)carbamate

Dess-Martin periodinane (0.724 g) was added to a mixture of Reference Example A-63 (0.660 g) and DCM (3 mL) under ice cooling. The reaction mixture was stirred at room temperature for one hour. A saturated sodium bicarbonate aqueous solution and a 1 mol/L sodium thiosulfate aqueous solution were added to the reaction mixture, and the mixture was extracted with DCM. The extract was concentrated under reduced pressure. The residue was purified by Method A (eluent: n-hexane/ethyl acetate = 98/2 to 85/15), and the title compound (0.610 g) was obtained.

Reference Example A-65 was synthesized by the same method as in Reference Example A-5 except that Reference Example A-64 was used in place of Reference Example A-4.

Reference Example A-66 was synthesized by the same method as in Reference Example A-60 except that 1-fluoro-2-iodobenzene was used in place of 1-iodo-2-methylbenzene.

Reference Example A-67 was synthesized by the same method as in Reference Example A-64 except that Reference Example A-66 was used in place of Reference Example A-63.

Reference Example A-68 was synthesized by the same method as in Reference Example A-5 except that Reference Example A-67 was used in place of Reference Example A-4.

### Reference Example A-69

### Potassium 2-(3-fluoropyridin-2-yl)acetate

A mixture of 2,3-difluoropyridine (2.00 g), cesium carbonate (13.6 g), diethyl malonate (6.68 g), and DMSO (20 mL) was stirred at 100°C for 4.5 hours. Water was added to the reaction mixture under ice cooling, and the mixture was extracted with n-hexane/ethyl acetate. The extract was washed with water and a saturated saline solution, then, dried over anhydrous sodium sulfate, and concentrated under reduced pressure. The residue was purified by silica gel column chromatography (eluent: n-hexane/ethyl acetate = 95/5 to 70/30), and diethyl 2-(3-fluoropyridin-2-yl)malonate was obtained.

A mixture of the obtained compound, sodium chloride (0.831 g), water (0.466 mL), and DMSO (20 mL) was stirred at 140°C for 10 hours, at room temperature for 14 hours, and then at 140°C for eight hours, and then cooled to room temperature. n-Hexane and ethyl acetate were added to the reaction mixture. The mixture was washed with water and a saturated saline solution, then, dried over anhydrous sodium sulfate, and concentrated under reduced pressure. The residue was purified by silica gel column chromatography (eluent: n-hexane/ethyl acetate = 80/20 to 50/50), and ethyl 2-(3-fluoropyridin-2-yl)acetate was obtained.

A solution of potassium tert-butoxide (1.71 g) in ethanol (10 mL) was added to a mixture of the obtained compound, water (0.274 mL), and ethanol (20 mL) at 60°C over 30 minutes. The reaction mixture was stirred at the same temperature for two hours. The reaction mixture was concentrated under reduced pressure. Et₂O (20 mL) was added to the residue, and insoluble matter was collected. The resulting solid was sequentially washed with Et₂O/ethanol (1/1) (10 mL) and Et₂O (30 mL) and then dried under reduced pressure, thereby obtaining the title compound (2.15 g).

### Reference Example A-70

### 3-Fluoro-2-(2-fluoro-3-nitrobenzyl)pyridine

tris(Dibenzylideneacetone)dipalladium(0) (0.042 g) was added to a mixture of 1-bromo-2-fluoro-3-nitrobenzene (0.500 g), Reference Example A-69 (0.527 g), Xantphos (0.079 g), and 2-methoxyethyl ether (4 mL) at room temperature under an argon atmosphere. The reaction mixture was stirred at 150°C for one hour under microwave irradiation. The reaction mixture was filtered through Celite, and the filtrate was partitioned between water and ethyl acetate. The organic layer was separated, washed with water and a saturated saline solution, then, dried over anhydrous sodium sulfate, and concentrated under reduced pressure. The residue was purified by amino silica gel column chromatography (eluent: n-hexane/ethyl acetate = 95/5 to 70/30), and the title compound (0.440 g) was obtained.

Reference Example A-71 was synthesized by the same method as in Reference Example A-7 except that Reference Example A-70 was used in place of Reference Example A-6.

Reference Example A-72 was synthesized by the same method as in Reference Example A-8 except that Reference Example A-71 was used in place of Reference Example A-7.
¹H-NMR (CDCl₃) δ ppm : 3.74 (2H, br), 4.31-4.44 (2H, m), 6.56-6.68 (1H, m), 7.10-7.22 (2H, m), 7.31-7.43 (1H, m), 8.25-8.40 (1H, m)

Reference Example A-73 was synthesized by the same method as in Reference Example A-29 except that Reference Example A-72 was used in place of Reference Example A-28.

### Reference Example A-74

### tert-Butyl (2-fluoro-3-((3-fluoropyridin-2-yl)methyl)-4-(4,4,5,5-tetramethyl-1,3,2-dioxaborolan-2-yl)phenyl)carbamate

Pd(dppf)Cl₂·DCM (0.045 g) was added to a mixture of Reference Example A-73 (0.220 g), bis(pinacolato)diboron (0.210 g), potassium acetate (0.162 g), and 1,4-dioxane (2 mL) at room temperature. The reaction mixture was refluxed for four hours. Pd(dppf)Cl₂·DCM (0.045 g) was added to the reaction mixture at room temperature. The reaction mixture was refluxed for 2.5 hours. The reaction mixture was cooled to room temperature and purified by silica gel column chromatography (eluent: n-hexane/ethyl acetate = 90/10 to 60/40), and the title compound (0.160 g) was obtained.

### Reference Example A-75

### 4-Bromo-2-fluoro-3-(morpholinomethyl)aniline

Morpholine (0.083 g) was added to a mixture of 1-(chloromethyl)-2-fluoro-3-nitrobenzene (0.090 g) and THF (1 mL) at room temperature. The reaction mixture was refluxed for six hours. The reaction mixture was cooled to room temperature, water was added thereto, and the mixture was extracted with DCM. The extract was concentrated under reduced pressure. The residue was purified by silica gel column chromatography (eluent: n-hexane/ethyl acetate = 80/20 to 10/90), and 4-(2-fluoro-3-nitrobenzyl)morpholine was obtained.

A mixture of the obtained compound, 10% Pd/C (0.050 g), methanol (2 mL), and THF (2 mL) was stirred at room temperature for three hours under a hydrogen atmosphere. The reaction mixture was filtered through Celite, and the filtrate was concentrated under reduced pressure.

NBS (0.052 g) was added to a mixture of the residue and DCM (3 mL) under ice cooling. The reaction mixture was stirred for one hour under ice cooling. A saturated sodium bicarbonate aqueous solution and a 1 mol/L sodium thiosulfate aqueous solution were added to the reaction mixture, and the mixture was extracted with DCM. The extract was concentrated under reduced pressure. The residue was purified by silica gel column chromatography (eluent: n-hexane/ethyl acetate = 95/5 to 70/30), and the title compound (0.051 g) was obtained.
¹H-NMR (CDCl₃) δ ppm : 2.43-2.68 (4H, m), 3.58-3.84 (8H, m), 6.56-6.69 (1H, m), 7.14 (1H, dd, J=1.5, 8.5 Hz)

Reference Example A-76 was synthesized by the same method as in Reference Example A-54 except that Reference Example A-75 was used in place of Reference Example A-53.

### Reference Example A-77

### tert-Butyl (2-fluoro-3-(morpholinomethyl)-4-(4,4,5,5-tetramethyl-1,3,2-dioxaborolan-2-yl)phenyl)carbamate

Potassium acetate (0.045 g) was added to a mixture of Reference Example A-76 (0.060 g), bis(pinacolato)diboron (0.051 g), Pd(dppf)Cl₂·DCM (0.011 g), and 1,4-dioxane (2 mL) at room temperature. The reaction mixture was refluxed for three hours. The reaction mixture was cooled to room temperature, a saturated ammonium chloride aqueous solution and water were added thereto, and the mixture was extracted with DCM. The extract was concentrated under reduced pressure. The residue was purified by silica gel column chromatography (eluent: n-hexane/ethyl acetate/methanol = 50/50/0 to 0/100/0 to 0/80/20), and the title compound (0.030 g) was obtained.

### Reference Example A-78

### (2-Fluoro-3-nitrophenyl)(morpholino)methanone

T3P (1.7 mol/L in ethyl acetate) (1.91 mL) was added to a mixture of 2-fluoro-3-nitrobenzoic acid (0.300 g), morpholine (0.141 g), DIPEA (0.419 g), and DCM (5 mL) at room temperature. The reaction mixture was stirred at the same temperature for three hours. Water was poured into the reaction mixture, and the mixture was extracted with DCM. The extract was concentrated under reduced pressure. The residue was purified by silica gel column chromatography (eluent: n-hexane/ethyl acetate = 40/60 to 0/100), and the title compound (0.380 g) was obtained.

### Reference Example A-79

### (3-Amino-2-fluorophenyl)(morpholino)methanone

A mixture of Reference Example A-78 (0.380 g), 10% Pd/C (0.100 g), methanol (4 mL), and THF (4 mL) was stirred at room temperature for three hours under a hydrogen atmosphere. The reaction mixture was filtered through Celite, and the filtrate was concentrated under reduced pressure. The residue was purified by amino silica gel column chromatography (eluent: n-hexane/ethyl acetate = 40/60 to 0/100), and the title compound (0.300 g) was obtained.

Reference Example A-80 was synthesized by the same method as in Reference Example A-48 except that Reference Example A-79 was used in place of Reference Example A-47.
¹H-NMR (CDCl₃) δ ppm : 1.53 (9H, s), 3.19-3.36 (2H, m), 3.57-3.94 (6H, m), 6.69 (1H, br), 7.34 (1H, dd, J=1.5, 9.0 Hz), 7.98-8.14 (1H, m)

Reference Example A-81 was synthesized by the same method as in Reference Example A-55 except that Reference Example A-80 was used in place of Reference Example A-54.

### Reference Example A-82

### 1-Allyl-2-fluoro-3-nitrobenzene

A mixture of 3-bromo-2-fluoronitrobenzene (0.515 g), Pd(PPh₃)₄ (0.541 g), allyltrin-butyltin (0.929 g), and toluene (10 mL) was stirred at 130°C for 30 minutes under microwave irradiation. The reaction mixture was concentrated under reduced pressure. The residue was purified by Method A (eluent: n-hexane/ethyl acetate = 100/0 to 80/20), and the title compound (0.321 g) was obtained.

### Reference Example A-83

### 1-(Cyclopropylmethyl)-2-fluoro-3-nitrobenzene

Under an argon atmosphere, a solution of TFA (1.08 g) in DCM (20 mL) was slowly added to a mixture of diethylzinc (1 mol/L in n-hexane) (9.49 mL) and DCM (20 mL) under ice cooling. The reaction mixture was stirred under ice cooling for 20 minutes. A solution of diiodomethane (2.54 g) in DCM (20 mL) was slowly added to the reaction mixture under ice cooling. The reaction mixture was stirred under ice cooling for 20 minutes. A solution of Reference Example A-82 (0.215 g) in DCM (10 mL) was slowly added to the reaction mixture under ice cooling. The reaction mixture was stirred at room temperature for 1.5 hours. 1 mol/L hydrochloric acid was added to the reaction mixture under ice cooling, and the mixture was extracted with DCM. The extract was washed with a saturated saline solution, dried over anhydrous sodium sulfate, and concentrated under reduced pressure. The residue was purified by silica gel column chromatography (eluent: n-hexane/ethyl acetate = 100/0 to 90/10), and the title compound (0.166 g) was obtained.

### Reference Example A-84

### 4-Bromo-3-(cyclopropylmethyl)-2-fluoroaniline

Ammonium chloride (0.142 g) was added to a mixture of Reference Example A-83 (0.130 g), a zinc powder (0.261 g), and methanol (5 mL) under ice cooling. The reaction mixture was stirred for 30 hours under ice cooling. The reaction mixture was filtered through Celite, and the filtrate was concentrated under reduced pressure. The residue was purified by silica gel column chromatography (eluent: n-hexane/ethyl acetate = 100/0 to 90/10), and 3-(cyclopropylmethyl)-2-fluoroaniline was obtained.

A solution of NBS (0.044 g) in DMF (0.5 mL) was slowly added to a mixture of the obtained compound and DMF (1 mL) under ice cooling. The reaction mixture was stirred under ice cooling for 30 minutes. Water was added to the reaction mixture, and the mixture was extracted with n-hexane/ethyl acetate (3/1). The extract was washed with a saturated saline solution, then, dried over anhydrous sodium sulfate, and concentrated under reduced pressure. The residue was purified by silica gel column chromatography (eluent: n-hexane/ethyl acetate = 100/0 to 97/3), and the title compound (0.052 g) was obtained. ¹H-NMR (CDCl₃) δ ppm : 0.26-0.36 (2H, m), 0.37-0.50 (2H, m), 1.02-1.15 (1H, m), 2.70 (2H, dd, J=2.6, 7.0 Hz), 3.70 (2H, br), 6.49-6.61 (1H, m), 7.08-7.18 (1H, m)

### Reference Example A-85

### tert-Butyl (4-bromo-3-(cyclopropylmethyl)-2-fluorophenyl)carbamate

TEA (0.065 g), DMAP (0.003 g), and Boc₂O (0.121 g) were added to a mixture of Reference Example A-84 (0.052 g) and THF (2 mL). The reaction mixture was refluxed for three hours. DMAP (0.003 g) and Boc₂O (0.121 g) were added to the reaction mixture at room temperature. The reaction mixture was refluxed for one hour. The reaction mixture was cooled to room temperature, a saturated ammonium chloride aqueous solution and water were added to the reaction mixture, and the mixture was extracted with ethyl acetate. The extract was washed with 1 mol/L hydrochloric acid and a saturated saline solution, then, dried over anhydrous sodium sulfate, and concentrated under reduced pressure.

A mixture of the residue, potassium carbonate (0.088 g), and methanol (2 mL) was stirred at 60°C for 30 minutes. The reaction mixture was cooled to room temperature, water was added thereto, and the mixture was extracted with ethyl acetate. The extract was washed with a saturated saline solution, then, dried over anhydrous sodium sulfate, and concentrated under reduced pressure. The residue was purified by silica gel column chromatography (eluent: n-hexane/ethyl acetate = 100/0 to 95/5), and the title compound (0.058 g) was obtained.

### Reference Example A-86

### tert-Butyl (3-(cyclopropylmethyl)-2-fluoro-4-(4,4,5,5-tetramethyl-1,3,2-dioxaborolan-2-yl)phenyl)carbamate

Pd(dppf)Cl₂·DCM (0.022 g) was added to a mixture of Reference Example A-85 (0.046 g), bis(pinacolato)diboron (0.068 g), potassium acetate (0.046 g), and 1,4-dioxane (4 mL) at room temperature. The reaction mixture was stirred at 100°C for six hours. The reaction mixture was cooled to room temperature, a saturated ammonium chloride aqueous solution, water, and DCM were added thereto. The mixture was stirred at the same temperature for 10 minutes and then filtered through Celite. The filtrate was extracted with DCM, and the extract was concentrated under reduced pressure. The residue was purified by silica gel column chromatography (eluent: n-hexane/ethyl acetate = 100/0 to 95/5), and the title compound (0.025 g) was obtained.

### Reference Example B-1

### 1,2-Difluoro-4-nitro-3-phenoxybenzene

Under an argon atmosphere, sodium tert-butoxide (1.63 g) was added to a mixture of phenol (1.59 g) and THF (34 mL) under ice cooling. The reaction mixture was stirred under ice cooling for 15 minutes. 2,3,4-Trifluoronitrobenzene (3.00 g) was slowly added to the reaction mixture under ice cooling. The reaction mixture was stirred at room temperature for one hour. The reaction mixture was poured into a saturated ammonium chloride aqueous solution under ice cooling, and the mixture was extracted with ethyl acetate. The extract was washed with water and a saturated saline solution, then, dried over anhydrous sodium sulfate, and concentrated under reduced pressure, thereby obtaining the title compound (4.45 g).

### Reference Example B-2

### 2-Fluoro-4-nitro-3-phenoxyaniline

A mixture of Reference B-1 (4.38 g) and 15 mol/L ammonia water (24 mL) was stirred at 130°C for 30 minutes under microwave irradiation. Insoluble matter was collected and washed with water. The obtained solid was dried under reduced pressure, thereby obtaining the title compound (3.93 g).

### Reference Example B-3

### tert-Butyl (2-fluoro-4-nitro-3-phenoxyphenyl)carbamate

Boc₂O (5.52 g) was added to a mixture of Reference Example B-2 (3.92 g), TEA (4.79 g), DMAP (1.93 g), and THF (40 mL) under ice cooling. The reaction mixture was stirred at room temperature for nine hours. A saturated ammonium chloride aqueous solution and water were added to the reaction mixture, and the mixture was extracted with ethyl acetate. The extract was washed with 1 mol/L hydrochloric acid and a saturated saline solution, then, dried over anhydrous sodium sulfate, and concentrated under reduced pressure.

Potassium carbonate (6.56 g) was added to a mixture of the residue and methanol (40 mL) at room temperature. The reaction mixture was stirred at 60°C for 30 minutes. The reaction mixture was cooled to room temperature, water was added thereto, and the mixture was extracted with DCM. The extract was washed with water, then, dried over anhydrous sodium sulfate, and concentrated under reduced pressure. The residue was purified by silica gel column chromatography (eluent: n-hexane/ethyl acetate = 100/0 to 84/16), and the title compound (4.39 g) was obtained.

### Reference Example B-4

### tert-Butyl (4-amino-2-fluoro-3-phenoxyphenyl)carbamate

10% Pd/C (0.131 g) was added to a mixture of Reference Example B-3 (4.38 g) and methanol (42 mL) at room temperature under an argon atmosphere. The reaction mixture was stirred at the same temperature for 2.5 hours under a hydrogen atmosphere. The reaction mixture was filtered through Celite, and the filtrate was concentrated under reduced pressure. The residue was purified by silica gel column chromatography (eluent: n-hexane/ethyl acetate = 95/5 to 75/25), and the title compound (3.55 g) was obtained.

### Reference Example B-5

### tert-Butyl (4-bromo-2-fluoro-3-phenoxyphenyl)carbamate

tert-Butyl nitrite (1.21 g) was slowly added to a mixture of Reference Example B-4 (3.55 g), copper (II) bromide (2.62 g), and MeCN (50 mL) at room temperature. The reaction mixture was stirred at 77 °C for one hour. The reaction mixture was cooled to room temperature, 1 mol/L hydrochloric acid was added thereto, and the mixture was extracted with ethyl acetate. The extract was washed with a saturated saline solution, then, dried over anhydrous sodium sulfate, and concentrated under reduced pressure. The residue was purified by silica gel column chromatography (eluent: n-hexane/ethyl acetate = 100/0 to 92/8), and the title compound (3.50 g) was obtained.

### Reference Example B-6

### tert-Butyl (2,3-difluoro-4-nitrophenyl)carbamate

Diphenyl phosphoryl azide (0.407 g) was added to a mixture of 2,3-difluoro-4-nitrobenzoic acid (0.200 g), tert-butyl alcohol (0.365 g), TEA (0.149 g), and toluene (3 mL) at room temperature. The reaction mixture was refluxed for three hours. The reaction mixture was cooled to room temperature and concentrated under reduced pressure. The residue was purified by silica gel column chromatography (eluent: n-hexane/ethyl acetate = 90/10 to 75/25), and the title compound (0.044 g) was obtained.

### Reference Example B-7

### tert-Butyl (4-amino-2-fluoro-3-(2-fluorophenoxy)phenyl)carbamate

Sodium tert-butoxide (0.100 g) was added to a mixture of 2-fluorophenol (0.117 g) and THF (1 mL) under ice cooling. The reaction mixture was stirred for 30 minutes under ice cooling. A mixture of Reference Example B-6 (0.260 g) and THF (2 mL) was slowly added to the reaction mixture under ice cooling. The reaction mixture was stirred at room temperature for 13 hours. The reaction mixture was poured into a saturated ammonium chloride aqueous solution, and the mixture was extracted with DCM. The extract was concentrated under reduced pressure.

A mixture of the residue, 10% Pd/C (0.010 g), methanol (2 mL), and THF (2 mL) was stirred at room temperature for two hours under a hydrogen atmosphere. The reaction mixture was filtered through Celite, and the filtrate was concentrated under reduced pressure. The residue was purified by amino silica gel column chromatography (eluent: n-hexane/ethyl acetate = 95/5 to 65/35), and the title compound (0.240 g) was obtained.

### Reference Example B-8

### tert-Butyl (4-bromo-2-fluoro-3-(2-fluorophenoxy)phenyl)carbamate

tert-Butyl nitrite (0.085 g) was slowly added to a mixture of Reference Example B-7 (0.230 g), copper (II) bromide (0.153 g), and MeCN (3 mL) at room temperature. The reaction mixture was stirred at 70 °C for one hour. The reaction mixture was cooled to room temperature, 1 mol/L hydrochloric acid was added thereto, and the mixture was extracted with ethyl acetate. The extract was washed with a saturated saline solution, then, dried over anhydrous magnesium sulfate, and concentrated under reduced pressure. The residue was purified by silica gel column chromatography (eluent: n-hexane/ethyl acetate = 100/0 to 70/30), and the title compound (0.200 g) was obtained.

### Reference Example B-9

### tert-Butyl (2-fluoro-3-(2-fluorophenoxy)-4-(4,4,5,5-tetramethyl-1,3,2-dioxaborolan-2-yl)phenyl)carbamate

Potassium acetate (0.147 g) was added to a mixture of Reference Example B-8 (0.200 g), bis(pinacolato)diboron (0.165 g), Pd(dppf)Cl₂·DCM (0.018 g), and 1,4-dioxane (3 mL) at room temperature. The reaction mixture was stirred at 135°C for one hour under microwave irradiation. A saturated ammonium chloride aqueous solution and water were added to the reaction mixture, and the mixture was extracted with DCM. The extract was concentrated under reduced pressure. The residue was purified by silica gel column chromatography (eluent: n-hexane/ethyl acetate = 90/10 to 55/45), and the title compound (0.034 g) was obtained.

### Reference Example B-10

### Methyl 4-bromo-3-phenoxybenzoate

A mixture of methyl 4-bromo-3-hydroxybenzoate (2.00 g), phenylboronic acid (2.11 g), copper (II) acetate (1.73 g), TEA (4.38 g), a molecular sieve 4A (10.0 g), and DCM (80 mL) was stirred at room temperature for 36 hours. Phenylboronic acid (0.633 g), copper (II) acetate (0.629 g), and TEA (1.40 g) were added to the reaction mixture at room temperature. The reaction mixture was stirred at the same temperature for eight hours. The reaction mixture was filtered through Celite. A saturated ammonium chloride aqueous solution and water were added to the filtrate, and the mixture was extracted with DCM. The extract was concentrated under reduced pressure. The residue was purified by silica gel column chromatography (eluent: n-hexane/ethyl acetate = 95/5 to 60/40), and the title compound (2.30 g) was obtained.

### Reference Example B-11

### 4-Bromo-3-phenoxybenzoic acid

A mixture of Reference Example B-10 (2.30 g), a 4 mol/L lithium hydroxide aqueous solution (5.62 mL), methanol (10 mL), and THF (10 mL) was stirred at room temperature for 18 hours. Water and 2 mol/L hydrochloric acid (15 mL) were added to the reaction mixture under ice cooling. The mixture was stirred at the same temperature for 10 minutes, and insoluble matter was collected. The obtained solid was dried under reduced pressure, thereby obtaining the title compound (1.84 g).

### Reference Example B-12

### tert-Butyl (4-bromo-3-phenoxyphenyl)carbamate

A mixture of Reference Example B-11 (1.83 g), diphenylphosphoryl azide (2.06 g), TEA (1.39 g), and tert-butyl alcohol (30 mL) was refluxed for seven hours. The reaction mixture was concentrated under reduced pressure. The residue was purified by Method A (eluent: n-hexane/ethyl acetate = 90/10 to 60/40), and the title compound (1.71 g) was obtained.

Reference Example B-13 was synthesized by the same method as in Reference Example A-5 except that Reference Example B-12 was used in place of Reference Example A-4.

### Reference Example C-1

### 1-((3-Bromopyridin-2-yl)methyl)urea

Trimethylsilyl isocyanate (0.600 g) was added to a mixture of 2-(aminomethyl)-3-bromopyridine hydrochloride (0.388 g), TEA (0.264 g), and THF (4.4 mL) at room temperature. The reaction mixture was stirred at the same temperature for three days. The reaction mixture was purified by Method A (eluent: ethyl acetate/methanol = 98/2 to 75/25), and the title compound (0.306 g) was obtained.

### Reference Example C-2

### 3-Bromo-2-(((tert-butyldimethylsilyl)oxy)methyl)pyridine

Imidazole (0.376 g) and tert-butyldimethylsilyl chloride (0.399 g) were added to a mixture of (3-bromopyridin-2-yl)methanol (0.415 g) and DCM (5 mL) at room temperature. The reaction mixture was stirred at the same temperature for one hour. Water was added to the reaction mixture, and the mixture was extracted with DCM. The extract was concentrated under reduced pressure. The residue was purified by silica gel column chromatography (eluent: n-hexane/ethyl acetate = 100/0 to 90/10), and the title compound (0.530 g) was obtained.

### Reference Example C-3

### 2-(((tert-Butyldimethylsilyl)oxy)methyl)-3-(4,4,5,5-tetramethyl-1,3,2-dioxaborolan-2-yl)pyridine

Pd(dppf)Cl₂·DCM (0.143 g) was added to a mixture of Reference Example C-2 (0.529 g), bis(pinacolato)diboron (0.578 g), potassium acetate (0.515 g), and 1,4-dioxane (10 mL) at room temperature. The reaction mixture was refluxed for two hours. The reaction mixture was cooled to room temperature, a saturated ammonium chloride aqueous solution was added thereto, and the mixture was extracted with ethyl acetate. The extract was washed with a saturated saline solution, then, dried over anhydrous sodium sulfate, and concentrated under reduced pressure. The residue was purified by amino silica gel column chromatography (eluent: n-hexane/ethyl acetate = 100/0 to 50/50), and the title compound (0.297 g) was obtained.

### Reference Example C-4

### 1-((3-Bromo-4-methylpyridin-2-yl)methyl)urea

Trimethylsilyl isocyanate (0.194 g) was added to a mixture of (3-bromo-4-methylpyridin-2-yl)methanamine hydrochloride (0.200 g), TEA (0.085 g), and THF (2 mL) at room temperature. The reaction mixture was stirred at the same temperature for 12 hours. The reaction mixture was purified by silica gel column chromatography (eluent: n-hexane/ethyl acetate/methanol = 50/50/0 to 0/100/0 to 0/90/10), and the title compound (0.048 g) was obtained.

### Reference Example C-5

### 1-((3-Bromo-5-methylpyridin-2-yl)methyl)urea

A mixture of (3-bromo-5-methylpyridin-2-yl)methanamine hydrochloride (0.100 g), DIPEA (0.071 g), and THF (3 mL) was stirred at room temperature for 10 minutes. Trimethylsilyl isocyanate (0.243 g) was added to the reaction mixture at room temperature. The reaction mixture was stirred at the same temperature for one hour. The reaction mixture was concentrated under reduced pressure, and the title compound (0.126 g) was obtained.

### Reference Example C-6

### Ethyl (E)-3-(3-bromopyridin-2-yl)acrylate

Sodium hydride (about 60%) (0.129 g) was added to a mixture of triethyl phosphonoacetate (0.663 g) and THF (10 mL) under ice cooling. The reaction mixture was stirred at room temperature for 30 minutes. 3 Bromopicolinaldehyde (0.500 g) was added to the reaction mixture under ice cooling. The reaction mixture was stirred at room temperature for 10 minutes and then at room temperature for one hour. A saturated ammonium chloride aqueous solution and water were added to the reaction mixture under ice cooling, and the mixture was extracted with ethyl acetate. The extract was washed with a saturated saline solution, then, dried over anhydrous sodium sulfate, and concentrated under reduced pressure. The residue was purified by silica gel column chromatography (eluent: n-hexane/ethyl acetate = 95/5 to 70/30), and the title compound (0.450 g) was obtained.

### Reference Example C-7

### (E)-3-(3-Bromopyridin-2-yl)acrylamide

A 4 mol/L lithium hydroxide aqueous solution (1.32 mL) was added to a mixture of Reference Example C-6 (0.450 g), methanol (3 mL), and THF (3 mL) under ice cooling. The reaction mixture was stirred at room temperature for two hours. Water and 1 mol/L hydrochloric acid (5.5 mL) were added to the reaction mixture, and the mixture was extracted with DCM. The extract was concentrated under reduced pressure.

15 mol/L ammonia water (0.176 mL) was added to a mixture of the residue, DMT-MM (0.730 g), and MeCN (6 mL) at room temperature. The reaction mixture was stirred at the same temperature for one hour. A saturated ammonium chloride aqueous solution was added to the reaction mixture, and the mixture was extracted with DCM. The extract was concentrated under reduced pressure. The residue was sequentially purified by silica gel column chromatography (eluent: n-hexane/ethyl acetate/methanol = 50/50/0 to 0/100/0 to 0/97/3) and amino silica gel column chromatography (eluent: n-hexane/ethyl acetate = 50/50 to 0/100), and the title compound (0.305 g) was obtained.

### Reference Example C-8

### Ethyl (E)-3-(3-(4,4,5,5-tetramethyl-1,3,2-dioxaborolan-2-yl)pyridin-2-yl)acrylate

Pd(dppf)Cl₂·DCM (0.198 g) was added to a mixture of Reference Example C-6 (0.620 g), bis(pinacolato)diboron (0.922 g), potassium acetate (0.713 g), and 1,4-dioxane (5 mL) at room temperature. The reaction mixture was stirred at 90°C for three hours. The reaction mixture was cooled to room temperature, and a saturated sodium bicarbonate aqueous solution, water, and ethyl acetate were added thereto. The mixture was stirred at the same temperature for 10 minutes and then filtered through Celite. The filtrate was extracted with ethyl acetate. The extract was washed with a saturated saline solution, then, dried over anhydrous sodium sulfate, and concentrated under reduced pressure. The residue was purified by silica gel column chromatography (eluent: n-hexane/ethyl acetate = 90/10 to 60/40), and the title compound (0.550 g) was obtained.

### Reference Example C-9

### Ethyl 3-(3-bromopyridin-2-yl)-3-oxopropanoate

CDI (1.69 g) was added to a mixture of 3-bromopyridine-2-carboxylic acid (2.00 g) and THF (10 mL) at room temperature little by little. The reaction mixture was stirred at the same temperature for 21 hours (A liquid). Magnesium (II) chloride (2.36 g) was added to a mixture of ethyl potassium malonate (3.37 g), TEA (3.01 g), and MeCN (40 mL) under ice cooling. The reaction mixture was stirred at room temperature for five hours. The A liquid was added to the reaction mixture under ice cooling. The reaction mixture was stirred at room temperature for 19 hour and then at 90°C for one hour. The reaction mixture was cooled to room temperature and filtered through Celite. The filtrate was concentrated under reduced pressure.

A mixture of the residue, 2 mol/L hydrochloric acid (50 mL), and toluene (50 mL) was stirred at room temperature for 30 minutes. The reaction mixture was extracted with ethyl acetate. The extract was washed with a saturated sodium bicarbonate solution and a saturated saline solution, then, dried over anhydrous magnesium sulfate, and concentrated under reduced pressure. The residue was purified by silica gel column chromatography (eluent: n-hexane/ethyl acetate = 85/15 to 50/50), and the title compound (1.73 g) was obtained.

### Reference Example D-1

### Methyl 4-(benzyloxy)-6-chloropyridazine-3-carboxylate

A mixture of methyl 4,6-dichloropyridazine-3-carboxylate (1.00 g), potassium carbonate (1.34 g), benzyl alcohol (0.575 g), and DMF (10 mL) was stirred at room temperature for one hour and then at 50 °C for six hours. Benzyl alcohol (0.104 g) was added to the reaction mixture at room temperature. The reaction mixture was stirred at the same temperature for 62 hours and then at 50°C for three hours. The reaction mixture was cooled to room temperature, and a saturated ammonium chloride aqueous solution, water, ethyl acetate, and n-hexane were added thereto. The mixture was extracted with ethyl acetate. The extract was washed with water and a saturated saline solution, then, dried over anhydrous sodium sulfate, and concentrated under reduced pressure. The residue was purified by silica gel column chromatography (eluent: n-hexane/ethyl acetate = 95/5 to 60/40), and the title compound (0.340 g) was obtained.
¹H-NMR (CDCl₃) δ ppm : 4.03 (3H, s), 5.26 (2H, s), 7.08 (1H, s), 7.33-7.52 (5H, m)

### Reference Example D-2

### (4-(Benzyloxy)-6-chloropyridazin-3-yl)methanol

Sodium borohydride (0.092 g) was added to a mixture of Reference Example D-1 (0.340 g) and methanol (5 mL) under ice cooling. The reaction mixture was stirred at room temperature for one hour. A saturated ammonium chloride aqueous solution was added to the reaction mixture, and the mixture was extracted with ethyl acetate. The extract was washed with a saturated saline solution, then, dried over anhydrous sodium sulfate, and concentrated under reduced pressure. The residue was purified by Method A (eluent: n-hexane/ethyl acetate/methanol = 59/39/2 to 0/98/2), and the title compound (0.240 g) was obtained.

### Reference Example D-3

### 2-((4-(Benzyloxy)-6-chloropyridazin-3-yl)methyl)isoindoline-1,3-dione

Methanesulfonyl chloride (0.143 g) was added to a mixture of Reference Example D-2 (0.240 g), TEA (0.194 g), and DCM (3 mL) under ice cooling. The reaction mixture was stirred at room temperature for one hour. A saturated sodium bicarbonate aqueous solution and water were added to the reaction mixture, and the mixture was extracted with DCM. The extract was concentrated under reduced pressure.

A mixture of the residue, phthalimide (0.169 g), cesium carbonate (0.624 g), and MeCN (3 mL) was stirred at room temperature for one hour and then at 50°C for two hours. The reaction mixture was cooled to room temperature, a saturated ammonium chloride aqueous solution and water were added thereto, and the mixture was extracted with DCM. The extract was concentrated under reduced pressure. MTBE was added to the residue, and insoluble matter was collected. The obtained solid was washed with water and MTBE, and then dried under reduced pressure, thereby obtaining the title compound (0.290 g).

### Reference Example D-4

### 2-((4-Hydroxypyridazin-3-yl)methyl)isoindoline-1,3-dione hydrochloride

A mixture of Reference Example D-3 (0.290 g), 10% Pd/C (0.050 g), methanol (2 mL), and THF (2 mL) was stirred at 50°C for 10 minutes. The reaction mixture was stirred at room temperature for 13 hours under a hydrogen atmosphere. The reaction mixture was filtered through Celite, and the filtrate was concentrated under reduced pressure. Ethyl acetate was added to the residue, and insoluble matter was collected. The obtained solid was dried under reduced pressure, thereby obtaining the title compound (0.072 g).

### Reference Example D-5

### 2-((4-Chloropyridazin-3-yl)methyl)isoindoline-1,3-dione

A mixture of Reference Example D-4 (0.040 g), phosphorus oxychloride (0.823 g), and DMF (0.53 mL) was stirred at 90°C for one hour. The reaction mixture was concentrated under reduced pressure. A 5 mol/L sodium hydroxide aqueous solution and a saturated sodium bicarbonate aqueous solution were added to the residue, and the mixture was extracted with DCM. The extract was washed with water and a saturated saline solution, then, dried over anhydrous sodium sulfate, and concentrated under reduced pressure. MTBE was added to the residue, and insoluble matter was collected. The obtained solid was dried under reduced pressure, thereby obtaining the title compound (0.027 g).

### Reference Example D-6

### 2-((4-Bromopyridazin-3-yl)methyl)isoindoline-1,3-dione

Phosphorus oxybromide (0.079 g) was added to a mixture of Reference Example D-4 (0.020 g), DIPEA (0.027 g), and toluene (1 mL) at room temperature. The reaction mixture was stirred at 80°C for one hour. Water and a saturated sodium bicarbonate aqueous solution were slowly added to the reaction mixture under ice cooling. The mixture was stirred at room temperature for 10 minutes. DCM and a small amount of methanol were added to the mixture at room temperature. The mixture was stirred at the same temperature for 10 minutes. The mixture was extracted with DCM. The extract was concentrated under reduced pressure. The residue was purified by silica gel column chromatography (eluent: DCM/ethyl acetate = 90/10 to 40/60), and the title compound (0.012 g) was obtained.

### Reference Example E-1

### tert-Butyl (4-(2-(((tert-butyldimethylsilyl)oxy)methyl)pyridin-3-yl)-3-(2-cyano-6-fluorobenzyl)-2-fluorophenyl)carbamate

Pd(PPh₃)₄ (0.031 g) was added to a mixture of Reference Example A-14 (0.056 g), Reference Example C-3 (0.046 g), potassium carbonate (0.055 g), water (0.4 mL), ethanol (0.4 mL), and toluene (2 mL) at room temperature. The reaction mixture was stirred at 100°C for two hours under microwave irradiation. Water was added to the reaction mixture, and the mixture was extracted with DCM. The extract was concentrated under reduced pressure. The residue was purified by silica gel column chromatography (eluent: n-hexane/ethyl acetate = 100/0 to 80/20), and the title compound (0.028 g) was obtained.

### Reference Example E-2

### tert-Butyl (4-(2-(azidomethyl)pyridin-3-yl)-3-(2-cyano-6-fluorobenzyl)-2-fluorophenyl)carbamate

TBAF (1 mol/L in THF) (0.217 mL) was added to a mixture of Reference Example E-1 (0.041 g) and THF (1 mL) under ice cooling. The reaction mixture was stirred at room temperature for five hours. A saturated ammonium chloride aqueous solution was poured into the reaction mixture, and the mixture was extracted with ethyl acetate. The extract was washed with a saturated saline solution, then, dried over anhydrous sodium sulfate, and concentrated under reduced pressure. The residue was purified by silica gel column chromatography (eluent: n-hexane/ethyl acetate = 90/10 to 40/60), and (3-(2-cyano-6-fluorobenzyl)-2-fluoro-4-(2-(hydroxymethyl)pyridin-3-yl)phenyl)carbamate was obtained.

Methanesulfonyl chloride (0.012 g) was added to a mixture of the obtained compound, TEA (0.021 g), and DCM (1 mL) at room temperature. The reaction mixture was stirred at the same temperature for one hour. Water was added to the reaction mixture, and the mixture was extracted with DCM. The extract was concentrated under reduced pressure.

Sodium azide (0.007 g) was added to a mixture of the residue and DMF (1 mL) at room temperature. The reaction mixture was stirred at room temperature for two hours. Water was poured into the reaction mixture, and the mixture was extracted with ethyl acetate. The extract was washed with a saturated saline solution, then, dried over anhydrous sodium sulfate, and concentrated under reduced pressure. The residue was purified by silica gel column chromatography (eluent: n-hexane/ethyl acetate = 100/0 to 70/30), and the title compound (0.028 g) was obtained.

### Reference Example E-3

### tert-Butyl (4-(2-(aminomethyl)pyridin-3-yl)-3-(2-cyano-6-fluorobenzyl)-2-fluorophenyl)carbamate

Triphenylphosphine (0.023 g) was added to a mixture of Reference Example E-2 (0.028 g), water (0.1 mL), and THF (1 mL) at room temperature. The reaction mixture was stirred at 50°C for two hours. The reaction mixture was concentrated under reduced pressure. The residue was purified by amino silica gel column chromatography (eluent: n-hexane/ethyl acetate = 65/35 to 0/100), and the title compound (0.026 g) was obtained.

### Reference Example E-4

### 1-((3-(4-Amino-3-fluoro-2-(2-fluorobenzyl)phenyl)pyridin-2-yl)methyl)urea

A mixture of Example A-1 (0.953 g) and hydrogen chloride (4 mol/L in 1,4-dioxane) (20 mL) was stirred at room temperature for three hours. The reaction mixture was concentrated under reduced pressure. The residue was purified by amino silica gel column chromatography (eluent: ethyl acetate/methanol = 98/2 to 70/30), and the title compound (0.704 g) was obtained.

### Reference Example E-5

### tert-Butyl (3-(((tert-butyldimethylsilyl)oxy)methyl)-2-fluoro-4-(2-(ureidomethyl)pyridin-3-yl)phenyl)carbamate

Pd(PPh₃)₄ (0.091 g) was added to a mixture of Reference Example A-49 (0.190 g), Reference Example C-1 (0.100 g), a 2 mol/L potassium carbonate aqueous solution (0.592 mL), ethanol (0.592 mL), and toluene (3 mL) at room temperature. The reaction mixture was stirred at 110°C for one hour under microwave irradiation. Water was added to the reaction mixture, and the mixture was extracted with DCM. The extract was concentrated under reduced pressure. The residue was purified by silica gel column chromatography (eluent: ethyl acetate/methanol = 100/0 to 85/15), and the title compound (0.161 g) was obtained.

### Reference Example E-6

### tert-Butyl (2-fluoro-3-(hydroxymethyl)-4-(2-(ureidomethyl)pyridin-3-yl)phenyl)carbamate

TBAF (1 mol/L in THF) (0.951 mL) was added to a mixture of Reference Example E-5 (0.160 g) and THF (2 mL) under ice cooling. The reaction mixture was stirred at room temperature for one hour. A saturated ammonium chloride aqueous solution and water were added to the reaction mixture, and the mixture was extracted with THF. The extract was dried over anhydrous sodium sulfate and concentrated under reduced pressure. The residue was purified by Method A (eluent: ethyl acetate/methanol = 100/0 to 80/20), and the title compound (0.246 g) was obtained.

### Reference Example E-7

### tert-Butyl (3-(bromomethyl)-2-fluoro-4-(2-(ureidomethyl)pyridin-3-yl)phenyl)carbamate

Carbon tetrabromide (0.263 g) and triphenylphosphine (0.208 g) were added to a mixture of Reference Example E-6 (0.246 g) and DCM (5 mL) at room temperature. The reaction mixture was stirred at the same temperature for two hours and then refluxed for two hours. The reaction mixture was cooled to room temperature and stirred overnight. The reaction mixture was poured into a saturated ammonium chloride aqueous solution, and the mixture was extracted with ethyl acetate. The extract was washed with a saturated saline solution, then, dried over anhydrous sodium sulfate, and concentrated under reduced pressure. The residue was purified by silica gel column chromatography (eluent: ethyl acetate/methanol = 100/0 to 80/20), and the title compound (0.089 g) was obtained.

### Reference Example E-8

### 1-((3-(4-Amino-2-(2-cyanobenzyl)-3-fluorophenyl)pyridin-2-yl)methyl)urea hydrochloride

A mixture of Example A-2 (0.126 g) and hydrogen chloride (4 mol/L in 1,4-dioxane) (1.2 mL) was stirred at room temperature for two hours. The reaction mixture was concentrated under reduced pressure, and the title compound (0.159 g) was obtained.

Reference Example E-9 was synthesized by the same method as in Reference Example E-4 except that Example E-3 was used in place of Example A-1.

Reference Example E-10 was synthesized by the same method as in Reference Example E-4 except that Example E-8 was used in place of Example A-1.

### Reference Example E-11

### Ethyl (E)-3-(3-(4-((tert-butoxycarbonyl)amino)-3-fluoro-2-((3-fluoropyridin-2-yl)methyl)phenyl)pyridin-2-yl)acrylate

Pd(PPh₃)₄ (0.087 g) was added to a mixture of Reference Example A-73 (0.150 g), Reference Example C-8 (0.114 g), a 2 mol/L potassium carbonate aqueous solution (0.564 mL), ethanol (0.564 mL), and toluene (1.5 mL) at room temperature. The reaction mixture was stirred at 110°C for one hour under microwave irradiation. Water was added to the reaction mixture, and the mixture was extracted with DCM. The extract was concentrated under reduced pressure. The residue was purified by Method A (eluent: n-hexane/ethyl acetate = 95/5 to 50/50), and the title compound (0.072 g) was obtained.

### Reference Example E-12

### 3-(3-(4-Amino-3-fluoro-2-((3-fluoropyridine-2-yl)methyl)phenyl)pyridine-2-yl)propanamide

A 4 mol/L lithium hydroxide aqueous solution (0.109 mL) was added to a mixture of Reference Example E-11 (0.072 g), THF (0.5 mL), and methanol (0.5 mL) at room temperature. The reaction mixture was stirred at the same temperature for one hour. Water and 1 mol/L hydrochloric acid were added to the reaction mixture, and the mixture was extracted with DCM. The extract was concentrated under reduced pressure.

DMT-MM (0.060 g) and 15 mol/L ammonia water (0.015 mL) were added to a mixture of the residue and MeCN (1 mL) at room temperature. The reaction mixture was stirred at the same temperature for 30 minutes. A saturated ammonium chloride aqueous solution was added to the reaction mixture, and the mixture was extracted with DCM. The extract was concentrated under reduced pressure. The residue was purified by Method A (eluent: n-hexane/ethyl acetate/methanol = 49/49/2 to 0/98/2 to 0/86/14), and tert-butyl (E)-(4-(2-(3-amino-3-oxopropan-1-en-1-yl)pyridin-3-yl)-2-fluoro-3-((3-fluoropyridin-2-yl)methyl)phenyl)carbamate was obtained.

A mixture of the obtained compound, 10% Pd/C (0.005 g), methanol (1 mL), and THF (1 mL) was stirred at room temperature for one hour under a hydrogen atmosphere. The reaction mixture was filtered through Celite, and the filtrate was concentrated under reduced pressure.

A mixture of the residue and hydrogen chloride (4 mol/L in 1,4-dioxane) (1 mL) was stirred at 40°C for one hour. The reaction mixture was concentrated under reduced pressure. The residue was purified by amino silica gel column chromatography (eluent: ethyl acetate/methanol = 98/2 to 80/20), and the title compound (0.027 g) was obtained.

### Reference Example F-1

### tert-Butyl (4-(2-(((tert-butyldimethylsilyl)oxy)methyl)pyridin-3-yl)-2-fluoro-3-phenoxyphenyl)carbamate

A mixture of Reference Example B-5 (0.206 g), Reference Example C-3 (0.183 g), Pd(PPh₃)₄ (0.122 g), a 0.84 mol/L potassium carbonate aqueous solution (1.87 mL), and 1,4-dioxane (7.5 mL) was stirred at 120°C for one hour under microwave irradiation. Water was added to the reaction mixture, and the mixture was extracted with DCM. The extract was dried over anhydrous sodium sulfate and concentrated under reduced pressure. The residue was purified by silica gel column chromatography (eluent: n-hexane/ethyl acetate = 100/0 to 82/18), and the title compound (0.125 g) was obtained.

### Reference Example F-2

### tert-Butyl (4-(2-(azidomethyl)pyridine-3-yl)-2-fluoro-3-phenoxyphenyl)carbamate

TBAF (1 mol/L in THF) (0.705 mL) was added to a mixture of Reference Example F-1 (0.123 g) and THF (3.4 mL) at room temperature. The reaction mixture was stirred at room temperature for 4.5 hours. The reaction mixture was concentrated under reduced pressure. The residue was purified by Method A (eluent: n-hexane/ethyl acetate = 100/0 to 25/75), and tert-butyl (2-fluoro-4-(2-(Hydroxymethyl)pyridine-3-yl)-3-phenoxyphenyl)carbamate was obtained.

Methanesulfonyl chloride (0.037 g) was added to a mixture of the obtained compound, TEA (0.066 g), and DCM (3 mL) at room temperature. The reaction mixture was stirred at the same temperature for two hours. Water was added to the reaction mixture, and the mixture was extracted with DCM. The extract was washed with water, then, dried over anhydrous sodium sulfate, and concentrated under reduced pressure.

Sodium azide (0.021 g) was added to a mixture of the residue and DMF (3 mL) at room temperature. The reaction mixture was stirred at the same temperature for three hours. Water was poured into the reaction mixture, and the mixture was extracted with ethyl acetate. The extract was washed with a saturated saline solution, then, dried over anhydrous sodium sulfate, and concentrated under reduced pressure. The residue was purified by silica gel column chromatography (eluent: n-hexane/ethyl acetate = 95/5 to 75/25), and the title compound (0.085 g) was obtained.

### Reference Example F-3

### tert-Butyl (4-(2-(aminomethyl)pyridine-3-yl)-2-fluoro-3-phenoxyphenyl)carbamate

Triphenylphosphine (0.076 g) was added to a mixture of Reference Example F-2 (0.083 g), water (0.33 mL), and THF (3.3 mL) at room temperature. The reaction mixture was stirred at 50°C for three hours. The reaction mixture was cooled to room temperature and concentrated under reduced pressure. The residue was purified by amino silica gel column chromatography (eluent: ethyl acetate/methanol = 99/1 to 91/9), and the title compound (0.045 g) was obtained.

### Reference Example F-4

### 1-((3-(4-Amino-3-fluoro-2-(2-fluorophenoxy)phenyl)pyridin-2-yl)methyl)urea

A mixture of Example B-2 (0.105 g) and hydrogen chloride (4 mol/L in 1,4-dioxane) (2 mL) was stirred at room temperature for three hours. The reaction mixture was concentrated under reduced pressure. The residue was purified by amino silica gel column chromatography (eluent: ethyl acetate/methanol = 100/0 to 70/30), and the title compound (0.065 g) was obtained.

### Reference Example F-5

### tert-Butyl (4-(2-acetylpyridin-3-yl)-3-phenoxyphenyl)carbamate

Pd(amphos)₂Cl₂ (0.017 g) was added to a mixture of Reference Example B-13 (0.100 g), Reference Example C-9 (0.066 g), a 2 mol/L sodium carbonate aqueous solution (0.243 mL), and toluene (1 mL) at room temperature. The reaction mixture was stirred at 120°C for one hour and then at 150°C for one hour under microwave irradiation. Water was added to the reaction mixture, and the mixture was extracted with DCM. The extract was concentrated under reduced pressure. The residue was purified by Method A (eluent: n-hexane/ethyl acetate = 90/10 to 50/50), and the title compound (0.055 g) was obtained.

### Reference Example G-1

### tert-Butyl (4-(3-((1,3-dioxoisoindolin-2-yl)methyl)pyridazin-4-yl)-2-fluoro-3-(2-fluorobenzyl)phenyl)carbamate

Pd(amphos)₂Cl₂ (0.042 g) was added to a mixture of Reference Example A-5 (0.178 g), Reference Example D-5 (0.089 g), a 2 mol/L potassium carbonate aqueous solution (0.444 mL), ethanol (0.444 mL), and toluene (1 mL) at room temperature. The reaction mixture was stirred at 100°C for one hour under microwave irradiation. Water was added to the reaction mixture, and the mixture was extracted with DCM. The extract was concentrated under reduced pressure. The residue was purified by Method A (eluent: n-hexane/ethyl acetate = 80/20 to 20/80), and the title compound (0.038 g) was obtained.

### Reference Example G-2

### 1-((4-(4-Amino-3-fluoro-2-(2-fluorobenzyl)phenyl)pyridazin-3-yl)methyl)urea

A mixture of Example C-1 (0.015 g) and hydrogen chloride (4 mol/L in 1,4-dioxane) (1 mL) was stirred at room temperature for one hour. The reaction mixture was concentrated under reduced pressure. The residue was purified by amino silica gel column chromatography (eluent: ethyl acetate/methanol = 98/2 to 80/20), and the title compound (0.010 g) was obtained.

### Reference Example G-3

### 1-Methylcyclopropyl (4-(3-((1,3-dioxoisoindolin-2-yl)methyl)pyridazin-4-yl)-2-fluoro-3-(2-fluorobenzyl)phenyl)carbamate

A mixture of Reference Example A-16 (0.109 g), Reference Example D-6 (0.047 g), Pd(PPh₃)₄ (0.034 g), a 2 mol/L potassium carbonate aqueous solution (0.222 mL), ethanol (0.222 mL), and toluene (1 mL) was stirred at 100°C for one hour under microwave irradiation. Water was added to the reaction mixture, and the mixture was extracted with DCM. The extract was concentrated under reduced pressure. The residue was purified by silica gel column chromatography (eluent: n-hexane/ethyl acetate = 80/20 to 20/80), and the title compound (0.014 g) was obtained.

### Reference Example G-4

### tert-Butyl (4-(6-chloro-3-oxo-2,3-dihydropyridazin-4-yl)-2-fluoro-3-(2-fluorobenzyl)phenyl)carbamate

Pd(PPh₃)₄ (0.152 g) was added to a mixture of Reference Example A-5 (0.400 g), 4-bromo-6-chloropyridazine-3(2H)-one (0.151 g), a 2 mol/L potassium carbonate aqueous solution (0.984 mL), and 1,4-dioxane (3 mL) at room temperature. The reaction mixture was stirred at 160°C for 30 minutes under microwave irradiation. Water was added to the reaction mixture, and the mixture was extracted with DCM. The extract was concentrated under reduced pressure. The residue was purified by silica gel column chromatography (eluent: n-hexane/ethyl acetate = 80/20 to 60/40), and the title compound (0.036 g) was obtained.
¹H-NMR (CDCl₃) δ ppm : 1.53 (9H, s), 4.04 (2H, s), 6.76-7.02 (6H, m), 7.10-7.20 (1H, m), 8.05-8.18 (1H, m), 10.29 (1H, br)

### Reference Example G-5

### Tert-Butyl (2-fluoro-3-(2-fluorobenzyl)-4-(3-oxo-2,3-dihydropyridazin-4-yl)phenyl)carbamate

A mixture of Reference Example G-4 (0.036 g), 10% Pd/C (0.020 g), and methanol (1 mL) was stirred at room temperature for one hour under a hydrogen atmosphere. The reaction mixture was filtered through Celite, and the filtrate was concentrated under reduced pressure. The residue was purified by amino silica gel column chromatography (eluent: ethyl acetate/methanol =100/0 to 90/10), and the title compound (0.030 g) was obtained.

### Reference Example G-6

### 4-(4-((tert-Butoxycarbonyl)amino)-3-fluoro-2-(2-fluorobenzyl)phenyl)pyridazin-3-yl trifluoromethanesulfonate

N-phenylbis(trifluoromethanesulfonimide) (0.031 g) was added to a mixture of Reference Example G-5 (0.030 g), cesium carbonate (0.047 g), and DMF (1 mL) under ice cooling. The reaction mixture was stirred for one hour under ice cooling. Water was poured into the reaction mixture, and the mixture was extracted with ethyl acetate. The extract was washed with water and a saturated saline solution, then, dried over anhydrous magnesium sulfate, and concentrated under reduced pressure. The residue was purified by silica gel column chromatography (eluent: n-hexane/ethyl acetate = 80/20 to 30/70), and the title compound (0.036 g) was obtained.

### Reference Example G-7

### Ethyl (E)-3-(4-(4-((tert-butoxycarbonyl)amino)-3-fluoro-2-(2-fluorobenzyl)phenyl)pyridazin-3-yl)acrylate

Pd(PPh₃)₄ (0.015 g) was added to a mixture of Reference Example G-6 (0.036 g), ethyl (E)-3-(4,4,5,5-tetramethyl-1,3,2-dioxaborolan-2-yl)acrylate (0.016 g), a 2 mol/L potassium carbonate aqueous solution (0.099 mL), ethanol (0.1 mL), and DME (1 mL) at room temperature. The reaction mixture was stirred at 110°C for 30 minutes under microwave irradiation. A saturated ammonium chloride aqueous solution was poured into the reaction mixture, and the mixture was extracted with DCM. The extract was concentrated under reduced pressure. The residue was purified by Method A (eluent: n-hexane/ethyl acetate = 80/20 to 10/90), and the title compound (0.011 g) was obtained.

### Reference Example G-8

### 3-(4-(4-Amino-3-fluoro-2-(2-fluorobenzyl)phenyl)pyridazin-3-yl)propanamide

A mixture of Example C-4 (0.017 g) and hydrogen chloride (4 mol/L in 1,4-dioxane) (1 mL) was stirred at room temperature for four hours. The reaction mixture was concentrated under reduced pressure. The residue was purified by amino silica gel column chromatography (eluent: ethyl acetate/methanol = 100/0 to 60/40), and the title compound (0.009 g) was obtained.

### Reference Example G-9

### [1,1'-Bi(cyclopropane)]-1-yl (4-(3-((1,3-dioxoisoindolin-2-yl)methyl)pyridazin-4-yl)-2-fluoro-3-(2-fluorobenzyl)phenyl)carbamate

Pd(amphos)₂Cl₂ (0.015 g) was added to a mixture of Reference Example A-57 (0.050 g), Reference Example D-5 (0.029 g), a 2 mol/L potassium carbonate aqueous solution (0.160 mL), ethanol (0.160 mL), and toluene (2 mL) at room temperature. The reaction mixture was stirred at 100°C for one hour under microwave irradiation. Water was added to the reaction mixture, and the mixture was extracted with ethyl acetate. The extract was washed with a saturated saline solution, then, dried over anhydrous sodium sulfate, and concentrated under reduced pressure. The residue was purified by silica gel column chromatography (eluent: n-hexane/ethyl acetate/methanol = 70/30/0 to 0/100/0 to 0/98/2), and the title compound (0.009 g) was obtained.

### Reference Example H-1

### tert-Butyl (4-(3-((1,3-dioxoisoindolin-2-yl)methyl)pyridazin-4-yl)-2-fluoro-3-(2-fluorophenoxy)phenyl)carbamate

Pd(amphos)₂Cl₂ (0.044 g) was added to a mixture of Reference Example B-9 (0.120 g), Reference Example D-5 (0.086 g), a 2 mol/L potassium carbonate aqueous solution (0.469 mL), ethanol (0.469 mL), and toluene (1.5 mL) at room temperature. The reaction mixture was stirred at 100°C for one hour under microwave irradiation. Water was added to the reaction mixture, and the mixture was extracted with ethyl acetate. The extract was washed with a saturated saline solution, then, dried over anhydrous sodium sulfate, and concentrated under reduced pressure. The residue was purified by Method A (eluent: n-hexane/ethyl acetate/methanol = 69/29/2 to 0/98/2), and the title compound (0.062 g) was obtained.

### Reference Example H-2

### 1-((4-(4-Amino-3-fluoro-2-(2-fluorophenoxy)phenyl)pyridazin-3-yl)methyl)urea

A mixture of Example D-1 (0.020 g) and hydrogen chloride (4 mol/L in 1,4-dioxane) (1 mL) was stirred at room temperature for 30 minutes and then at 50°C for one hour. The reaction mixture was concentrated under reduced pressure. The residue was purified by amino silica gel column chromatography (eluent: ethyl acetate/methanol = 98/2 to 80/20), and the title compound (0.016 g) was obtained.

Reference Example H-3 was synthesized by the same method as in Reference Example G-4 except that Reference Example B-9 was used in place of Reference Example A-5.
¹H-NMR (CDCl₃) δ ppm : 1.53 (9H, s), 6.71-6.89 (2H, m), 6.92-7.04 (2H, m), 7.06-7.38 (1H, m), 7.45 (1H, s), 7.47-7.53 (1H, m), 8.01-8.20 (1H, m), 10.56 (1H, br)

### Reference Example H-4

### tert-Butyl (2-fluoro-3-(2-fluorophenoxy)-4-(3-oxo-2,3-dihydropyridazine-4-yl)phenyl)carbamate

A mixture of Reference Example H-3 (0.064 g), 10% Pd/C (0.064 g), potassium carbonate (0.020 g), and 1,4-dioxane (3 mL) was stirred at room temperature for 16 hours under a hydrogen atmosphere. The reaction mixture was filtered through Celite, and the filtrate was concentrated under reduced pressure. The residue was purified by silica gel column chromatography (eluent: n-hexane/ethyl acetate = 95/5 to 50/50), and the title compound (0.058 g) was obtained.

### Reference Example H-5

### 4-(4-((tert-Butoxycarbonyl)amino)-3-fluoro-2-(2-fluorophenoxy)phenyl)pyridazin-3-yl trifluoromethanesulfonate

N-phenylbis(trifluoromethanesulfonimide) (0.075 g) was added to a mixture of Reference Example H-4 (0.058 g), cesium carbonate (0.136 g), and DMF (2 mL) under ice cooling. The reaction mixture was stirred for one hour under ice cooling. Water was poured into the reaction mixture, and the mixture was extracted with ethyl acetate. The extract was washed with water and a saturated saline solution, then, dried over anhydrous magnesium sulfate, and concentrated under reduced pressure. The residue was purified by silica gel column chromatography (eluent: n-hexane/ethyl acetate = 80/20 to 30/70), and the title compound (0.040 g) was obtained.

### Reference Example H-6

### Ethyl (E)-3-(4-(4-((tert-butoxycarbonyl)amino)-3-fluoro-2-(2-fluorophenoxy)phenyl)pyridazin-3-yl)acrylate

Pd(PPh₃)₄ (0.017 g) was added to a mixture of Reference Example H-5 (0.040 g), ethyl (E)-3-(4,4,5,5-tetramethyl-1,3,2-dioxaborolan-2-yl)acrylate (0.025 g), a 2 mol/L potassium carbonate aqueous solution (0.110 mL), ethanol (0.1 mL), and DME (2 mL) at room temperature. The reaction mixture was stirred at 110°C for 30 minutes under microwave irradiation. A saturated ammonium chloride aqueous solution was poured into the reaction mixture, and the mixture was extracted with DCM. The extract was concentrated under reduced pressure. The residue was purified by Method A (eluent: n-hexane/ethyl acetate = 80/20 to 10/90), and the title compound (0.034 g) was obtained.

### Reference Example H-7

### 3-(4-(4-Amino-3-fluoro-2-(2-fluorophenoxy)phenyl)pyridazin-3-yl)propanamide

A mixture of Example D-3 (0.019 g) and hydrogen chloride (4 mol/L in 1,4-dioxane) (2 mL) was stirred at room temperature for one hour. The reaction mixture was concentrated under reduced pressure. The residue was purified by amino silica gel column chromatography (eluent: ethyl acetate/methanol = 100/0 to 60/40), and the title compound (0.008 g) was obtained.

### Reference Example I-1

### (4-Nitrophenyl)1-methylcyclopropyl carbonate

Under an argon atmosphere, 4-nitrophenyl chloroformate (1.54 g) was added to a mixture of 1-methylcyclopropan-1-ol (0.500 g), pyridine (1.10 g), and DCM (10 mL) under ice cooling. The reaction mixture was stirred at room temperature for three hours. Water was added to the reaction mixture, and the mixture was extracted with DCM. The extract was concentrated under reduced pressure. n-Hexane was added to the residue, and insoluble matter was collected. The filtrate was concentrated under reduced pressure, and the title compound (1.31 g) was obtained.

### Reference Example I-2

### (4-Nitrophenyl)1-(benzyloxy)-2-methylpropan-2-yl carbonate

Pyridine (0.088 g) and 4-nitrophenyl chloroformate (0.124 g) were sequentially added to a mixture of 1-(benzyloxy)-2-methylpropan-2-ol (0.101 g) and DCM (1 mL) under ice cooling. The reaction mixture was stirred at room temperature for 3.5 hours. Water was added to the reaction mixture, and the mixture was extracted with DCM. The extract was washed with a saturated ammonium chloride aqueous solution three times, dried over anhydrous sodium sulfate, and then concentrated under reduced pressure. The residue was purified by silica gel column chromatography (eluent: n-hexane/ethyl acetate = 100/0 to 88/12), and the title compound (0.153 g) was obtained.

### Reference Example I-3

### Quinuclidine-3-yl carbonochloride hydrochloride

Trichloromethyl chloroformate (0.404 g) was slowly added to a mixture of 3-quinuclidinol (0.201 g) and MeCN (16 mL) under ice cooling. The reaction mixture was stirred for one hour under ice cooling and then at room temperature for 16 hours. The reaction mixture was concentrated under reduced pressure, and the title compound (0.357 g) was obtained.

### Reference Example I-4

### (4-Nitrophenyl)1,1-difluoro-2-methylpropan-2-yl carbonate

Pyridine (0.158 g) and 4-nitrophenyl chloroformate (0.222 g) were added to a mixture of 1,1-difluoro-2-methylpropan-2-ol (0.110 g) and DCM (5 mL) at room temperature. The reaction mixture was stirred at the same temperature for four hours. A saturated ammonium chloride aqueous solution and water were added to the reaction mixture, and the mixture was extracted with DCM. The extract was concentrated under reduced pressure, and a small amount of ethyl acetate was added to the residue. n-Hexane was added to the mixture, and insoluble matter was collected. The filtrate was concentrated under reduced pressure, thereby obtaining the title compound (0.099 g).

Reference Example I-5 was synthesized by the same method as in Reference Example I-2 except that bicyclo[2.2.2]octan-3-ol was used in place of 1-(benzyloxy)-2-methylpropan-2-ol.

Reference Example I-6 was synthesized by the same method as in Reference Example I-4 except that [1,1'-bi(cyclopropane)]-1-ol was used in place of 1,1-difluoro-2-methylpropan-2-ol.

### Reference Example J-1

### Ethyl (E)-3-(3-bromopyrazin-2-yl)acrylate

Under an argon atmosphere, sodium hydride (about 60%) (0.021 g) was added to a mixture of triethyl phosphonoacetate (0.121 g) and THF (2.5 mL) under ice cooling. The reaction mixture was stirred for 30 minutes under ice cooling. A solution of 3-bromopyrazine-2-carbaldehyde (0.084 g) in THF (2 mL) was added to the reaction mixture under ice cooling. The reaction mixture was stirred at room temperature for five hours. Water was added to the reaction mixture, and the mixture was extracted with ethyl acetate. The extract was washed with a saturated sodium bicarbonate solution and a saturated saline solution, then, dried over anhydrous magnesium sulfate, and concentrated under reduced pressure. The residue was purified by silica gel column chromatography (eluent: n-hexane/ethyl acetate = 98/2 to 75/25), and the title compound (0.076 g) was obtained.

### Reference Example J-2

### (E)-3-(3-Bromopyrazin-2-yl)acrylamide

A 4 mol/L lithium hydroxide aqueous solution (0.219 mL) was added to a mixture of Reference Example J-1 (0.075 g), THF (1 mL), and methanol (1 mL) at room temperature. The reaction mixture was stirred at the same temperature for one hour. Water and 1 mol/L hydrochloric acid (1 mL) were added to the reaction mixture, and the mixture was extracted with DCM. The extract was concentrated under reduced pressure.

DMT-MM (0.121 g) and 15 mol/L ammonia water (0.029 mL) were added to a mixture of the residue and MeCN (1 mL) at room temperature. The reaction mixture was stirred at the same temperature for one hour. A saturated ammonium chloride aqueous solution and DCM were added to the reaction mixture. The mixture was stirred at the same temperature for 10 minutes, and insoluble matter was then collected. The obtained solid was dried under reduced pressure, thereby obtaining the title compound (0.026 g).

The structural formulae of Reference Examples are shown in tables below.

**[Table 1]**

| Ref. No. | Str. | Ref. No. | Str. |
|---|---|---|---|
| A-1 | 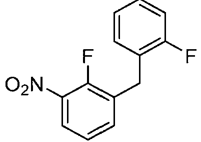 | A-2 | 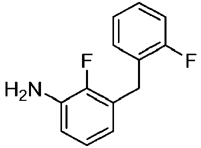 |
| A-3 | 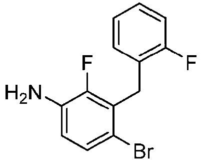 | A-4 | 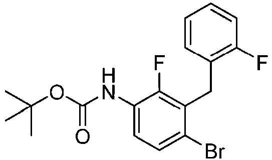 |
| A-5 | 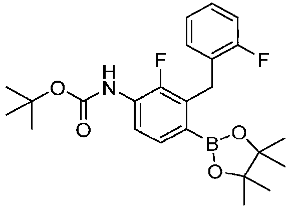 | A-6 | 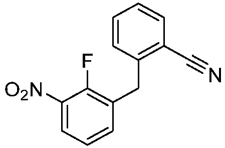 |
| A-7 | 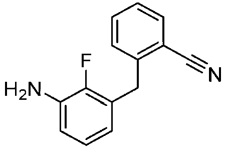 | A-8 | 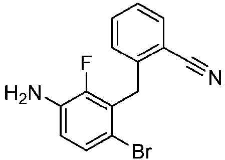 |
| A-9 | 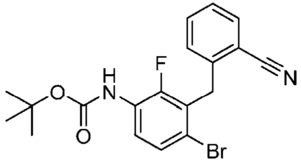 | A-10 | 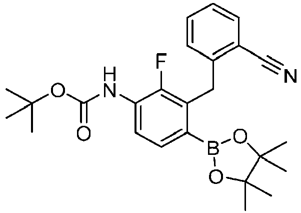 |

**[Table 2]**

| Ref. No. | Str. | Ref. No. | Str. |
|---|---|---|---|
| A-11 | 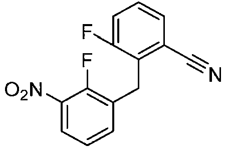 | A-12 | 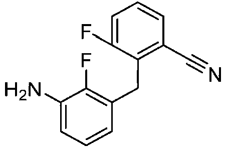 |
| A-13 | 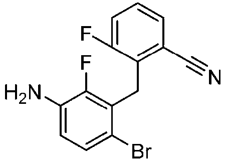 | A-14 | 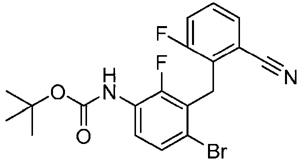 |
| A-15 | 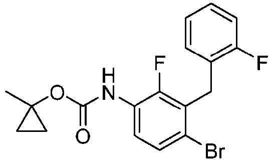 | A-16 | 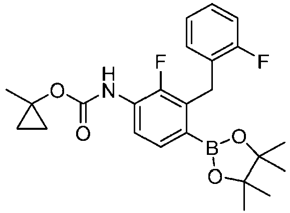 |
| A-17 | 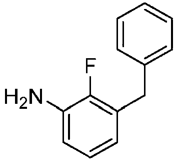 | A-18 | 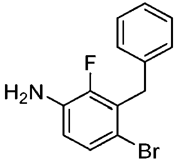 |
| A-19 | 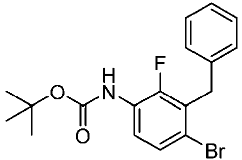 | A-20 | 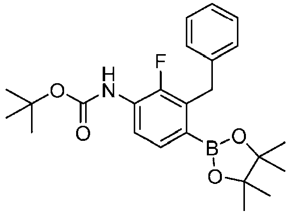 |

**[Table 3]**

| Ref. No. | Str. | Ref. No. | Str. |
|---|---|---|---|
| A-21 | 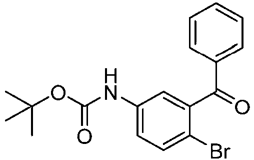 | A-22 | 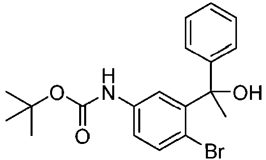 |
| A-23 | 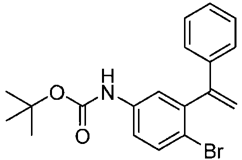 | A-24 | 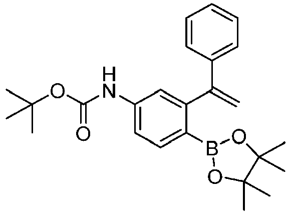 |
| A-25 | 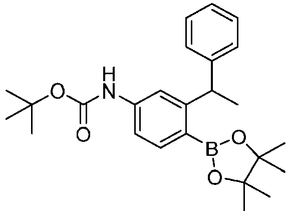 | A-26 | 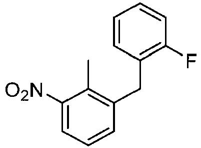 |
| A-27 | 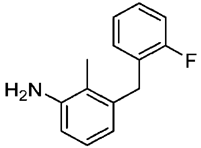 | A-28 | 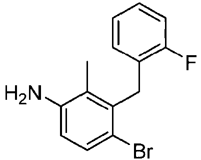 |
| A-29 | 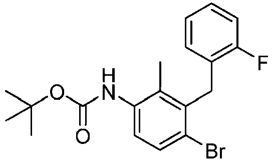 | A-30 | 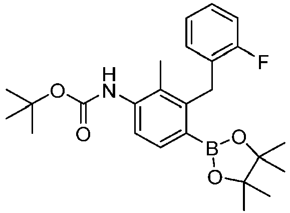 |

**[Table 4]**

| Ref. No. | Str. | Ref. No. | Str. |
|---|---|---|---|
| A-31 | 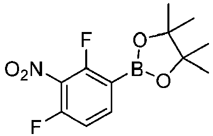 | A-32 | 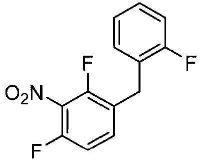 |
| A-33 | 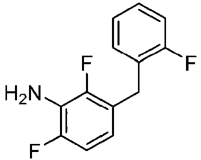 | A-34 | 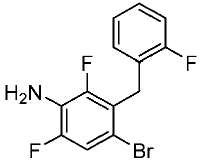 |
| A-35 | 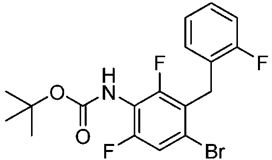 | A-36 | 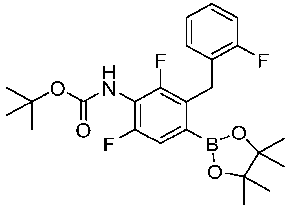 |
| A-37 | 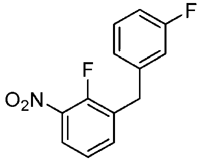 | A-38 | 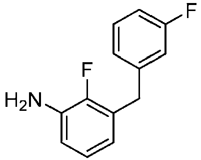 |
| A-39 | 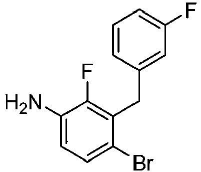 | A-40 | 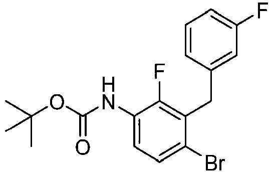 |

**[Table 5]**

| Ref. No. | Str. | Ref. No. | Str. |
|---|---|---|---|
| A-41 | 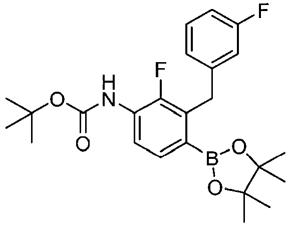 | A-42 | 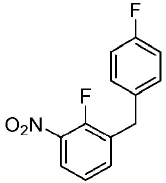 |
| A-43 | 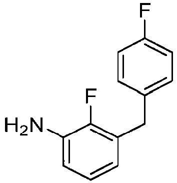 | A-44 | 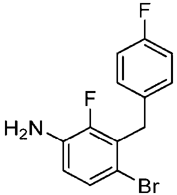 |
| A-45 | 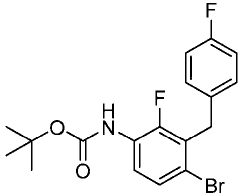 | A-46 | 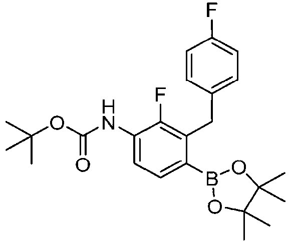 |
| A-47 | 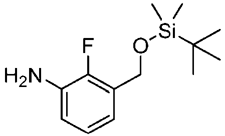 | A-48 | 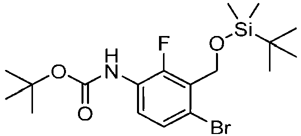 |
| A-49 | 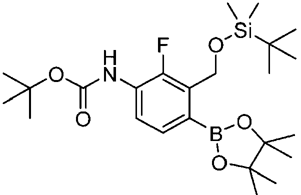 | A-50 | 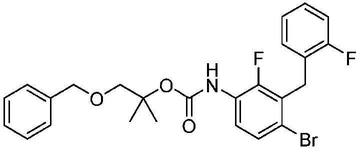 |

**[Table 6]**

| Ref. No. | Str. | Ref. No. | Str. |
|---|---|---|---|
| A-51 | 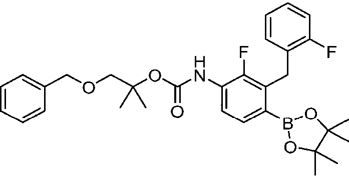 | A-52 | 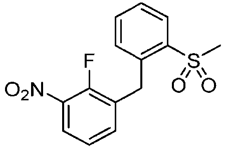 |
| A-53 | 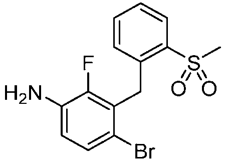 | A-54 | 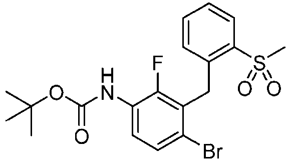 |
| A-55 | 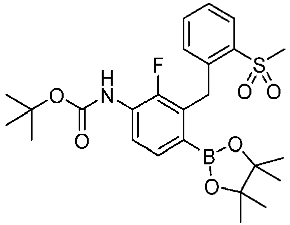 | A-56 | 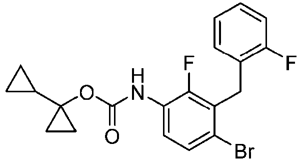 |
| A-57 | 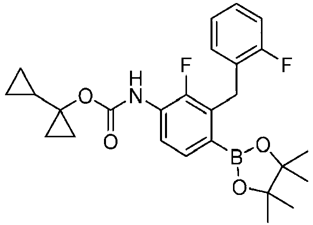 | A-58 | 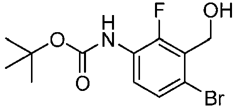 |
| A-59 | 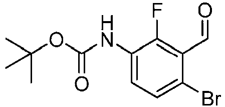 | A-60 | 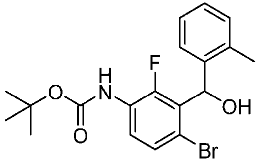 |

**[Table 7]**

| Ref. No. | Str. | Ref. No. | Str. |
|---|---|---|---|
| A-61 | 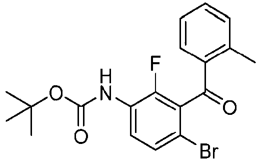 | A-62 | 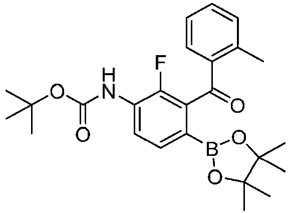 |
| A-63 | 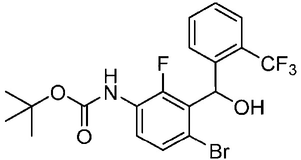 | A-64 | 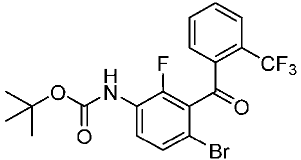 |
| A-65 | 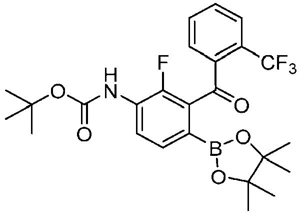 | A-66 | 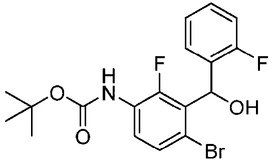 |
| A-67 | 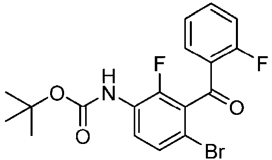 | A-68 | 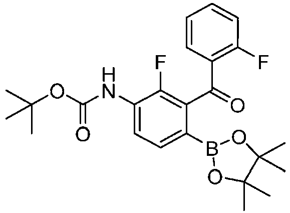 |
| A-69 | 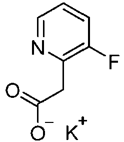 | A-70 | 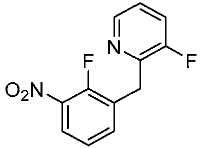 |

**[Table 8]**

| Ref. No. | Str. | Ref. No. | Str. |
|---|---|---|---|
| A-71 | 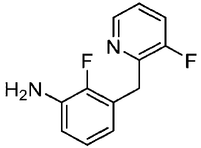 | A-72 | 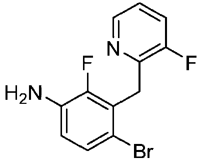 |
| A-73 | 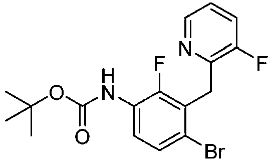 | A-74 | 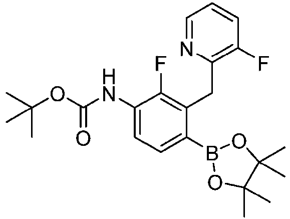 |
| A-75 | 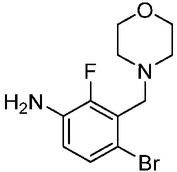 | A-76 | 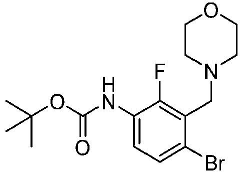 |
| A-77 | 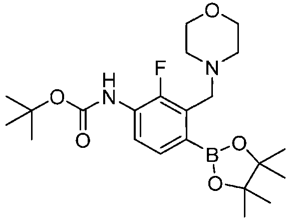 | A-78 | 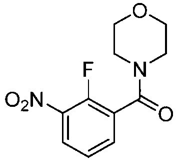 |
| A-79 | 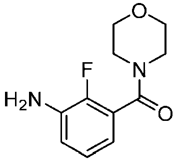 | A-80 | 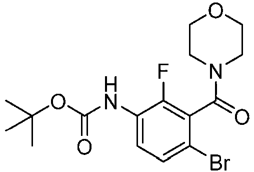 |

**[Table 9]**

| Ref. No. | Str. | Ref. No. | Str. |
|---|---|---|---|
| A-81 | 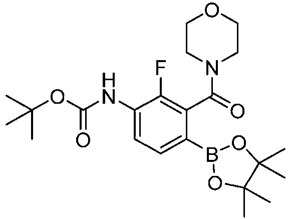 | A-82 | 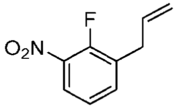 |
| A-83 | 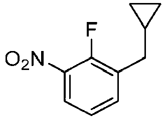 | A-84 | 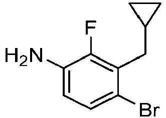 |
| A-85 | 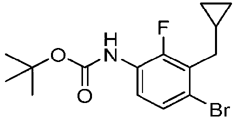 | A-86 | 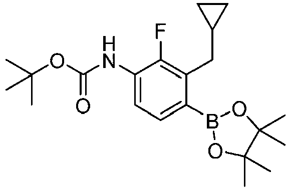 |
| B-1 | 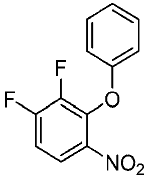 | B-2 | 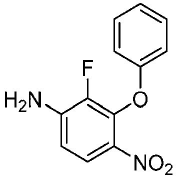 |
| B-3 | 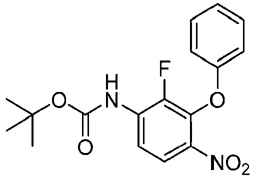 | B-4 | 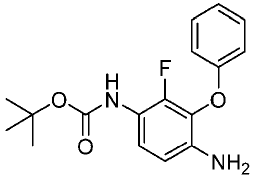 |
| B-5 | 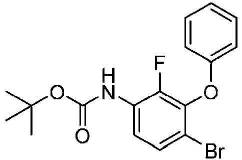 | B-6 | 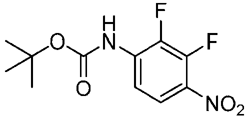 |

**[Table 10]**

| Ref. No. | Str. | Ref. No. | Str. |
|---|---|---|---|
| B-7 | 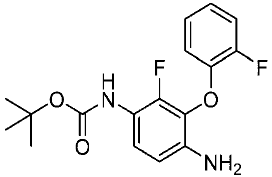 | B-8 | 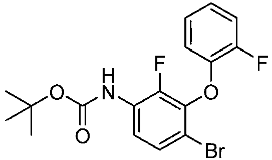 |
| B-9 | 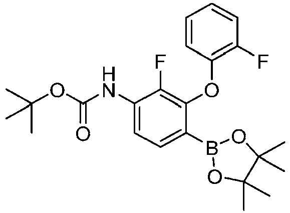 | B-10 | 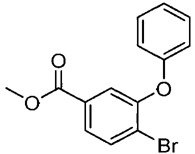 |
| B-11 | 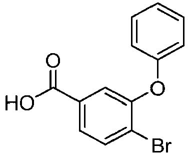 | B-12 | 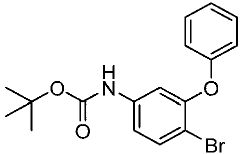 |
| B-13 | 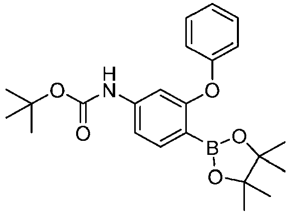 | C-1 | 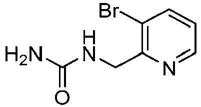 |
| C-2 | 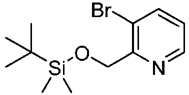 | C-3 | 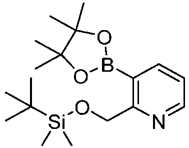 |
| C-4 | 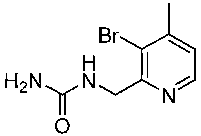 | C-5 | 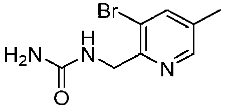 |

**[Table 11]**

| Ref. No. | Str. | Ref. No. | Str. |
|---|---|---|---|
| C-6 | 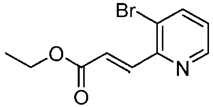 | C-7 | 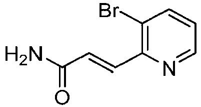 |
| C-8 | 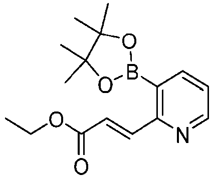 | C-9 | 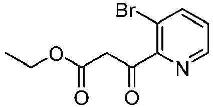 |
| D-1 | 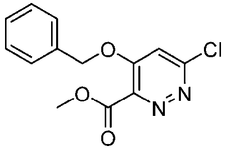 | D-2 | 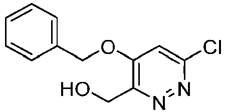 |
| D-3 | 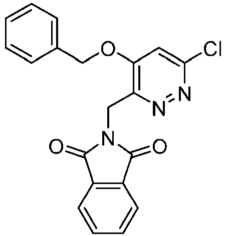 | D-4 | 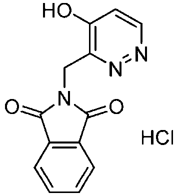 |
| D-5 | 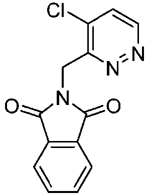 | D-6 | 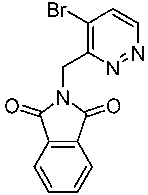 |
| E-1 | 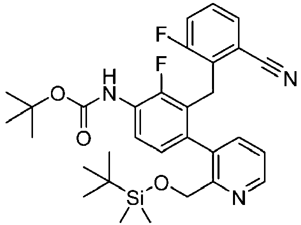 | E-2 | 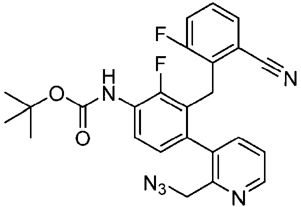 |

**[Table 12]**

| Ref. No. | Str. | Ref. No. | Str. |
|---|---|---|---|
| E-3 | 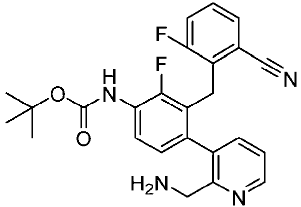 | E-4 | 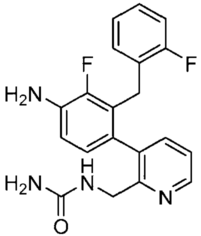 |
| E-5 | 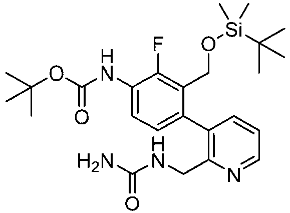 | E-6 | 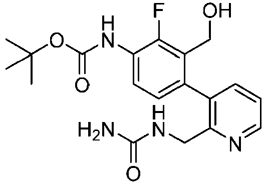 |
| E-7 | 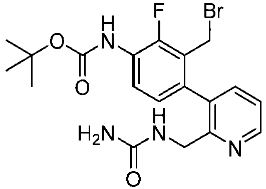 | E-8 | 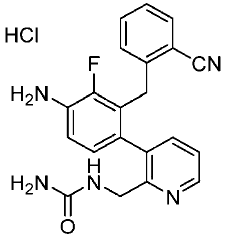 |
| E-9 | 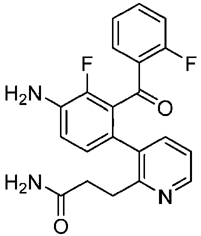 | E-10 | 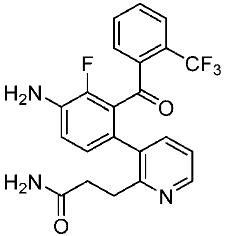 |
| E-11 | 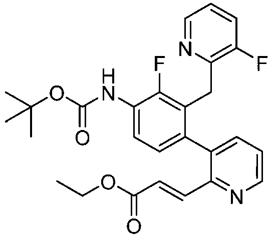 | E-12 | 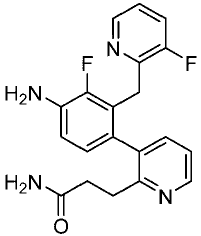 |

**[Table 13]**

| Ref. No. | Str. | Ref. No. | Str. |
|---|---|---|---|
| F-1 | 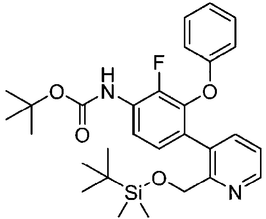 | F-2 | 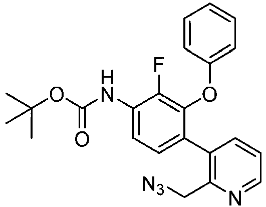 |
| F-3 | 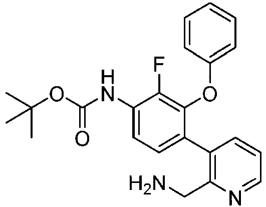 | F-4 | 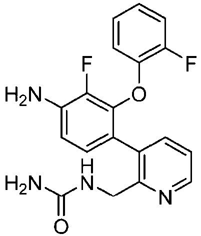 |
| F-5 | 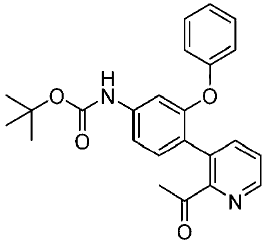 | G-1 | 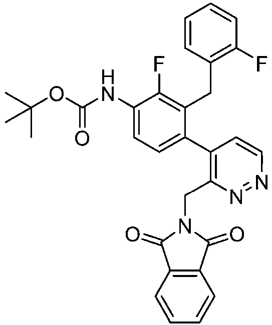 |
| G-2 | 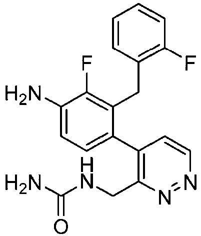 | G-3 | 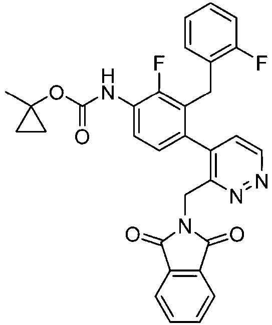 |

**[Table 14]**

| Ref. No. | Str. | Ref. No. | Str. |
|---|---|---|---|
| G-4 | 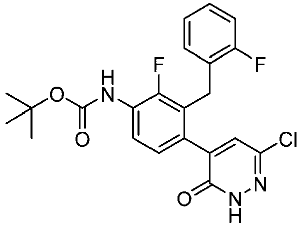 | G-5 | 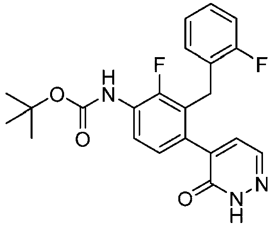 |
| G-6 | 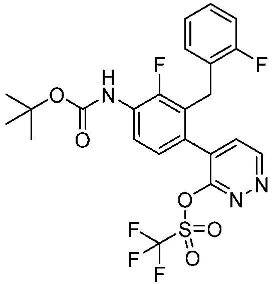 | G-7 | 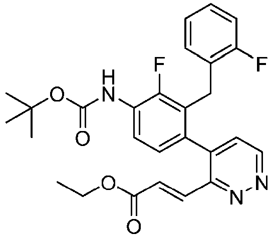 |
| G-8 | 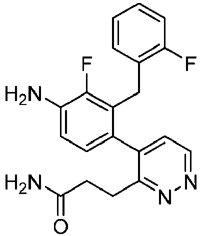 | G-9 | 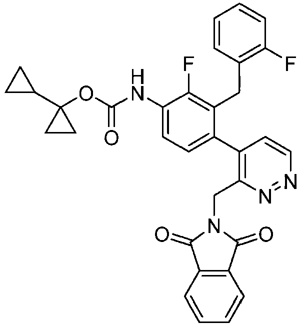 |
| H-1 | 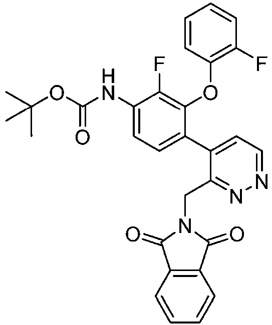 | H-2 | 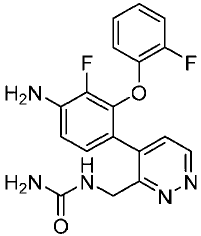 |

**[Table 15]**

| Ref. No. | Str. | Ref. No. | Str. |
|---|---|---|---|
| H-3 | 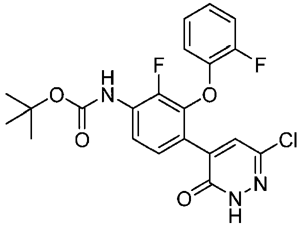 | H-4 | 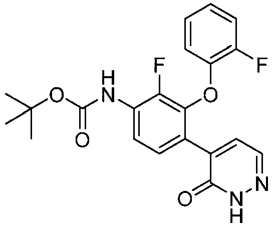 |
| H-5 | 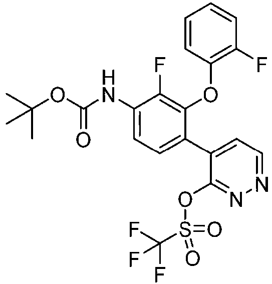 | H-6 | 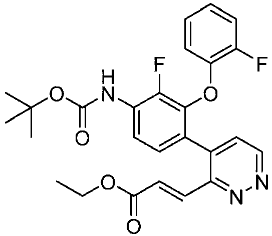 |
| H-7 | 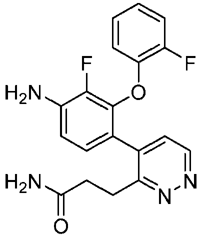 | I-1 | 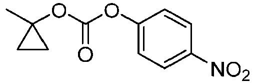 |
| I-2 | 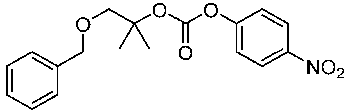 | I-3 | 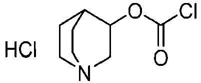 |
| I-4 | 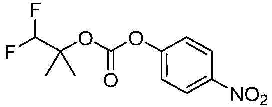 | I-5 | 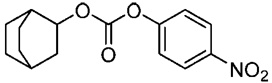 |

**[Table 16]**

| Ref. No. | Str. | Ref. No. | Str. |
|---|---|---|---|
| I-6 | 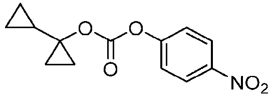 | J-1 | 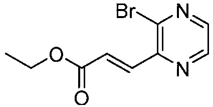 |
| J-2 | 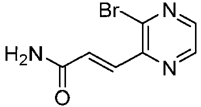 | | |

### Example A-1

### tert-Butyl (2-fluoro-3-(2-fluorobenzyl)-4-(2-(ureidomethyl)pyridin-3-yl)phenyl)carbamate

Pd(PPh₃)₄ (0.023 g) was added to a mixture of Reference Example A-5 (0.060 g), Reference Example C-1 (0.023 g), a 2 mol/L potassium carbonate aqueous solution (0.150 mL), ethanol (0.150 mL), and toluene (2 mL) at room temperature. The reaction mixture was stirred at 100°C for one hour under microwave irradiation. Water was added to the reaction mixture, and the mixture was extracted with DCM. The extract was concentrated under reduced pressure. The residue was purified by silica gel column chromatography (eluent: ethyl acetate/methanol = 98/2 to 85/15), and the title compound (0.032 g) was obtained.

### Example A-2

### tert-Butyl (3-(2-cyanobenzyl)-2-fluoro-4-(2-(ureidomethyl)pyridin-3-yl)phenyl)carbamate

Pd(PPh₃)₄ (0.058 g) was added to a mixture of Reference Example A-10 (0.160 g), Reference Example C-1 (0.058 g), a 2 mol/L potassium carbonate aqueous solution (0.377 mL), ethanol (0.377 mL), and toluene (2 mL) at room temperature. The reaction mixture was stirred at 100°C for one hour under microwave irradiation. Water was added to the reaction mixture, and the mixture was extracted with DCM. The extract was concentrated under reduced pressure. The residue was sequentially purified by silica gel column chromatography (eluent: ethyl acetate/methanol = 98/2 to 85/15) and amino silica gel column chromatography (eluent: ethyl acetate/methanol = 100/0 to 85/15), and the title compound (0.070 g) was obtained.

### Example A-3

### tert-Butyl (3-(2-cyano-6-fluorobenzyl)-2-fluoro-4-(2-(ureidomethyl)pyridin-3-yl)phenyl)carbamate

Trimethylsilyl isocyanate (0.017 g) was added to a mixture of Reference Example E-3 (0.026 g) and THF (0.75 mL) at room temperature. The reaction mixture was stirred at the same temperature for 30 minutes. The reaction mixture was purified by amino silica gel column chromatography (eluent: n-hexane/ethyl acetate/methanol = 50/50/0 to 0/100/0 to 0/95/5), and the title compound (0.019 g) was obtained.

### Example A-4

### 1-Methylcyclopropyl (2-fluoro-3-(2-fluorobenzyl)-4-(2-(ureidomethyl)pyridin-3-yl)phenyl)carbamate

HOBt hydrate (0.042 g) and DIPEA (0.049 g) were added to a mixture of Reference Example E-4 (0.040 g), Reference Example I-1 (0.471 g), and MeCN (2 mL) at room temperature. The reaction mixture was stirred at 80°C for 70 hours. The reaction mixture was cooled to room temperature, a saturated sodium bicarbonate aqueous solution was added thereto, and the mixture was extracted with DCM. The extract was concentrated under reduced pressure. The residue was purified by Method A (eluent: ethyl acetate/methanol = 100/0 to 90/10), and the title compound (0.008 g) was obtained.

### Example A-5

### trans-3-Fluorocyclobutyl (2-fluoro-3-(2-fluorobenzyl)-4-(2-(ureidomethyl)pyridin-3-yl)phenyl)carbamate

A mixture of Reference Example E-4 (0.020 g), 4-nitrophenyl chloroformate (0.016 g), and THF (1 mL) was stirred under ice cooling for 15 minutes and then at room temperature for 15 minutes. trans-3-Fluorocyclobutan-1-ol (0.024 g) and DMAP (0.010 g) were added to the reaction mixture at room temperature. The reaction mixture was stirred at the same temperature for four hours. A saturated sodium bicarbonate aqueous solution was poured into the reaction mixture, and the mixture was extracted with DCM. The extract was concentrated under reduced pressure. The residue was purified by Method A (eluent: ethyl acetate/methanol = 100/0 to 90/10), and the title compound (0.013 g) was obtained.

Example A-6 to Example A-8 were synthesized by the same method as in Example A-5 except that corresponding raw materials were used in place of trans-3-fluorocyclobutan-1-ol.

### Example A-9

### tert-Butyl (3-benzyl-2-fluoro-4-(4-methyl-2-(ureidomethyl)pyridin-3-yl)phenyl)carbamate

Pd(PPh₃)₄ (0.019 g) was added to a mixture of Reference Example A-20 (0.035 g), Reference Example C-4 (0.020 g), a 2 mol/L potassium carbonate aqueous solution (0.123 mL), ethanol (0.123 mL), and DME (0.5 mL) at room temperature. The reaction mixture was stirred at 120°C for 30 minutes under microwave irradiation. Water was added to the reaction mixture, and the mixture was extracted with DCM. The extract was concentrated under reduced pressure. The residue was purified by silica gel column chromatography (eluent: ethyl acetate/methanol = 100/0 to 85/15), and the title compound (0.009 g) was obtained.

### Example A-10

### tert-Butyl (2-fluoro-3-(2-fluorobenzyl)-4-(5-methyl-2-(ureidomethyl)pyridin-3-yl)phenyl)carbamate

A mixture of Reference Example A-5 (0.030 g), Reference Example C-5 (0.016 g), Pd(PPh₃)₄ (0.016 g), potassium carbonate (0.028 g), water (0.2 mL), ethanol (0.2 mL), and toluene (2 mL) was refluxed for 30 minutes under an argon atmosphere. The reaction mixture was concentrated under reduced pressure. The residue was purified by silica gel column chromatography (eluent: n-hexane/ethyl acetate/methanol = 30/70/0 to 0/100/0 to 0/70/30), and the title compound (0.009 g) was obtained.

### Example A-11

### tert-Butyl (E)-(4-(2-(3-amino-3-oxopropan-1-en-1-yl)pyridin-3-yl)-3-(1-phenylethyl)phenyl)carbamate

Pd(PPh₃)₄ (0.011 g) was added to a mixture of Reference Example A-25 (0.021 g), Reference Example C-7 (0.012 g), potassium carbonate (0.021 g), water (0.14 mL), ethanol (0.14 mL), and toluene (0.56 mL) at room temperature under an argon atmosphere. The reaction mixture was stirred at 100°C for one hour under microwave irradiation. A saturated ammonium chloride aqueous solution was poured into the reaction mixture, and the mixture was extracted with DCM. The extract was concentrated under reduced pressure. The residue was sequentially purified by silica gel column chromatography (eluent: n-hexane/ethyl acetate/methanol = 70/30/0 to 0/100/0 to 0/95/5) and diamine silica gel column chromatography (eluent: n-hexane/ethyl acetate/methanol = 70/30/0 to 0/100/0 to 0/90/10), and the title compound (0.006 g) was obtained.

### Example A-12

### tert-Butyl (4-(2-(3-amino-3-oxopropyl)pyridine-3-yl)-3-(1-phenylethyl)phenyl)carbamate

10% Pd/C (0.012 g) was added to a mixture of Example A-11 (0.011 g), ethanol (1 mL), and THF (1 mL) at room temperature under an argon atmosphere. The reaction mixture was stirred at the same temperature for one hour under a hydrogen atmosphere. The reaction mixture was filtered through Celite, and the filtrate was concentrated under reduced pressure. The residue was purified by amino silica gel column chromatography (eluent: n-hexane/ethyl acetate/methanol = 50/50/0 to 0/100/0 to 0/95/5), and the title compound (0.010 g) was obtained.

### Example A-13

### tert-Butyl (3-(2-fluorobenzyl)-2-methyl-4-(2-(ureidomethyl)pyridin-3-yl)phenyl)carbamate

Pd(PPh₃)₄ (0.023 g) was added to a mixture of Reference Example A-30 (0.050 g), Reference Example C-1 (0.023 g), a 2 mol/L potassium carbonate aqueous solution (0.148 mL), ethanol (0.170 mL), and toluene (1 mL) at room temperature. The reaction mixture was stirred at 100°C for one hour under microwave irradiation. Water was added to the reaction mixture, and the mixture was extracted with DCM. The extract was concentrated under reduced pressure. The residue was purified by Method A (eluent: n-hexane/ethyl acetate/methanol = 49/49/2 to 0/98/2 to 0/86/14), and the title compound (0.034 g) was obtained.

### Example A-14

### tert-Butyl (2,6-difluoro-3-(2-fluorobenzyl)-4-(2-(ureidomethyl)pyridin-3-yl)phenyl)carbamate

Pd(PPh₃)₄ (0.026 g) was added to a mixture of Reference Example A-36 (0.060 g), Reference Example C-1 (0.029 g), a 2 mol/L potassium carbonate aqueous solution (0.169 mL), ethanol (0.169 mL), and toluene (0.5 mL) at room temperature. The reaction mixture was stirred at 100°C for one hour under microwave irradiation. Water was added to the reaction mixture, and the mixture was extracted with DCM. The extract was concentrated under reduced pressure. The residue was purified by Method A (eluent: n-hexane/ethyl acetate/methanol = 49/49/2 to 0/98/2 to 0/86/14), and the title compound (0.043 g) was obtained.

Example A-15 was synthesized by the same method as in Example A-1 except that Reference Example A-41 was used in place of Reference Example A-5.

Example A-16 was synthesized by the same method as in Example A-1 except that Reference Example A-46 was used in place of Reference Example A-5.

### Example A-17

### tert-Butyl (2-fluoro-3-(2-methoxybenzyl)-4-(2-(ureidomethyl)pyridin-3-yl)phenyl)carbamate

Pd(PPh₃)₄ (0.006 g) was added to a mixture of Reference Example E-7 (0.050 g), 2-methoxyphenylboronic acid (0.017 g), sodium carbonate (0.023 g), water (0.25 mL), and DME (0.66 mL) at room temperature. The reaction mixture was stirred at 100°C for one hour under microwave irradiation. A saturated ammonium chloride aqueous solution was added to the reaction mixture, and the mixture was extracted with ethyl acetate. The extract was washed with a saturated saline solution, then, dried over anhydrous sodium sulfate, and concentrated under reduced pressure. The residue was purified by silica gel column chromatography (eluent: ethyl acetate/methanol = 100/0 to 94/6), and the title compound (0.006 g) was obtained.

### Example A-18

### tert-Butyl (3-(2-(benzyloxy)benzyl)-2-fluoro-4-(2-(ureidomethyl)pyridin-3-yl)phenyl)carbamate

Pd(PPh₃)₄ (0.003 g) was added to a mixture of Reference Example E-7 (0.030 g), (2-benzyloxyphenyl)boronic acid (0.015 g), sodium carbonate (0.014 g), water (0.125 mL), and DME (0.33 mL) at room temperature. The reaction mixture was stirred at 100°C for one hour under microwave irradiation. A saturated ammonium chloride aqueous solution was added to the reaction mixture, and the mixture was extracted with ethyl acetate. The extract was washed with a saturated saline solution, then, dried over anhydrous sodium sulfate, and concentrated under reduced pressure. The residue was purified by Method A (eluent: ethyl acetate/methanol = 100/0 to 93/7), and the title compound (0.019 g) was obtained.

### Example A-19

### tert-Butyl (2-fluoro-3-(2-hydroxybenzyl)-4-(2-(ureidomethyl)pyridin-3-yl)phenyl)carbamate

10% Pd/C (0.005 g) was added to a mixture of Example A-18 (0.017 g) and methanol (1 mL) at room temperature. The reaction mixture was stirred at the same temperature for 30 minutes and then at 60°C for four hours under a hydrogen atmosphere. The reaction mixture was cooled to room temperature and then filtered through Celite, and the filtrate was concentrated under reduced pressure. The residue was purified by silica gel column chromatography (eluent: ethyl acetate/methanol = 100/0 to 90/10), and the title compound (0.011 g) was obtained.

### Example A-20

### 1-(Benzyloxy)-2-methylpropan-2-yl (2-fluoro-3-(2-fluorobenzyl)-4-(2-(ureidomethyl)pyridin-3-yl)phenyl)carbamate

A mixture of Reference Example A-51 (0.076 g), Reference Example C-1 (0.023 g), potassium carbonate (0.041 g), Pd(PPh₃)₄ (0.023 g), water (0.21 mL), ethanol (0.21 mL), and DME (0.84 mL) was stirred at 100°C for one hour under microwave irradiation. Water was added to the reaction mixture, and the mixture was extracted with DCM. The extract was dried over anhydrous sodium sulfate and concentrated under reduced pressure. The residue was purified by silica gel column chromatography (eluent: ethyl acetate/methanol = 100/0 to 92/8), and the title compound (0.020 g) was obtained.

### Example A-21

### 1-Hydroxy-2-methylpropan-2-yl (2-fluoro-3-(2-fluorobenzyl)-4-(2-(ureidomethyl)pyridin-3-yl)phenyl)carbamate

10% Pd/C (0.007 g) was added to a mixture of Example A-20 (0.017 g), ethanol (1 mL), and THF (1 mL) at room temperature under an argon atmosphere. The reaction mixture was stirred at the same temperature for 2.5 hours and then at 70°C for nine hours under a hydrogen atmosphere. The reaction mixture was cooled to room temperature and stirred for 10 hours. The reaction mixture was stirred at 70°C for 12 hours. The reaction mixture was cooled to room temperature and then filtered through Celite, and the filtrate was concentrated under reduced pressure. The residue was purified by Method A (eluent: ethyl acetate/methanol = 100/0 to 89/11), and the title compound (0.011 g) was obtained.

Example A-22 to Example A-24 were synthesized by the same method as in Example A-5 except that corresponding raw materials were used in place of trans-3-fluorocyclobutan-1-ol.

### Example A-25

### (S)-1-Methoxypropan-2-yl (2-fluoro-3-(2-fluorobenzyl)-4-(2-(ureidomethyl)pyridin-3-yl)phenyl)carbamate

A mixture of Reference Example E-4 (0.020 g), 4-nitrophenyl chloroformate (0.016 g), and THF (1 mL) was stirred under ice cooling for 15 minutes and then at room temperature for 15 minutes. (S)-1-Methoxypropan-2-ol (0.024 g) and DMAP (0.010 g) were added to the reaction mixture at room temperature. The reaction mixture was stirred at 50°C for three hours. The reaction mixture was cooled to room temperature and poured into a saturated sodium bicarbonate aqueous solution, and the mixture was extracted with DCM. The extract was concentrated under reduced pressure. The residue was purified by Method A (eluent: ethyl acetate/methanol = 100/0 to 85/15), and the title compound (0.015 g) was obtained.

### Example A-26

### Cyclopropyl (2-fluoro-3-(2-fluorobenzyl)-4-(2-(ureidomethyl)pyridin-3-yl)phenyl)carbamate

A mixture of Reference Example E-4 (0.018 g), 4-nitrophenyl chloroformate (0.015 g), and THF (1 mL) was stirred under ice cooling for 15 minutes and then at room temperature for 15 minutes. Cyclopropanol (0.0029 g) and DMAP (0.009 g) were added to the reaction mixture at room temperature. The reaction mixture was stirred at the same temperature for four hours. A saturated sodium bicarbonate aqueous solution was poured into the reaction mixture, and the mixture was extracted with DCM. The extract was concentrated under reduced pressure. The residue was sequentially purified by amino silica gel column chromatography (eluent: ethyl acetate/methanol = 100/0 to 95/5) and silica gel column chromatography (eluent: ethyl acetate/methanol = 100/0 to 95/5), and the title compound (0.003 g) was obtained.

### Example A-27

### 3,3-Difluorocyclopentyl (2-fluoro-3-(2-fluorobenzyl)-4-(2-(ureidomethyl)pyridin-3-yl)phenyl)carbamate

4-Nitrophenyl chloroformate (0.014 g) was added to a mixture of Reference Example E-4 (0.020 g) and THF (0.5 mL) under ice cooling. The reaction mixture was stirred under ice cooling for 30 minutes. 3,3-Difluorocyclopentanol (0.033 g) and DMAP (0.009 g) were added to the reaction mixture under ice cooling. The reaction mixture was stirred at room temperature for three hours. The reaction mixture was partitioned between a saturated sodium bicarbonate aqueous solution and DCM. The organic layer was separated and concentrated under reduced pressure. The residue was purified by Method A (eluent: n-hexane/ethyl acetate/methanol = 50/50/0 to 0/100/0 to 0/85/15), and the title compound (0.012 g) was obtained.

Example A-28 was synthesized by the same method as in Example A-5 except that tetrahydrofuran-3-ol was used in place of trans-3-fluorocyclobutan-1-ol.

### Example A-29

### (2-Fluoro-3-(2-fluorobenzyl)-4-(2-(ureidomethyl)pyridine-3-yl)phenyl)carbamate quinuclidine-3-yl

NaHMDS (1 mol/L in THF) (0.081 mL) was slowly added to a mixture of Reference Example E-4 (0.031 g), Reference Example I-3 (0.028 g), and THF (1 mL) under ice cooling. The reaction mixture was stirred under ice cooling for one hour. Reference Example I-3 (0.018 g) was added to the reaction mixture under ice cooling. The reaction mixture was stirred under ice cooling for one hour and then at room temperature for two hours. A saturated sodium bicarbonate aqueous solution was added to the reaction mixture, and the mixture was extracted with ethyl acetate. The extract was dried over anhydrous sodium sulfate and concentrated under reduced pressure. The residue was sequentially purified by amino silica gel column chromatography (eluent: ethyl acetate/methanol = 100/0 to 89/11) and amino silica gel column chromatography (eluent: ethyl acetate/methanol = 100/0 to 89/11), and the title compound (0.003 g) was obtained.

### Example A-30

### 3-(Dimethylamino)propyl (2-fluoro-3-(2-fluorobenzyl)-4-(2-(ureidomethyl)pyridin-3-yl)phenyl)carbamate

4-Nitrophenyl chloroformate (0.025 g) was added to a mixture of Reference Example E-4 (0.030 g) and THF (1 mL) under ice cooling. The reaction mixture was stirred under ice cooling for 15 minutes. 3-Dimethylamino-1-propanol (0.042 g) and DMAP (0.010 g) were added to the reaction mixture under ice cooling. The reaction mixture was stirred at room temperature overnight. Water was added to the reaction mixture, and the mixture was extracted with ethyl acetate. The extract was concentrated under reduced pressure. The residue was purified by amino silica gel column chromatography (eluent: ethyl acetate/methanol = 100/0 to 48/52), and the title compound (0.040 g) was obtained.

### Example A-31

### Cyclopropylmethyl (2-fluoro-3-(2-fluorobenzyl)-4-(2-(ureidomethyl)pyridin-3-yl)phenyl)carbamate

4-Nitrophenyl chloroformate (0.025 g) was added to a mixture of Reference Example E-4 (0.030 g) and THF (1 mL) under ice cooling. The reaction mixture was stirred under ice cooling for 25 minutes and then at room temperature for 10 minutes. Cyclopropylmethanol (0.029 g) and DMAP (0.015 g) were added to the reaction mixture at room temperature. The reaction mixture was stirred at the same temperature for three hours. The reaction mixture was poured into a saturated sodium bicarbonate aqueous solution, and the mixture was extracted with DCM. The extract was washed with a saturated sodium bicarbonate aqueous solution and then concentrated under reduced pressure. The residue was purified by amino silica gel column chromatography (eluent: n-hexane/ethyl acetate/methanol = 30/70/0 to 0/100/0 to 0/95/5), and the title compound (0.031 g) was obtained.

Example A-32 to Example A-35 were synthesized by the same method as in Example A-5 except that corresponding raw materials were used in place of trans-3-fluorocyclobutan-1-ol.

Example A-36 was synthesized by the same method as in Example A-31 except that (2-fluorophenyl)methanol was used in place of cyclopropylmethanol.

### Example A-37

### 1,1-Difluoro-2-methylpropan-2-yl (3-(2-cyanobenzyl)-2-fluoro-4-(2-(ureidomethyl)pyridin-3-yl)phenyl)carbamate

HOBt hydrate (0.023 g) and TEA (0.022 g) were added to a mixture of Reference Example E-8 (0.025 g), Reference Example I-4 (0.033 g), and MeCN (2 mL) at room temperature. The reaction mixture was stirred at 80°C for 31 hours. The reaction mixture was cooled to room temperature and poured into a saturated sodium bicarbonate aqueous solution, and the mixture was extracted with DCM. The extract was concentrated under reduced pressure. The residue was sequentially purified by Method A (eluent: n-hexane/ethyl acetate/methanol = 10/90/0 to 0/100/0 to 0/90/10) and amino silica gel column chromatography (eluent: n-hexane/ethyl acetate/methanol = 100/0/0 to 0/100/0 to 0/90/10), and the title compound (0.007 g) was obtained.

### Example A-38

### Bicyclo[2.2.2]octan-2-yl (3-(2-cyanobenzyl)-2-fluoro-4-(2-(ureidomethyl)pyridin-3-yl)phenyl)carbamate

HOBt hydrate (0.022 g) and TEA (0.026 g) were sequentially added to a mixture of Reference Example E-8 (0.030 g), Reference Example I-5 (0.064 g), and MeCN (1.2 mL) at room temperature. The reaction mixture was refluxed for 27 hours. TEA (0.011 g) was added to the reaction mixture at room temperature. The reaction mixture was refluxed for 10 hours. The reaction mixture was cooled to room temperature and diluted with DCM. The mixture was washed with a saturated sodium bicarbonate aqueous solution, then, dried over anhydrous sodium sulfate, and concentrated under reduced pressure. The residue was sequentially purified by Method A (eluent: ethyl acetate/methanol = 100/0 to 87/13), silica gel column chromatography (eluent: ethyl acetate/methanol = 100/0 to 87/13), and amino silica gel column chromatography (eluent: ethyl acetate/methanol = 100/0/ to 89/11), and the title compound (0.002 g) was obtained.

### Example A-39

### tert-Butyl (4-(2-(3-amino-3-oxopropyl)pyridin-3-yl)-3-(2-cyanobenzyl)-2-fluorophenyl)carbamate

Pd(PPh₃)₄ (0.018 g) was added to a mixture of Reference Example A-10 (0.112 g), Reference Example C-7 (0.019 g), a 2 mol/L potassium carbonate aqueous solution (0.115 mL), ethanol (0.122 mL), and DME (0.5 mL) at room temperature. The reaction mixture was stirred at 120°C for one hour under microwave irradiation. Water was added to the reaction mixture, and the mixture was extracted with DCM. The extract was concentrated under reduced pressure. The residue was purified by silica gel column chromatography (eluent: ethyl acetate/methanol = 100/0 to 90/10), and tert-butyl (E)-(4-(2-(3-amino-3-oxopropan-1-en-1-yl)pyridin-3-yl)-3-(2-cyanobenzyl)-2-fluorophenyl)carbamate was obtained.

10% Pd/C (0.003 g) was added to a mixture of the obtained compound and methanol (1 mL) at room temperature. The reaction mixture was stirred at the same temperature for two hours under a hydrogen atmosphere. The reaction mixture was filtered through Celite, and the filtrate was concentrated under reduced pressure. The residue was purified by amino silica gel column chromatography (eluent: ethyl acetate/methanol = 100/0 to 90/10), and the title compound (0.019 g) was obtained.

### Example A-40

### tert-Butyl (4-(2-(3-amino-3-oxopropyl)pyridin-3-yl)-2-fluoro-3-(2-(methylsulfonyl)benzyl)phenyl)carbamate

Pd(PPh₃)₄ (0.007 g) was added to a mixture of Reference Example A-55 (0.027 g), Reference Example C-7 (0.007 g), a 2 mol/L potassium carbonate aqueous solution (0.043 mL), ethanol (0.043 mL), and DME (2 mL) at room temperature. The reaction mixture was stirred at 110°C for one hour under microwave irradiation. Water was added to the reaction mixture, and the mixture was extracted with DCM. The extract was concentrated under reduced pressure. The residue was purified by silica gel column chromatography (eluent: ethyl acetate/methanol = 100/0 to 75/25), and tert-butyl (E)-(4-(2-(3-amino-3-oxopropan-1-en-1-yl)pyridine-3-yl)-2-fluoro-3-(2-(methylsulfonyl)benzyl)phenyl)carbamate was obtained.

10% Pd/C (0.010 g) was added to a mixture of the obtained compound, methanol (1 mL), and THF (1 mL) at room temperature. The reaction mixture was stirred at the same temperature for one hour under a hydrogen atmosphere. The reaction mixture was filtered through Celite, and the filtrate was concentrated under reduced pressure. The residue was sequentially purified by amino silica gel column chromatography (eluent: ethyl acetate/methanol = 98/2 to 85/15) and silica gel column chromatography (eluent: ethyl acetate/methanol = 100/0 to 70/30), and the title compound (0.002 g) was obtained.

### Example B-1

### tert-Butyl (2-fluoro-3-phenoxy-4-(2-(ureidomethyl)pyridin-3-yl)phenyl)carbamate

Trimethylsilyl isocyanate (0.037 g) was added to a mixture of Reference Example F-3 (0.044 g) and THF (1 mL) at room temperature. The reaction mixture was stirred at the same temperature for 1.5 hours. The reaction mixture was concentrated under reduced pressure. The residue was purified by Method A (eluent: ethyl acetate/methanol = 100/0 to 92/8), and the title compound (0.041 g) was obtained.

### Example B-2

### tert-Butyl (2-fluoro-3-(2-fluorophenoxy)-4-(2-(ureidomethyl)pyridin-3-yl)phenyl)carbamate

Pd(PPh₃)₄ (0.018 g) was added to a mixture of Reference Example B-9 (0.034 g), Reference Example C-1 (0.017 g), a 2 mol/L potassium carbonate aqueous solution (0.114 mL), ethanol (0.114 mL), and toluene (2 mL) at room temperature. The reaction mixture was stirred at 110°C for one hour under microwave irradiation. Water was added to the reaction mixture, and the mixture was extracted with DCM. The extract was concentrated under reduced pressure. The residue was purified by silica gel column chromatography (eluent: ethyl acetate/methanol = 100/0 to 65/35), and the title compound (0.014 g) was obtained.

### Example B-3

### (S)-1,1,1-Trifluoropropan-2-yl (2-fluoro-3-(2-fluorophenoxy)-4-(2-(ureidomethyl)pyridin-3-yl)phenyl)carbamate

4-Nitrophenyl chloroformate (0.020 g) was added to a mixture of Reference Example F-4 (0.025 g) and THF (2 mL) under ice cooling. The reaction mixture was stirred under ice cooling for one hour and then at room temperature for 15 minutes. (S)-1,1,1-Trifluoropropan-2-ol (0.038 g) and DMAP (0.025 g) were added to the reaction mixture at room temperature. The reaction mixture was stirred at the same temperature for one hour. The reaction mixture was poured into a saturated sodium bicarbonate aqueous solution, and the mixture was extracted with DCM. The extract was concentrated under reduced pressure. The residue was purified by Method A (eluent: ethyl acetate/methanol = 100/0 to 85/15), and the title compound (0.016 g) was obtained.

Example B-4 was synthesized by the same method as in Example B-3 except that (3,3-difluorocyclobutyl)methanol was used in place of (S)-1,1,1-trifluoropropan-2-ol.

Example B-5 was synthesized by the same method as in Example A-5 except that Reference Example F-4 and 3,3-difluorocyclobutan-1-ol were used in place of Reference Example E-4 and trans-3-fluorocyclobutan-1-ol.

### Example B-6

### tert-Butyl (4-(2-(3-amino-3-oxopropyl)pyridin-3-yl)-2-fluoro-3-(2-fluorophenoxy)phenyl)carbamate

A mixture of Reference Example B-9 (0.021 g), Reference Example C-7 (0.010 g), Pd(PPh₃)₄ (0.010 g), a 2 mol/L potassium carbonate aqueous solution (0.069 mL), ethanol (0.069 mL), and toluene (1.2 mL) was stirred at 110°C for one hour under microwave irradiation. Water was added to the reaction mixture, and the mixture was extracted with DCM. The extract was dried over anhydrous sodium sulfate and concentrated under reduced pressure. The residue was sequentially purified by silica gel column chromatography (eluent: n-hexane/ethyl acetate/methanol = 0/10/90 to 0/100/0 to 0/98/2) and amino silica gel column chromatography (eluent: n-hexane/ethyl acetate/methanol = 50/50/0 to 0/100/0 to 0/95/5), and tert-butyl (E)-(4-(2-(3-amino-3-oxopropan-1-en-1-yl)pyridin-3-yl)-2-fluoro-3-(2-fluorophenoxy)phenyl)carbamate was obtained.

10% Pd/C (0.006 g) was added to a mixture of the obtained compound and methanol (1 mL) at room temperature under an argon atmosphere. The reaction mixture was stirred at the same temperature for 2.5 hours under a hydrogen atmosphere. The reaction mixture was filtered through Celite, and the filtrate was concentrated under reduced pressure. The residue was sequentially purified by silica gel column chromatography (eluent: n-hexane/ethyl acetate/methanol = 10/90/0 to 0/100/0 to 0/91/9) and ODS column chromatography (eluent: water/MeCN = 90/10 to 10/90), and the title compound (0.003 g) was obtained.

### Example B-7

### tert-Butyl (4-(2-(1-hydroxyethyl)pyridine-3-yl)-3-phenoxyphenyl)carbamate

Sodium borohydride (0.004 g) was added to a mixture of Reference Example F-5 (0.020 g) and methanol (1 mL) under ice cooling. The reaction mixture was stirred at the same temperature for 30 minutes. A saturated ammonium chloride aqueous solution was added to the reaction mixture, and the mixture was extracted with DCM. The extract was concentrated under reduced pressure. The residue was purified by Method A (eluent: n-hexane/ethyl acetate = 80/20 to 30/70), and the title compound (0.018 g) was obtained.

### Example C-1

### tert-Butyl (2-fluoro-3-(2-fluorobenzyl)-4-(3-(ureidomethyl)pyridazin-4-yl)phenyl)carbamate

A mixture of Reference Example G-1 (0.085 g), hydrazine monohydrate (0.076 g), and ethanol (1 mL) was refluxed for one hour. The reaction mixture was cooled to room temperature, a saturated sodium bicarbonate aqueous solution was added thereto, and the mixture was extracted with DCM. The extract was concentrated under reduced pressure.

A mixture of the residue, trimethylsilyl isocyanate (0.053 g), and THF (1 mL) was stirred at room temperature for 1.5 hours. The reaction mixture was concentrated under reduced pressure. The residue was purified by Method A (eluent: ethyl acetate/methanol = 98/2 to 85/15), and the title compound (0.054 g) was obtained.

### Example C-2

### (S)-1,1,1-Trifluoropropan-2-yl (2-fluoro-3-(2-fluorobenzyl)-4-(3-(ureidomethyl)pyridazin-4-yl)phenyl)carbamate

4-Nitrophenyl chloroformate (0.008 g) was added to a mixture of Reference Example G-2 (0.010 g) and THF (0.5 mL) under ice cooling. The reaction mixture was stirred under ice cooling for 30 minutes. (S)-1,1,1-trifluoropropan-2-ol (0.015 g) and DMAP (0.010 g) were added to the reaction mixture under ice cooling. The reaction mixture was stirred at room temperature for 14 hours. A saturated sodium bicarbonate aqueous solution was added to the reaction mixture, and the mixture was extracted with DCM. The extract was concentrated under reduced pressure. The residue was purified by Method A (eluent: ethyl acetate/methanol = 98/2 to 85/15), and the title compound (0.007 g) was obtained.

### Example C-3

### 1-Methylcyclopropyl (2-fluoro-3-(2-fluorobenzyl)-4-(3-(ureidomethyl)pyridazin-4-yl)phenyl)carbamate

A mixture of Reference Example G-3 (0.014 g), hydrazine monohydrate (0.012 g), and ethanol (1 mL) was refluxed for one hour. The reaction mixture was cooled to room temperature, a saturated sodium bicarbonate aqueous solution was added thereto, and the mixture was extracted with DCM. The extract was concentrated under reduced pressure.

A mixture of the residue, trimethylsilyl isocyanate (0.009 g), and THF (1 mL) was stirred at room temperature for one hour. The reaction mixture was concentrated under reduced pressure. The residue was purified by silica gel column chromatography (eluent: ethyl acetate/methanol = 98/2 to 85/15), and the title compound (0.007 g) was obtained.

### Example C-4

### tert-Butyl (4-(3-(3-amino-3-oxopropyl)pyridazin-4-yl)-2-fluoro-3-(2-fluorobenzyl)phenyl)carbamate

A 4 mol/L lithium hydroxide aqueous solution (0.055 mL) was added to a mixture of Reference Example G-7 (0.011 g), THF (0.5 mL), and methanol (0.5 mL) under ice cooling. The reaction mixture was stirred at room temperature for four hours. Water and 2 mol/L hydrochloric acid were added to the reaction mixture, and the mixture was extracted with DCM. The extract was concentrated under reduced pressure.

15 mol/L ammonia water (0.015 mL) was added to a mixture of the residue, DMT-MM (0.010 g), and MeCN (1 mL) at room temperature. The reaction mixture was stirred at the same temperature for 30 minutes. A saturated ammonium chloride aqueous solution was added to the reaction mixture, and the mixture was extracted with DCM. The extract was concentrated under reduced pressure. The residue was purified by amino silica gel column chromatography (eluent: ethyl acetate/methanol = 90/10 to 60/40), and tert-butyl (E)-(4-(3-(3-amino-3-oxopropan-1-en-1-yl)pyridazin-4-yl)-2-fluoro-3-(2-fluorobenzyl)phenyl)carbamate was obtained.

10% Pd/C (0.010 g) was added to a mixture of the obtained compound, methanol (1 mL), and THF (1 mL) at room temperature. The reaction mixture was stirred at the same temperature for one hour under a hydrogen atmosphere. The reaction mixture was filtered through Celite, and the filtrate was concentrated under reduced pressure. The residue was purified by silica gel column chromatography (eluent: ethyl acetate/methanol = 100/0 to 70/30), and the title compound (0.003 g) was obtained.

### Example C-5

### (S)-1,1,1-Trifluoropropan-2-yl (4-(3-(3-amino-3-oxopropyl)pyridazin-4-yl)-2-fluoro-3-(2-fluorobenzyl)phenyl)carbamate

4-Nitrophenyl chloroformate (0.008 g) and pyridine (0.004 g) were added to a mixture of Reference Example G-8 (0.009 g) and THF (1 mL) under ice cooling. The reaction mixture was stirred under ice cooling for one hour. (S)-1,1,1-trifluoropropan-2-ol (0.015 g) and DMAP (0.005 g) were added to the reaction mixture under ice cooling. The reaction mixture was stirred at room temperature for 40 minutes. (S)-1,1,1-Trifluoropropan-2-ol (0.015 g) and TEA (0.008 g) were added to the reaction mixture at room temperature. The reaction mixture was stirred at the same temperature for one hour. The reaction mixture was partitioned between a saturated sodium bicarbonate aqueous solution and DCM. The organic layer was separated and concentrated under reduced pressure. The residue was purified by Method A (eluent: ethyl acetate/methanol = 100/0 to 70/30), and the title compound (0.008 g) was obtained.

### Example C-6

### (R)-1-Cyclopropylethyl (4-(3-(3-amino-3-oxopropyl)pyridazin-4-yl)-2-fluoro-3-(2-fluorobenzyl)phenyl)carbamate

4-Nitrophenyl chloroformate (0.008 g) was added to a mixture of Reference Example G-8 (0.010 g) and THF (1 mL) under ice cooling. The reaction mixture was stirred under ice cooling for 30 minutes. (R)-1-Cyclopropylethane-1-ol (0.012 g) and DMAP (0.010 g) were added to the reaction mixture under ice cooling. The reaction mixture was stirred at room temperature for one hour and then at 40°C for two hours. The reaction mixture was cooled to room temperature, a saturated sodium bicarbonate aqueous solution was added thereto, and the mixture was extracted with DCM. The extract was concentrated under reduced pressure. The residue was purified by Method A (eluent: ethyl acetate/methanol = 100/0 to 90/10), and the title compound (0.004 g) was obtained.

### Example C-7

### trans-3-Fluorocyclobutyl (4-(3-(3-amino-3-oxopropyl)pyridazin-4-yl)-2-fluoro-3-(2-fluorobenzyl)phenyl)carbamate

4-Nitrophenyl chloroformate (0.003 g) was added to a mixture of Reference Example G-8 (0.004 g) and THF (1 mL) under ice cooling. The reaction mixture was stirred at room temperature for five minutes. trans-3-Fluorocyclobutan-1-ol (0.006 g) and DMAP (0.002 g) were added to the reaction mixture at room temperature. The reaction mixture was stirred at the same temperature for three hours. The reaction mixture was poured into a saturated sodium bicarbonate aqueous solution, and the mixture was extracted with DCM. The extract was concentrated under reduced pressure. The residue was purified by silica gel column chromatography (eluent: ethyl acetate/methanol = 100/0 to 70/30), and the title compound (0.002 g) was obtained.

Example C-8 was synthesized by the same method as in Example C-6 except that 3,3-difluorocyclobutan-1-ol was used in place of (R)-1-cyclopropylethan-1-ol.

### Example C-9

### tert-Butyl (2-fluoro-3-(2-fluorobenzyl)-4-(3-methylpyridazin-4-yl)phenyl)carbamate

A mixture of Reference Example G-6 (0.020 g), Pd(PPh₃)₄ (0.005 g), tetramethyltin (0.013 g), and N-methylpyrrolidone (1 mL) was stirred at 140°C for 10 minutes under microwave irradiation. Water was poured into the reaction mixture, and the mixture was extracted with ethyl acetate. The extract was washed with water and a saturated saline solution, then, dried over anhydrous magnesium sulfate, and concentrated under reduced pressure. The residue was sequentially purified by silica gel column chromatography (eluent: n-hexane/ethyl acetate = 80/20 to 10/90) and amino silica gel column chromatography (eluent: n-hexane/ethyl acetate = 70/30 to 20/80), and the title compound (0.004 g) was obtained.

Example C-10 was synthesized by the same method as in Example C-3 except that Reference Example G-9 was used in place of Reference Example G-3.

### Example D-1

### tert-Butyl (2-fluoro-3-(2-fluorophenoxy)-4-(3-(ureidomethyl)pyridazin-4-yl)phenyl)carbamate

A mixture of Reference Example H-1 (0.060 g), hydrazine monohydrate (0.054 g), and ethanol (1 mL) was refluxed for one hour. The reaction mixture was cooled to room temperature, a saturated sodium bicarbonate aqueous solution was added thereto, and the mixture was extracted with DCM. The extract was concentrated under reduced pressure.

A mixture of the residue, trimethylsilyl isocyanate (0.037 g), and THF (0.5 mL) was stirred at room temperature for one hour. The reaction mixture was concentrated under reduced pressure. The residue was sequentially purified by Method A (eluent: ethyl acetate/methanol = 98/2 to 85/15) and ODS column chromatography (eluent: water/MeCN = 70/30 to 10/90), and the title compound (0.020 g) was obtained.

### Example D-2

### (S)-1,1,1-Trifluoropropan-2-yl (2-fluoro-3-(2-fluorophenoxy)-4-(3-(ureidomethyl)pyridazin-4-yl)phenyl)carbamate

4-Nitrophenyl chloroformate (0.013 g) was added to a mixture of Reference Example H-2 (0.016 g) and THF (0.5 mL) under ice cooling. The reaction mixture was stirred under ice cooling for one hour. (S)-1,1,1-Trifluoropropan-2-ol (0.025 g) and DMAP (0.016 g) were added to the reaction mixture under ice cooling. The reaction mixture was stirred at room temperature for one hour. A saturated sodium bicarbonate aqueous solution was added to the reaction mixture, and the mixture was extracted with DCM. The extract was concentrated under reduced pressure. The residue was purified by Method A (eluent: ethyl acetate/methanol = 98/2 to 85/15), and the title compound (0.014 g) was obtained.

### Example D-3

### tert-Butyl (4-(3-(3-amino-3-oxopropyl)pyridazin-4-yl)-2-fluoro-3-(2-fluorophenoxy)phenyl)carbamate

A 4 mol/L lithium hydroxide aqueous solution (0.171 mL) was added to a mixture of Reference Example H-6 (0.034 g), THF (0.5 mL), and methanol (0.5 mL) at room temperature. The reaction mixture was stirred at room temperature for 30 minutes. Water and 1 mol/L hydrochloric acid were added to the reaction mixture, and the mixture was extracted with DCM. The extract was concentrated under reduced pressure.

15 mol/L ammonia water (0.046 mL) was added to a mixture of the residue, DMT-MM (0.028 g), and MeCN (1 mL) at room temperature. The reaction mixture was stirred at the same temperature for 15 minutes. A saturated ammonium chloride aqueous solution was added to the reaction mixture, and the mixture was extracted with DCM. The extract was concentrated under reduced pressure.

A mixture of the residue, 10% Pd/C (0.032 g), and THF (2 mL) was stirred at room temperature for one hour under a hydrogen atmosphere. The reaction mixture was filtered through Celite, and the filtrate was concentrated under reduced pressure. The residue was purified by silica gel column chromatography (eluent: ethyl acetate/methanol = 95/5 to 70/30), and the title compound (0.020 g) was obtained.

### Example D-4

### (S)-1,1,1-Trifluoropropan-2-yl (4-(3-(3-amino-3-oxopropyl)pyridazin-4-yl)-2-fluoro-3-(2-fluorophenoxy)phenyl)carbamate

4-Nitrophenyl chloroformate (0.007 g) was added to a mixture of Reference Example H-7 (0.008 g) and THF (2 mL) under ice cooling. The reaction mixture was stirred under ice cooling for 30 minutes and then at room temperature for 15 minutes. (S)-1,1,1-Trifluoropropan-2-ol (0.012 g) and DMAP (0.008 g) were added to the reaction mixture at room temperature. The reaction mixture was stirred at the same temperature for one hour. A saturated sodium bicarbonate aqueous solution was poured into the reaction mixture, and the mixture was extracted with DCM. The extract was concentrated under reduced pressure. The residue was purified by amino silica gel column chromatography (eluent: ethyl acetate/methanol = 100/0 to 80/20), and the title compound (0.007 g) was obtained.

### Example E-1

### tert-Butyl (2-fluoro-3-(2-methylbenzoyl)-4-(2-(ureidomethyl)pyridin-3-yl)phenyl)carbamate

Pd(PPh₃)₄ (0.013 g) was added to a mixture of Reference Example A-62 (0.025 g), Reference Example C-1 (0.014 g), a 2 mol/L potassium carbonate aqueous solution (0.082 mL), ethanol (0.082 mL), and toluene (0.5 mL) at room temperature. The reaction mixture was stirred at 100°C for one hour. The reaction mixture was cooled to room temperature, water was added thereto, and the mixture was extracted with DCM. The extract was concentrated under reduced pressure. The residue was sequentially purified by Method A (eluent: n-hexane/ethyl acetate/methanol = 49/49/2 to 0/98/2 to 0/86/14) and silica gel column chromatography (eluent: ethyl acetate/methanol = 98/2 to 85/15), and the title compound (0.008 g) was obtained.

Example E-2 was synthesized by the same method as in Example A-14 except that Reference Example A-65 was used in place of Reference Example A-36.

### Example E-3

### tert-Butyl (4-(2-(3-amino-3-oxopropyl)pyridin-3-yl)-2-fluoro-3-(2-fluorobenzoyl)phenyl)carbamate

Pd(PPh₃)₄ (0.025 g) was added to a mixture of Reference Example A-68 (0.050 g), Reference Example C-7 (0.027 g), a 2 mol/L potassium carbonate aqueous solution (0.163 mL), ethanol (0.163 mL), and toluene (1 mL) at room temperature. The reaction mixture was stirred at 100°C for one hour under microwave irradiation. Water was added to the reaction mixture, and the mixture was extracted with DCM. The extract was concentrated under reduced pressure. The residue was purified by Method A (eluent: n-hexane/ethyl acetate/methanol = 49/49/2 to 0/98/2 to 0/86/14), and tert-butyl (E)-(4-(2-(3-amino-3-oxopropan-1-en-1-yl)pyridine-3-yl)-2-fluoro-3-(2-fluorobenzoyl)phenyl)carbamate was obtained.

A mixture of the obtained compound, 10% Pd/C (0.005 g), and methanol (1 mL) was stirred at room temperature for one hour under a hydrogen atmosphere. The reaction mixture was filtered through Celite, and the filtrate was concentrated under reduced pressure. The residue was purified by Method A (eluent: ethyl acetate/methanol = 98/2 to 88/12), and the title compound (0.022 g) was obtained.

### Example E-4

### (S)-1,1,1-Trifluoropropan-2-yl (4-(2-(3-amino-3-oxopropyl)pyridin-3-yl)-2-fluoro-3-(2-fluorobenzoyl)phenyl)carbamate

4-Nitrophenyl chloroformate (0.013 g) was added to a mixture of Reference Example E-9 (0.016 g) and THF (1 mL) under ice cooling. The reaction mixture was stirred under ice cooling for one hour. (S)-1,1,1-Trifluoropropan-2-ol (0.023 g), DMAP (0.007 g), and TEA (0.012 g) were added to the reaction mixture under ice cooling. The reaction mixture was stirred at room temperature for four hours. The reaction mixture was partitioned between a saturated sodium bicarbonate aqueous solution and DCM. The organic layer was separated and concentrated under reduced pressure. The residue was purified by Method A (eluent: ethyl acetate/methanol = 100/0 to 80/20), and the title compound (0.010 g) was obtained.

### Example E-5

### (S)-1-Cyclopropylethyl (4-(2-(3-amino-3-oxopropyl)pyridin-3-yl)-2-fluoro-3-(2-fluorobenzoyl)phenyl)carbamate

4-Nitrophenyl chloroformate (0.014 g) was added to a mixture of Reference Example E-9 (0.017 g) and THF (1.2 mL) under ice cooling. The reaction mixture was stirred under ice cooling for one hour. (S)-1-Cyclopropylethane-1-ol (0.019 g), DMAP (0.009 g), and TEA (0.014 g) were sequentially added to the reaction mixture under ice cooling. The reaction mixture was stirred at room temperature for 1.5 hours. A saturated sodium bicarbonate aqueous solution was added to the reaction mixture, and the mixture was extracted with ethyl acetate. The extract was dried over anhydrous sodium sulfate and concentrated under reduced pressure. The residue was purified by Method A (eluent: n-hexane/ethyl acetate/methanol = 5/95/0 to 0/100/0 to 0/90/10), and the title compound (0.014 g) was obtained.

Example E-6 and Example E-7 were synthesized by the same method as in Example E-5 except that corresponding raw materials were used in place of (S)-1-cyclopropylethan-1-ol.

### Example E-8

### tert-Butyl (4-(2-(3-amino-3-oxopropyl)pyridin-3-yl)-2-fluoro-3-(2-(trifluoromethyl)benzoyl)phenyl)carbamate

Pd(PPh₃)₄ (0.045 g) was added to a mixture of Reference Example A-65 (0.100 g), Reference Example C-7 (0.045 g), a 2 mol/L potassium carbonate aqueous solution (0.295 mL), ethanol (0.295 mL), and toluene (1,5 mL) at room temperature. The reaction mixture was stirred at 110°C for one hour under microwave irradiation. Water was added to the reaction mixture, and the mixture was extracted with DCM. The extract was concentrated under reduced pressure. The residue was purified by silica gel column chromatography (eluent: ethyl acetate/methanol = 98/2 to 85/15), and tert-butyl (E)-(4-(2-(3-amino-3-oxopropan-1-en-1-yl)pyridine-3-yl)-2-fluoro-3-(2-(trifluoromethyl)benzoyl)phenyl)carbamate was obtained.

10% Pd/C (0.008 g) was added to a mixture of the obtained compound and methanol (2 mL) at room temperature. The reaction mixture was stirred at the same temperature for 1.5 hours under a hydrogen atmosphere. 10% Pd/C (0.008 g) was added to the reaction mixture at room temperature. The reaction mixture was stirred at the same temperature for three hours under a hydrogen atmosphere. The reaction mixture was left to stand at room temperature for 64 hours under an argon atmosphere. The reaction mixture was stirred at the same temperature for three hours under a hydrogen atmosphere. The reaction mixture was filtered through Celite, and the filtrate was concentrated under reduced pressure. The residue was purified by silica gel column chromatography (eluent: ethyl acetate/methanol = 98/2 to 85/15), and a mixture of tert-butyl (E)-(4-(2-(3-amino-3-oxopropan-1-en-1-yl)pyridine-3-yl)-2-fluoro-3-(2-(trifluoromethyl)benzoyl)phenyl)carbamate and the title compound was obtained.

A mixture of the obtained compound, 10% Pd/C (0.016 g), and methanol (3 mL) was stirred at room temperature for 30 minutes under a hydrogen atmosphere. The reaction mixture was filtered through Celite, and the filtrate was concentrated under reduced pressure. The residue was purified by silica gel column chromatography (eluent: ethyl acetate/methanol = 100/0 to 85/15), and the title compound (0.045 g) was obtained.

### Example E-9

### 3,3-Difluorocyclobutyl (4-(2-(3-amino-3-oxopropyl)pyridin-3-yl)-2-fluoro-3-(2-(trifluoromethyl)benzoyl)phenyl)carbamate

A mixture of Reference Example E-10 (0.015 g), 4-nitrophenyl chloroformate (0.011 g), and THF (2 mL) was stirred under ice cooling for 15 minutes and then at room temperature for 15 minutes. 3,3-Difluorocyclobutan-1-ol (0.019 g) and DMAP (0.013 g) were added to the reaction mixture at room temperature. The reaction mixture was stirred at the same temperature for one hour. The reaction mixture was poured into a saturated sodium bicarbonate aqueous solution, and the mixture was extracted with DCM. The extract was concentrated under reduced pressure. The residue was purified by Method B (eluent: ethyl acetate/methanol = 100/0 to 85/15), and the title compound (0.012 g) was obtained.

### Example F-1

### tert-Butyl (2-fluoro-3-((3-fluoropyridin-2-yl)methyl)-4-(2-(ureidomethyl)pyridin-3-yl)phenyl)carbamate

Pd(PPh₃)₄ (0.021 g) was added to a mixture of Reference Example A-74 (0.080 g), Reference Example C-1 (0.031 g), a 2 mol/L potassium carbonate aqueous solution (0.134 mL), ethanol (0.134 mL), and toluene (1 mL) at room temperature. The reaction mixture was stirred at 100°C for one hour under microwave irradiation. Water was added to the reaction mixture, and the mixture was extracted with DCM. The extract was concentrated under reduced pressure. The residue was sequentially purified by amino silica gel column chromatography (eluent: n-hexane/ethyl acetate/methanol = 29/69/2 to 0/98/2 to 0/83/17) and silica gel column chromatography (eluent: ethyl acetate/methanol = 98/2 to 85/15), and the title compound (0.012 g) was obtained.

### Example F-2

### tert-Butyl (4-(2-(3-amino-3-oxopropyl)pyridin-3-yl)-2-fluoro-3-((3-fluoropyridin-2-yl)methyl)phenyl)carbamate

Pd(amphos)₂Cl₂ (0.011 g) was added to a mixture of Reference Example A-74 (0.035 g), Reference Example C-7 (0.020 g), a 2 mol/L sodium carbonate aqueous solution (0.078 mL), and 4-methyltetrahydropyran (1 mL) at room temperature. The reaction mixture was stirred at 100°C for three hours. The reaction mixture was cooled to room temperature, water was added thereto, and the mixture was extracted with DCM. The extract was concentrated under reduced pressure. The residue was purified by Method A (eluent: n-hexane/ethyl acetate/methanol = 49/49/2 to 0/98/2 to 0/86/14), and tert-butyl (E)-(4-(2-(3-amino-3-oxopropan-1-en-1-yl)pyridin-3-yl)-2-fluoro-3-((3-fluoropyridine-2-yl)methyl)phenyl)carbamate was obtained.

A mixture of the obtained compound, 10% Pd/C (0.005 g), and methanol (1 mL) was stirred at room temperature for one hour under a hydrogen atmosphere. The reaction mixture was filtered through Celite, and the filtrate was concentrated under reduced pressure. The residue was purified by Method A (eluent: ethyl acetate/methanol = 98/2 to 85/15), and the title compound (0.005 g) was obtained.

### Example F-3

### (R)-1-Cyclopropylethyl (4-(2-(3-amino-3-oxopropyl)pyridin-3-yl)-2-fluoro-3-((3-fluoropyridin-2-yl)methyl)phenyl)carbamate

4-Nitrophenyl chloroformate (0.011 g) was added to a mixture of Reference Example E-12 (0.013 g) and THF (0.5 mL) under ice cooling. The reaction mixture was stirred under ice cooling for 30 minutes. (R)-1-Cyclopropyletan-1-ol (0.015 g) and DMAP (0.013 g) were added to the reaction mixture under ice cooling. The reaction mixture was stirred at room temperature for one hour. TEA (0.011 g) was added to the reaction mixture at room temperature. The reaction mixture was stirred at the same temperature for two hours. A saturated sodium bicarbonate aqueous solution was added to the reaction mixture, and the mixture was extracted with DCM. The extract was concentrated under reduced pressure. The residue was purified by Method A (eluent: ethyl acetate/methanol = 98/2 to 85/15), and the title compound (0.005 g) was obtained.

### Example F-4

### tert-Butyl (2-fluoro-3-(morpholinomethyl)-4-(2-(ureidomethyl)pyridin-3-yl)phenyl)carbamate

Pd(PPh₃)₄ (0.016 g) was added to a mixture of Reference Example A-77 (0.030 g), Reference Example C-1 (0.014 g), a 2 mol/L potassium carbonate aqueous solution (0.103 mL), ethanol (0.103 mL), and toluene (2 mL) at room temperature. The reaction mixture was stirred at 100°C for one hour under microwave irradiation. Water was added to the reaction mixture, and the mixture was extracted with DCM. The extract was concentrated under reduced pressure. The residue was sequentially purified by amino silica gel column chromatography (eluent: ethyl acetate/methanol = 100/0 to 75/25), silica gel column chromatography (eluent: ethyl acetate/methanol = 95/5 to 60/40), and ODS column chromatography (eluent: water/MeCN = 90/10 to 10/90), and the title compound (0.005 g) was obtained.

### Example F-5

### tert-Butyl (S)-(2-fluoro-3-((2-methylmorpholino)methyl)-4-(2-(ureidomethyl)pyridin-3-yl)phenyl)carbamate

(S)-2-Methylmorpholine (0.007 g) was added to a mixture of Reference Example E-7 (0.010 g) and THF (0.5 mL) at room temperature. The reaction mixture was stirred at the same temperature for one hour. Water was added to the reaction mixture, and the mixture was extracted with DCM. The extract was concentrated under reduced pressure. The residue was purified by amino silica gel column chromatography (eluent: ethyl acetate/methanol = 100/0 to 90/10), and the title compound (0.011 g) was obtained.

### Example F-6

### tert-Butyl (3-((3,3-difluoropiperidin-1-yl)methyl)-2-fluoro-4-(2-(ureidomethyl)pyridin-3-yl)phenyl)carbamate

Methanesulfonyl chloride (0.009 g) was added to a mixture of Reference Example E-6 (0.022 g), TEA (0.016 g), and DCM (1 mL) at room temperature. The reaction mixture was stirred at room temperature for 30 minutes. Water was added to the reaction mixture, and the mixture was extracted with DCM. The extract was concentrated under reduced pressure.

3,3-Difluoropiperidine hydrochloride (0.025 g) and sodium hydride (about 60%) (0.013 g) were added to a mixture of the residue and THF (1 mL) at room temperature. The reaction mixture was stirred at 60°C for five hours. Potassium iodide (0.017 g) was added to the reaction mixture at 60°C. The reaction mixture was stirred at the same temperature for one hour. The reaction mixture was cooled to room temperature, water was added thereto, and the mixture was extracted with ethyl acetate. The extract was washed with a saturated saline solution, dried over anhydrous sodium sulfate, and concentrated under reduced pressure. The residue was purified by silica gel column chromatography (eluent: ethyl acetate/methanol = 100/0 to 93/7), and the title compound (0.003 g) was obtained.

### Example F-7

### tert-Butyl (3-((8-oxa-3-azabicyclo[3.2.1]octan-3-yl)methyl)-2-fluoro-4-(2-(ureidomethyl)pyridin-3-yl)phenyl)carbamate

8-oxa-3-Azabicyclo[3.2.1]octane (0.010 g) was added to a mixture of Reference Example E-7 (0.020 g) and THF (1 mL) at room temperature. The reaction mixture was stirred at 50°C for 30 minutes. The reaction mixture was concentrated under reduced pressure. The residue was purified by silica gel column chromatography (eluent: ethyl acetate/methanol = 95/5 to 70/30), and the title compound (0.017 g) was obtained.

### Example F-8

### tert-Butyl (2-fluoro-3-(morpholine-4-carbonyl)-4-(2-(ureidomethyl)pyridin-3-yl)phenyl)carbamate

Pd(PPh₃)₄ (0.051 g) was added to a mixture of Reference Example A-81 (0.100 g), Reference Example C-1 (0.051 g), a 2 mol/L potassium carbonate aqueous solution (0.333 mL), ethanol (0.333 mL), and toluene (2 mL) at room temperature. The reaction mixture was stirred at 110°C for one hour under microwave irradiation. Water was added to the reaction mixture, and the mixture was extracted with DCM. The extract was concentrated under reduced pressure. The residue was sequentially purified by silica gel column chromatography (eluent: ethyl acetate/methanol = 100/0 to 75/25) and ODS column chromatography (eluent: water/MeCN = 80/20 to 30/70), and the title compound (0.010 g) was obtained.

### Example F-9

### tert-Butyl (3-(cyclopropylmethyl)-2-fluoro-4-(2-(ureidomethyl)pyridin-3-yl)phenyl)carbamate

Pd(PPh₃)₄ (0.015 g) was added to a mixture of Reference Example A-86 (0.025 g), Reference Example C-1 (0.015 g), potassium carbonate (0.027 g), water (0.089 mL), ethanol (0.089 mL), and toluene (0.5 mL) at room temperature. The reaction mixture was stirred at 100°C for one hour under microwave irradiation. Water was added to the reaction mixture, and the mixture was extracted with DCM. The extract was concentrated under reduced pressure. The residue was sequentially purified by amino silica gel column chromatography (eluent: ethyl acetate/methanol = 90/10 to 50/50) and ODS column chromatography (eluent: water/MeCN = 70/30 to 10/90), and the title compound (0.006 g) was obtained.

### Example G-1

### N-(2-Fluoro-3-(2-fluorobenzyl)-4-(2-(ureidomethyl)pyridin-3-yl)phenyl)-1-methylcyclopropane-1-carboxamide

A mixture of Reference Example E-4 (0.020 g), 1-methylcyclopropane-1-carboxylic acid (0.011 g), DIPEA (0.021 g), T3P (1.6 mol/L in DMF) (0.068 mL), and DCM (1 mL) was stirred at room temperature for 16 hours. 1-Methylcyclopropane-1-carboxylic acid (0.011 g), DIPEA (0.021 g), and T3P (1.6 mol/L in DMF) (0.068 mL) were added to the reaction mixture at room temperature. The reaction mixture was stirred at the same temperature for 24 hours. A saturated ammonium chloride aqueous solution and water were added to the reaction mixture, and the mixture was extracted with DCM. The extract was concentrated under reduced pressure. The residue was purified by Method A (eluent: ethyl acetate/methanol = 100/0 to 90/10), and the title compound (0.010 g) was obtained.

### Example G-2

### N-(2-Fluoro-3-(2-fluorobenzyl)-4-(2-(ureidomethyl)pyridin-3-yl)phenyl)isobutylamide

A mixture of Reference Example E-4 (0.020 g), isobutyric acid (0.010 g), DIPEA (0.021 g), T3P (1.6 mol/L in DMF) (0.068 mL), and DCM (1 mL) was stirred at room temperature for three hours. Isobutyric acid (0.010 g) and T3P (1.6 mol/L in DMF) (0.068 mL) were added to the reaction mixture. The reaction mixture was stirred at the same temperature for one hour and then refluxed for 30 minutes. The reaction mixture was cooled to room temperature and stirred for three hours. A saturated ammonium chloride aqueous solution and water were added to the reaction mixture, and the mixture was extracted with DCM. The extract was concentrated under reduced pressure. The residue was purified by silica gel column chromatography (eluent: n-hexane/ethyl acetate/methanol = 50/50/0 to 0/100/0 to 0/85/15), and the title compound (0.010 g) was obtained.

### Example G-3

### N-(2-Fluoro-3-(2-fluorobenzyl)-4-(2-(ureidomethyl)pyridin-3-yl)phenyl)cyclopropanecarboxamide

A mixture of Reference Example E-4 (0.020 g), cyclopropanecarboxylic acid (0.009 g), DIPEA (0.021 g), T3P (1.6 mol/L in DMF) (0.068 mL), and DCM (1 mL) was stirred at room temperature for 24 hours. A saturated ammonium chloride aqueous solution and water were added to the reaction mixture, and the mixture was extracted with DCM. The extract was concentrated under reduced pressure. The residue was purified by Method A (eluent: n-hexane/ethyl acetate/methanol = 50/50/0 to 0/100/0 to 0/88/12), and the title compound (0.015 g) was obtained.

### Example G-4

### 3,3-Difluoro-N-(2-fluoro-3-(2-fluorobenzyl)-4-(2-(ureidomethyl)pyridin-3-yl)phenyl)cyclobutane-1-carboxamide

A mixture of Reference Example E-4 (0.020 g), 3,3-difluorocyclobutane-1-carboxylic acid (0.015 g), DIPEA (0.021 g), T3P (1.6 mol/L in DMF) (0.068 mL), and DCM (1 mL) was stirred at room temperature for 24 hours. A saturated ammonium chloride aqueous solution and water were added to the reaction mixture, and the mixture was extracted with DCM. The extract was concentrated under reduced pressure. The residue was purified by silica gel column chromatography (eluent: n-hexane/ethyl acetate/methanol = 50/50/0 to 0/100/0 to 0/88/12), and the title compound (0.019 g) was obtained.

Example G-5 was synthesized by the same method as in Example G-4 except that tetrahydrofuran-2-carboxylic acid was used in place of 3,3-difluorocyclobutane-1-carboxylic acid.

Example G-6 was synthesized by the same method as in Example A-30 except that pyrrolidine was used in place of 3-dimethylamino-1-propanol.

### Example G-7

### N-(2-Fluoro-3-(2-fluorobenzyl)-4-(2-(ureidomethyl)pyridin-3-yl)phenyl)-2-azaspiro[3.3]heptane-2-carboxamide

4-Nitrophenyl chloroformate (0.021 g) was added to a mixture of Reference Example E-4 (0.024 g) and THF (0.7 mL) at room temperature. The reaction mixture was stirred at the same temperature for 30 minutes. 2-Azaspiro[3.3]heptane (0.033 g) was added to the reaction mixture at room temperature. The reaction mixture was stirred at the same temperature for one hour. A saturated sodium bicarbonate aqueous solution was added to the reaction mixture, and the mixture was extracted with ethyl acetate. The extract was dried over anhydrous sodium sulfate and concentrated under reduced pressure. The residue was purified by Method A (eluent: ethyl acetate/methanol = 100/0 to 85/15), and the title compound (0.024 g) was obtained.

### Example G-8

### 1-Cyclopentyl-3-(2-fluoro-3-(2-fluorobenzyl)-4-(2-(ureidomethyl)pyridin-3-yl)phenyl)urea

4-Nitrophenyl chloroformate (0.014 g) was added to a mixture of Reference Example E-4 (0.017 g) and THF (0.5 mL) under ice cooling. The reaction mixture was stirred under ice cooling for 15 minutes. Cyclopentylamine (0.019 g) was added to the reaction mixture under ice cooling. The reaction mixture was stirred at the same temperature for 30 minutes. The reaction mixture was partitioned between a saturated sodium bicarbonate aqueous solution and DCM. The organic layer was separated and concentrated under reduced pressure. The residue was purified by Method A (eluent: ethyl acetate/methanol = 100/0 to 95/5), and the title compound (0.018 g) was obtained.

### Example G-9

### 1-Cyclopentyl-3-(2-fluoro-3-(2-fluorobenzyl)-4-(2-(ureidomethyl)pyridin-3-yl)phenyl)-1-methoxyurea

4-Nitrophenyl chloroformate (0.014 g) was added to a mixture of Reference Example E-4 (0.022 g) and THF (1 mL) under ice cooling. The reaction mixture was stirred under ice cooling for 30 minutes. N-Cyclopentyl-O-methylhydroxylamine hydrochloride (0.015 g) and DIPEA (0.024 g) were added to the reaction mixture under ice cooling. The reaction mixture was stirred at room temperature for 30 minutes. The reaction mixture was partitioned between a saturated sodium bicarbonate aqueous solution and DCM. The organic layer was separated and concentrated under reduced pressure. The residue was purified by Method A (eluent: ethyl acetate/methanol = 100/0 to 80/20), and the title compound (0.027 g) was obtained.

### Example H-1

### tert-Butyl (4-(3-(3-amino-3-oxopropyl)pyrazin-2-yl)-3-benzyl-2-fluorophenyl)carbamate

Pd(PPh₃)₄ (0.027 g) was added to a mixture of Reference Example A-20 (0.060 g), Reference Example J-2 (0.026 g), a 2 mol/L potassium carbonate aqueous solution (0.173 mL), ethanol (0.173 mL), and toluene (1 mL) at room temperature. The reaction mixture was stirred at 100°C for one hour under microwave irradiation. Water was added to the reaction mixture, and the mixture was extracted with DCM. The extract was concentrated under reduced pressure. The residue was purified by Method A (eluent: n-hexane/ethyl acetate/methanol = 48.5/48.5/3 to 0/97/3), and tert-butyl (E)-(4-(3-(3-amino-3-oxopropane-1-en-1-yl)pyrazine-2-yl)-3-benzyl-2-fluorophenyl)carbamate was obtained.

A mixture of the obtained compound, 10% Pd/C (0.006 g), methanol (1 mL), and THF (1 mL) was stirred at room temperature for one hour under a hydrogen atmosphere. The reaction mixture was filtered through Celite, and the filtrate was concentrated under reduced pressure. The residue was purified by Method A (eluent: ethyl acetate/methanol = 98/2 to 88/12), and the title compound (0.016 g) was obtained.

The structural formulae, physical property data, and TSHR antagonist activities (refer to Test Example 1) of examples are shown in the following tables.

**[Table 17]**

| Ex. No. | Str. | Phys. data | IC₅₀ |
|---|---|---|---|
| A-1 | 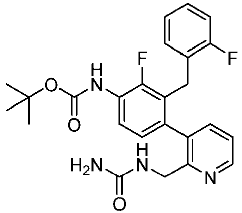 | ¹H-NMR (CDCl₃) δ ppm : 1. 55 (9H, s), 3.67 (1H, d, J=15. 8Hz), 3.82 (1H, dd, J=4.4, 16. 7Hz), 3.91 (1H, d, J=15. 8Hz), 4. 05-4. 20 (1H, m), 4. 30-4. 83 (2H, m), 5.84 (1H, br), 6. 70-6. 78 (1H, m), 6.79-6.88 (2H, m), 6. 89-6. 96 (2H, m), 7. 05-7. 21 (2H, m), 7. 24-7. 32 (1H, m), 8.00-8.15 (1H, m), 8.51 (1H, dd, J=1. 8, 4. 8Hz) | A |
| | | MS m/z : 469 (M+H)⁺ | |
| A-2 | 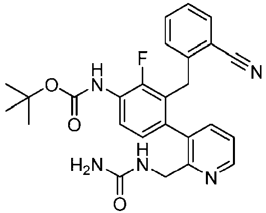 | ¹H-NMR (DMSO-d₆) δ ppm : 1.47 (9H, s), 3.67 (1H, dd, J=4.9, 16. 4Hz), 3.84 (1H, d, J=16. 1Hz), 3. 93-4. 11 (2H, m), 5.65 (2H, br), 6. 25-6. 43 (1H, m), 6. 92 (1H, d, J=7. 8Hz), 7. 03 (1H, d, J=8.5Hz), 7. 24-7. 38 (2H, m), 7. 40-7. 55 (2H, m), 7.60-7.80 (2H, m), 8.55 (1H, dd, J=1.8, 4. 8Hz), 9.17 (1H, br) | B |
| | | MS m/z : 476 (M+H)⁺ | |
| A-3 | 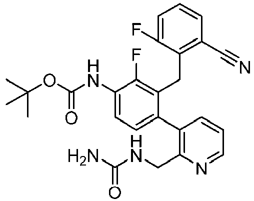 | ¹H-NMR (DMSO-d₆) δ ppm : 1.45 (9H, s), 3.68 (1H, dd, J=4.3, 16. 3Hz), 3.86-4.17 (3H, m), 5.66 (2H, br), 6.20-6.44 (1H, m), 6.95 (1H, d, J=8. 3Hz), 7.24-7.43 (3H, m), 7. 45-7. 55 (2H, m), 7. 58-7. 72 (1H, m), 8.55 (1H, dd, J=1.5, 4.8Hz), 9.09 (1H, br) | B |
| | | MS m/z : 494 (M+H)⁺ | |
| A-4 | 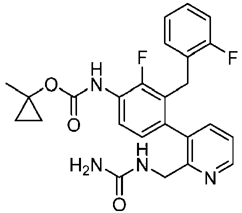 | ¹H-NMR (CDCl₃) δ ppm : 0.50-0. 75 (2H, m), 0. 80-1. 10 (2H, m), 1. 64 (3H, s), 3.66 (1H, d, J=15. 6Hz), 3.82 (1H, dd, J=4.4, 16. 7Hz), 3.90 (1H, d, J=15.6Hz), 4. 04-4. 18 (1H, m), 4. 32-4. 99 (2H, m), 5. 56-6. 22 (1H, m), 6. 67-6. 98 (5H, m), 7.02-7.21 (2H, m), 7.23-7.35 (1H, m), 8. 00-8. 20 (1H, m), 8.50 (1H, dd, J=1.6, 4. 9Hz) | A |
| | | MS m/z : 467 (M+H)⁺ | |
| A-5 | 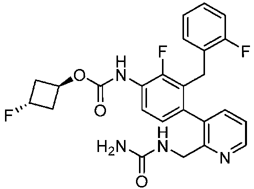 | ¹H-NMR (DMSO-d₆) δ ppm : 2. 28-2. 72 (4H, m), 3. 56-3. 71 (2H, m), 3. 80-4.08 (2H, m), 5.05-5.41 (2H, m), 5.65 (2H, br), 6.26-6.41 (1H, m), 6.67-6.75 (1H, m), 6. 88-7. 08 (3H, m), 7. 10-7. 22 (1H, m), 7. 30 (1H, dd, J=5. 0, 7. 5Hz), 7. 44 (1H, dd, J=1.6, 7.7Hz), 7.61-7.75 (1H, m), 8.56 (1H, dd, J=1. 8, 4. 8Hz), 9.58 (1H, br) | A |
| | | MS m/z : 485 (M+H)⁺ | |

**[Table 18]**

| Ex. No. | Str. | Phys. data | IC₅₀ |
|---|---|---|---|
| A-6 | 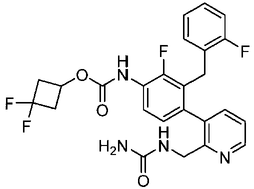 | ¹H-NMR (DMSO-d₆) δ ppm : 2.20-3.47 (4H, m), 3. 56-3. 70 (2H, m), 3.80-4.10 (2H, m), 4.80-5.10 (1H, m), 5. 65 (2H, br), 6.25-6.40 (1H, m), 6.65-6.75 (1H, m), 6.90-7.08 (3H, m), 7.10-7.25 (1H, m), 7. 30 (1H, dd, J=4.8, 7. 8Hz), 7.44 (1H, dd, J=1.8, 7.8Hz), 7.60-7.75 (1H, m), 8. 56 (1H, dd, J=1.8, 4.8Hz), 9.71 (1H, br) | A |
| | | MS m/z : 503 (M+H)⁺ | |
| A-7 | 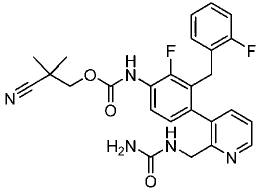 | ¹H-NMR (DMSO-d₆) δ ppm : 1.38 (6H, s), 3. 59-3. 70 (2H, m), 3. 88 (1H, d, J=15. 6Hz), 3.94-4.06 (1H, m), 4.12-4.18 (2H, m), 5. 65 (2H, br), 6. 27-6. 40 (1H, m), 6. 69-6. 77 (1H, m), 6.93-7.07 (3H, m), 7.13-7.21 (1H, m), 7.30 (1H, dd, J=4.8, 7. 5Hz), 7.44 (1H, dd, J=1.8, 7. 8Hz), 7.62-7.77 (1H, m), 8.56 (1H, dd, J=1.8, 4.8Hz), 9.75 (1H, br) | A |
| | | MS m/z : 494 (M+H)⁺ | |
| A-8 | 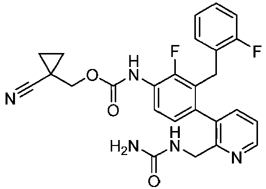 | ¹H-NMR (DMSO-d₆) δ ppm : 1. 10-1.26 (2H, m), 1. 29-1. 40 (2H, m), 3. 60-3. 68 (2H, m), 3.88 (1H, d, J=16. 1Hz), 3.94-4.22 (3H, m), 5.57-5.72 (2H, m), 6.28-6.40 (1H, m), 6.68-6.78 (1H, m), 6.94-7.08 (3H, m), 7.13-7.22 (1H, m), 7.30 (1H, dd, J=4.9, 7. 7Hz), 7.44 (1H, dd, J=1. 6, 7. 7Hz), 7.61-7.71 (1H, m), 8.56 (1H, dd, J=1. 6, 4. 9Hz), 9. 79 (1H, br) | A |
| | | MS m/z : 492 (M+H)⁺ | |
| A-9 | 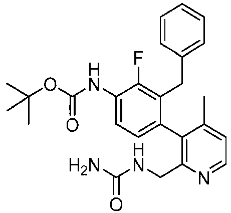 | MS m/z : 465 (M+H)⁺ | B |
| A-10 | 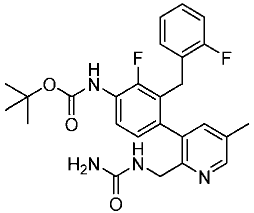 | MS m/z : 483 (M+H)⁺ | B |

**[Table 19]**

| Ex. No. | Str. | Phys. data | IC₅₀ |
|---|---|---|---|
| A-11 | 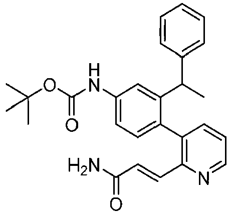 | MS m/z : 444 (M+H)⁺ | B |
| A-12 | 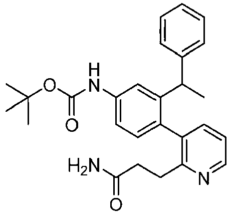 | MS m/z : 446 (M+H)⁺ | B |
| A-13 | 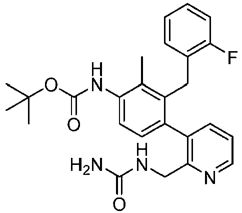 | MS m/z : 465 (M+H) ⁺ | A |
| A-14 | 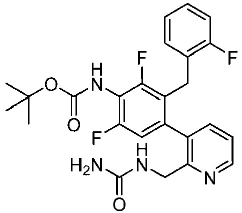 | MS m/z : 487 (M+H) ⁺ | A |
| A-15 | 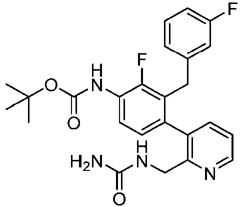 | MS m/z : 469 (M+H) ⁺ | B |

**[Table 20]**

| Ex. No. | Str. | Phys. data | IC₅₀ |
|---|---|---|---|
| A-16 | 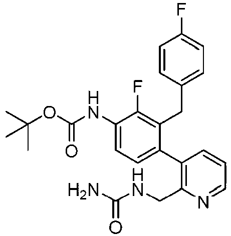 | MS m/z : 469 (M+H) ⁺ | A |
| A-17 | 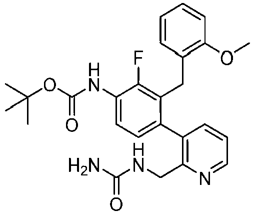 | MS m/z : 481 (M+H) ⁺ | B |
| A-18 | 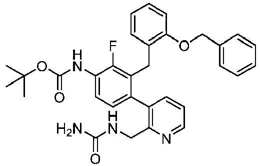 | MS m/z : 557 (M+H) ⁺ | B |
| A-19 | 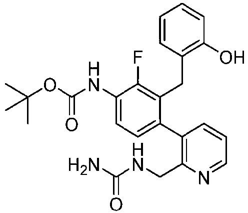 | MS m/z : 467 (M+H) ⁺ | A |
| A-20 | 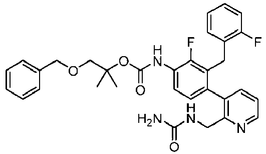 | MS m/z : 575 (M+H) ⁺ | A |

**[Table 21]**

| Ex. No. | Str. | Phys. data | IC₅₀ |
|---|---|---|---|
| A-21 | 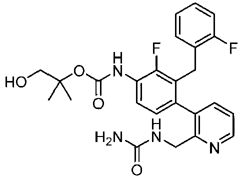 | MS m/z : 485 (M+H)⁺ | B |
| A-22 | 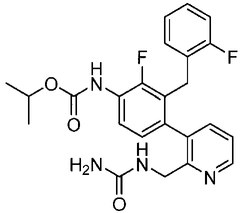 | MS m/z : 455 (M+H)⁺ | A |
| A-23 | 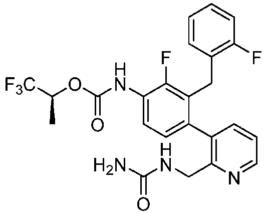 | MS m/z : 509 (M+H)⁺ | A |
| A-24 | 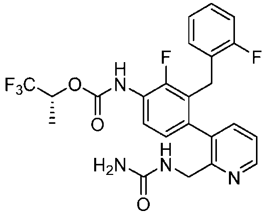 | MS m/z : 509 (M+H)⁺ | A |
| A-25 | 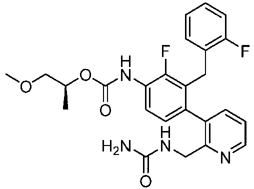 | MS m/z : 485 (M+H)⁺ | B |

**[Table 22]**

| Ex. No. | Str. | Phys. data | IC₅₀ |
|---|---|---|---|
| A-26 | 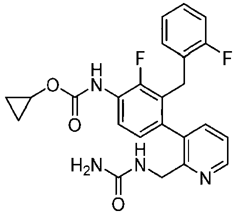 | ¹H-NMR (CDCl₃) δ ppm : 0.68-0.94 (4H, m), 3. 67 (1H, d, J=15. 7Hz), 3.81 (1H, dd, J=4. 1, 16.7Hz), 3.90 (1H, d, J=15. 7Hz), 4. 03-4. 27 (2H, m), 4. 59 (2H, br), 5.88 (1H, br), 6.67-6.78 (1H, m), 6. 79-6. 99 (4H, m), 7. 05-7. 35 (3H, m), 7.95-8.21 (1H, m), 8.51 (1H, dd, J=1.5, 4. 8Hz) | B |
| | | MS m/z : 453 (M+H)⁺ | |
| A-27 | 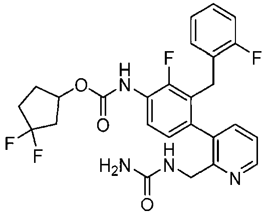 | MS m/z : 517 (M+H)⁺ | A |
| A-28 | 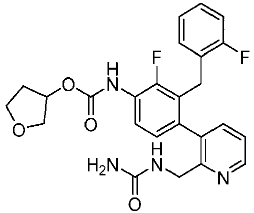 | MS m/z : 483 (M+H) ⁺ | B |
| A-29 | 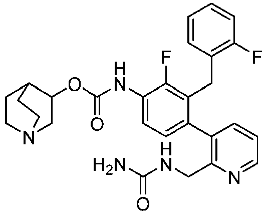 | MS m/z : 522 (M+H)⁺ | B |
| A-30 | 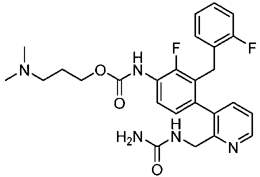 | MS m/z : 498 (M+H) ⁺ | B |

**[Table 23]**

| Ex. No. | Str. | Phys. data | IC₅₀ |
|---|---|---|---|
| A-31 | 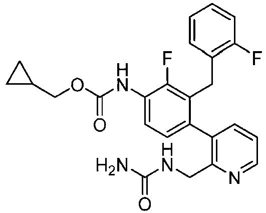 | MS m/z : 467 (M+H)⁺ | A |
| A-32 | 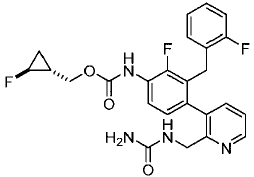 | ¹H-NMR (DMSO-d₆) δ ppm : 0.69-0. 80 (1H, m), 1. 05-1. 30 (1H, m), 1. 55-1. 75 (1H, m), 3.57-3.72 (2H, m), 3.81-4.09 (4H, m), 4.62-4.87 (1H, m), 5.65 (2H, br), 6. 28-6. 39 (1H, m), 6. 66-6. 77 (1H, m), 6.93-7.08 (3H, m), 7. 12-7. 22 (1H, m), 7.29 (1H, dd, J=4.8, 7. 5Hz), 7.43 (1H, dd, J=1.6, 7. 7Hz), 7.63-7.75 (1H, m), 8. 56 (1H, dd, J=1. 6, 4.9Hz), 9.59 (1H, br) | A |
| | | MS m/z : 485 (M+H)⁺ | |
| A-33 | 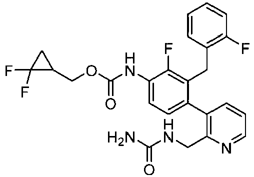 | ¹H-NMR (DMSO-d₆) δ ppm : 1.41-1.80 (2H, m), 2.05-2.25 (1H, m), 3.57-3.74 (2H, m), 3.87 (1H, d, J=15. 6Hz), 3.93-4.10 (2H, m), 4. 28-4. 41 (1H, m), 5.65 (2H, br), 6. 25-6. 42 (1H, m), 6. 66-6. 75 (1H, m), 6.92-7.08 (3H, m), 7.13-7.22 (1H, m), 7.29 (1H, dd, J=4.8, 7.8Hz), 7.40-7.48 (1H, m), 7.62-7.75 (1H, m), 8.56 (1H, dd, J=1. 6, 4. 9Hz), 9.66 (1H, br) | A |
| | | MS m/z : 503 (M+H)⁺ | |
| A-34 | 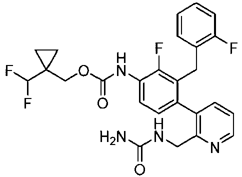 | MS m/z : 517 (M+H)⁺ | A |
| A-35 | 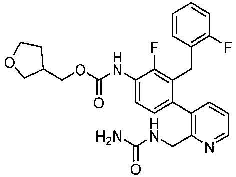 | MS m/z : 497 (M+H)⁺ | A |

The structural formula of Example A-32 in the table indicates a relative configuration.

**[Table 24]**

| Ex. No. | Str. | Phys. data | IC₅₀ |
|---|---|---|---|
| A-36 | 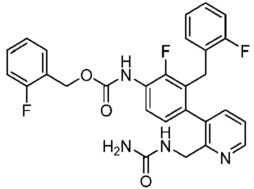 | MS m/z : 521 (M+H)⁺ | A |
| A-37 | 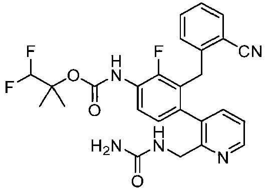 | ¹H-NMR (CDCl₃) δ ppm : 1. 52-1. 73 (6H, m), 3.81 (1H, dd, J=4. 5, 16. 6Hz), 3.94 (1H, d, J=16. 3Hz), 4.04-4.24 (2H, m), 4.56 (2H, br), 5.66-6.40 (2H, m), 6.91-7.09 (3H, m), 7. 12-7. 31 (3H, m), 7. 37-7.50 (2H, m), 7.97-8. 19 (1H, m), 8.53 (1H, dd, J=1.8, 4.8Hz) | B |
| | | MS m/z : 512 (M+H)⁺ | |
| A-38 | 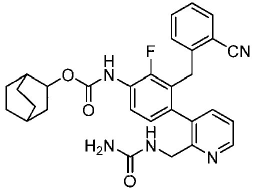 | MS m/z : 528 (M+H)⁺ | B |
| A-39 | 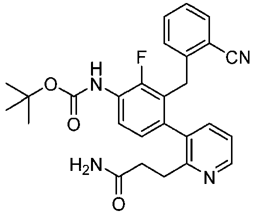 | MS m/z : 475 (M+H)⁺ | A |
| A-40 | 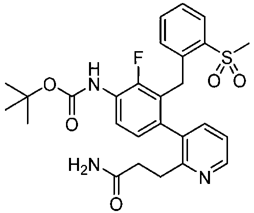 | MS m/z : 528 (M+H)⁺ | B |

**[Table 25]**

| Ex. No. | Str. | Phys. data | IC₅₀ |
|---|---|---|---|
| B-1 | 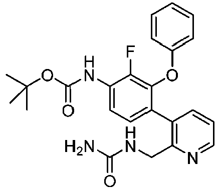 | ¹H-NMR (CDCl₃) δ ppm : 1.55 (9H, s), 4.21-4.85 (4H, m), 5.72-6.20 (1H, m), 6. 63-6. 74 (2H, m), 6. 75-6. 85 (1H, m), 6.87-7.08 (2H, m), 7.09-7.21 (3H, m), 7.43 (1H, dd, J=1.6, 7. 7Hz), 7.94-8. 18 (1H, m), 8.43 (1H, dd, J=1.6, 4.9Hz) | A |
| | | MS m/z : 453 (M+H)⁺ | |
| B-2 | 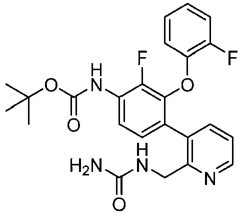 | ¹H-NMR (CDCl₃) δ ppm : 1. 55 (9H, s), 4.22-4.90 (4H, m), 5. 66-6. 17 (1H, m), 6. 60-6. 71 (1H, m), 6. 74-6. 83 (1H, m), 6.84-7.19 (5H, m), 7.45 (1H, dd, J=1. 8, 7.8Hz), 7.98-8.15 (1H, m), 8.45 (1H, dd, J=1.6, 4. 9Hz) | A |
| | | MS m/z : 471 (M+H)⁺ | |
| B-3 | 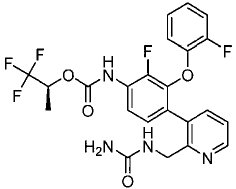 | ¹H-NMR (CDCl₃) δ ppm : 1.51 (3H, d, J=6.5Hz), 4.23-4.83 (4H, m), 5.28-5.44 (1H, m), 5. 75-6. 22 (1H, m), 6.59-6.74 (1H, m), 6.79-7.01 (3H, m), 7.04-7.22 (3H, m), 7.46 (1H, dd, J=1.8, 7.8Hz), 7. 92-8. 19 (1H, m), 8.46 (1H, dd, J=1. 8, 4. 8Hz) | A |
| | | MS m/z : 511 (M+H)⁺ | |
| B-4 | 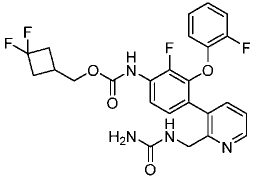 | ¹H-NMR (CDCl₃) δ ppm : 2. 30-2. 88 (5H, m), 4.23-4.77 (6H, m), 5. 74-6. 21 (1H, m), 6.59-6.73 (1H, m), 6.83-7.01 (4H, m), 7.07 (1H, dd, J=2.0, 8. 8 Hz), 7.11-7. 19 (1H, m), 7.46 (1H, dd, J=1. 6, 7.7Hz), 7.95-8.17 (1H, m), 8.46 (1H, dd, J=1.8, 4. 8Hz) | A |
| | | MS m/z : 519 (M+H)⁺ | |
| B-5 | 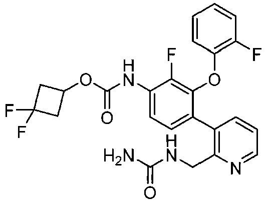 | ¹H-NMR (DMSO-d₆) δ ppm : 2.63-2.86 (2H, m), 3.01-3.21 (2H, m), 3.90-4.30 (2H, m), 4.87-5.05 (1H, m), 5.66 (2H, br), 6. 35-6.44 (1H, m), 6. 66-6. 78 (1H, m), 6.90-7.02 (2H, m), 7. 11-7.34 (3H, m), 7.59 (1H, dd, J=1.6, 7.7Hz), 7.66-7.76 (1H, m), 8.50 (1H, dd, J=1.8, 4.8Hz), 9.92 (1H, br) | B |
| | | MS m/z : 505 (M+H)⁺ | |

**[Table 26]**

| Ex. No. | Str. | Phys. data | IC₅₀ |
|---|---|---|---|
| B-6 | 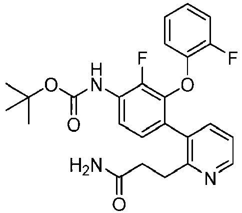 | MS m/z : 470 (M+H)⁺ | A |
| B-7 | 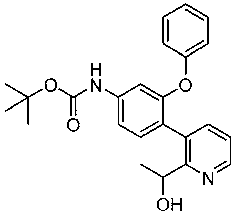 | MS m/z : 407 (M+H)⁺ | C |
| C-1 | 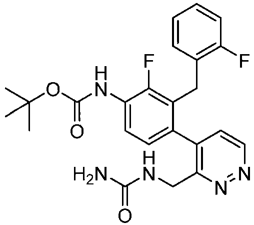 | ¹H-NMR (DMSO-d₆) δ ppm : 1.47 (9H, s), 3.65 (1H, d, J=16. 2Hz), 3.78 (1H, dd, J=4.9, 16. 7Hz), 3.89 (1H, d, J=16. 2Hz), 4.17 (1H, dd, J=5. 1, 16. 7Hz), 5.68 (2H, br), 6.57-6.66 (1H, m), 6.70-6.79 (1H, m), 6. 93-7. 09 (3H, m), 7. 13-7. 23 (1H, m), 7.38 (1H, d, J=5. OHz), 7. 64-7. 78 (1H, m), 9.14 (1H, d, J=5. 0Hz), 9.20 (1H, br) | A |
| | | MS m/z : 470 (M+H)⁺ | |
| C-2 | 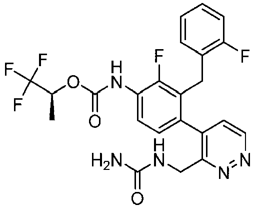 | ¹H-NMR (DMSO-d₆) δ ppm : 1.43 (3H, d, J=6. 5Hz), 3.67 (1H, d, J=16. 1Hz), 3.77 (1H, dd, J=5.1, 16. 7Hz), 3.91 (1H, d, J=16. 1Hz), 4.07-4.23 (1H, m), 5.36-5.56 (1H, m), 5.68 (2H, br), 6.54-6.68 (1H, m), 6.69-6.83 (1H, m), 6.92-7.05 (2H, m), 7.06-7.26 (2H, m), 7.39 (1H, dd, J=2.0, 5. 0Hz), 7. 59-7. 76 (1H, m), 9.16 (1H, d, J=5. 0Hz), 10.04 (1H, br) 0 | A |
| | | MS m/z : 510 (M+H)⁺ | |
| C-3 | 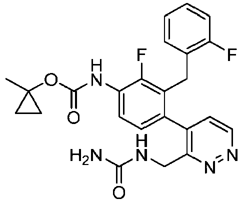 | ¹H-NMR (CDCl₃) δ ppm : 0.62-0. 80 (2H, m), 0. 85-1. 10 (2H, m), 1. 64 (3H, s), 3.65 (1H, d, J=15. 8Hz), 3.95 (1H, d, J=15.8Hz), 4.04 (1H, dd, J=4.4, 17. 2Hz), 4.35 (1H, dd, J=4.4, 17. 2Hz), 4.76 (2H, br), 6. 05-6. 69 (1H, m), 6. 71-7.21 (7H, m), 8.01-8.33 (1H, m), 9.04 (1H, d, J=4.8Hz) | B |
| | | MS m/z : 468 (M+H)⁺ | |

**[Table 27]**

| Ex. No. | Str. | Phys. data | IC₅₀ |
|---|---|---|---|
| C-4 | 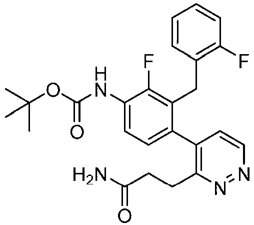 | ¹H-NMR (CDCl₃) δ ppm : 1. 55 (9H, s), 2. 60-2. 84 (3H, m), 2. 88-3. 04 (1H, m), 3.67 (1H, d, J=15. 8Hz), 3.96 (1H, d, J=15.8Hz), 5.28 (1H, br), 5.99 (1H, br), 6.71-6.80 (1H, m), 6. 81-6. 97 (4H, m), 6.99 (1H, d, J=5. 3Hz), 7. 07-7. 17 (1H, m), 8. 07-8. 22 (1H, m), 8.97 (1H, d, J=5.0Hz) | A |
| | | MS m/z : 469 (M+H)⁺ | |
| C-5 | 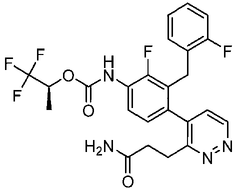 | ¹H-NMR (CDCl₃) δ ppm : 1. 45-1. 55 (3H, m), 2. 62-2. 86 (3H, m), 2. 89-3. 03 (1H, m), 3. 63-3. 75 (1H, m), 3. 97 (1H, d, J=15. 6Hz), 5.11-5.44 (2H, m), 5. 80-6. 11 (1H, m), 6. 70-6. 79 (1H, m), 6. 81-7. 05 (4H, m), 7. 08-7. 19 (2H, m), 8. 02-8. 25 (1H, m), 8.99 (1H, d, J=5.0Hz) | A |
| | | MS m/z : 509 (M+H)⁺ | |
| C-6 | 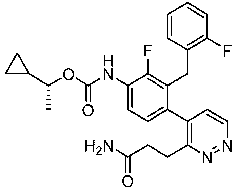 | ¹H-NMR (CDCl₃) δ ppm : 0.23-0. 36 (1H, m), 0.41-0.66 (3H, m), 0.98-1.11 (1H, m), 1.35-1.45 (3H, m), 2.56-3.21 (4H, m), 3.67 (1H, d, J=15.6Hz), 3.96 (1H, d, J=15.6Hz), 4.25-4.44 (1H, m), 5.01-5.56 (1H, m), 6.02 (1H, br), 6.64-7.22 (7H, m), 8.06-8.31 (1H, m), 8.98 (1H, d, J=4.8Hz) | A |
| | | MS m/z : 481 (M+H)⁺ | |
| C-7 | 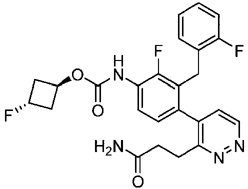 | ¹H-NMR (CDCl₃) δ ppm : 2.15-3.20 (8H, m), 3.68 (1H, d, J=15. 8Hz), 3.96 (1H, d, J=15. 8Hz), 5.10-5.50 (3H, m), 5.95 (1H, br), 6.65-7.06 (6H, m), 7.07-7.19 (1H, m), 8. 02-8. 22 (1H, m), 8.98 (1H, d, J=5.0Hz) | B |
| | | MS m/z : 485 (M+H)⁺ | |
| C-8 | 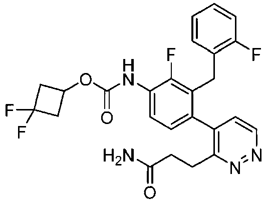 | ¹H-NMR (CDCl₃) δ ppm : 2.61-2.87 (5H, m), 2. 89-3. 20 (3H, m), 3. 68 (1H, d, J=15. 9Hz), 3.96 (1H, d, J=15. 9Hz), 4.94-5.52 (2H, m), 5.70-6.23 (1H, m), 6. 66-7. 21 (7H, m), 7.93-8.25 (1H, m), 8.98 (1H, d, J=4.8Hz) | A |
| | | MS m/z : 503 (M+H)⁺ | |

**[Table 28]**

| Ex. No. | Str. | Phys. data | IC₅₀ |
|---|---|---|---|
| C-9 | 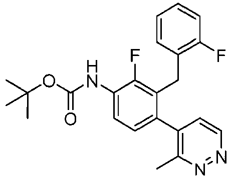 | MS m/z : 412 (M+H)⁺ | B |
| C-10 | 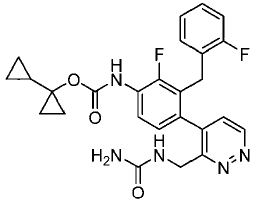 | MS m/z : 494 (M+H)⁺ | B |
| D-1 | 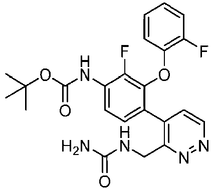 | ¹H-NMR (DMSO-d₆) δ ppm : 1.48 (9H, s), 4.36 (2H, d, J=5. 0Hz), 5.67 (2H, br), 6. 64-6. 80 (2H, m), 6. 94-7. 03 (2H, m), 7.13-7.22 (1H, m), 7.28 (1H, dd, J=1.5, 8. 5Hz), 7.53 (1H, d, J=5. 0Hz), 7.69-7.81 (1H, m), 9.13 (1H, d, J=5. 3Hz), 9.44 (1H, br) | B |
| | | MS m/z : 472 (M+H)⁺ | |
| D-2 | 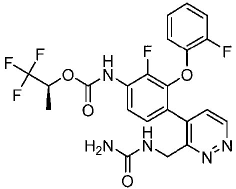 | ¹H-NMR (DMSO-d₆) δ ppm : 1.43 (3H, d, J=6.5Hz), 4.36 (2H, d, J=5.0Hz), 5.40-5.54 (1H, m), 5.67 (2H, br), 6.64-6.84 (2H, m), 6.93-7.07 (2H, m), 7.11-7.24 (1H, m), 7.35 (1H, dd, J=1.6, 8. 7Hz), 7.55 (1H, d, J=5.0Hz), 7.66-7.81 (1H, m), 9.14 (1H, d, J=5. 0Hz), 10.25 (1H, br) | B |
| | | MS m/z : 512 (M+H)⁺ | |
| D-3 | 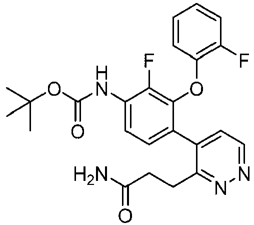 | ¹H-NMR (CDCl₃) δ ppm : 1. 55 (9H, s), 2. 71-2. 94 (2H, m), 3. 15-3. 25 (2H, m), 5.31 (1H, br), 6.01 (1H, br), 6.57-6.73 (1H, m), 6.78-7.11 (5H, m), 7.21 (1H, d, J=5.0Hz), 8. 05-8. 23 (1H, m), 8.97 (1H, d, J=5.0Hz) | A |
| | | MS m/z : 471 (M+H)⁺ | |

**[Table 29]**

| Ex. No. | Str. | Phys. data | IC₅₀ |
|---|---|---|---|
| D-4 | 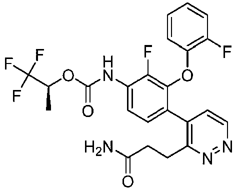 | ¹H-NMR (CDCl₃) δ ppm : 1.51 (3H, d, J=6.5Hz), 2.76-2.94 (2H, m), 3. 14-3. 27 (2H, m), 5.15-5.46 (2H, m), 5.95 (1H, br), 6.61-6.79 (1H, m), 6.85-7.17 (5H, m), 7.22 (1H, d, J=5.0Hz), 7.97-8.24 (1H, m), 8.99 (1H, d, J=5.0Hz) | A |
| | | MS m/z : 511 (M+H)⁺ | |
| E-1 | 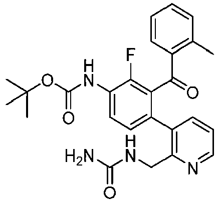 | MS m/z : 479 (M+H)⁺ | A |
| E-2 | 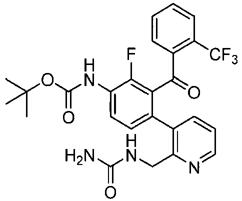 | MS m/z : 533 (M+H)⁺ | B |
| E-3 | 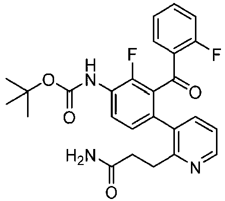 | MS m/z : 482 (M+H) ⁺ | A |
| E-4 | 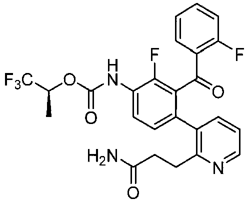 | ¹H-NMR (DMSO-d₆) δ ppm : 1.44 (3H, d, J=6.8Hz), 2.22-2.90 (4H, m), 5.40-5.53 (1H, m), 6.70 (1H, br), 7.09 (1H, dd, J=4.8, 7. 5Hz), 7. 18-7. 34 (5H, m), 7.43-7.52 (1H, m), 7.58-7.68 (1H, m), 7.79-7.95 (1H, m), 8.36 (1H, dd, J=1.8, 4.8Hz), 10.14 (1H, br) | A |
| | | MS m/z : 522 (M+H)⁺ | |

**[Table 30]**

| Ex. No. | Str. | Phys. data | IC₅₀ |
|---|---|---|---|
| E-5 | 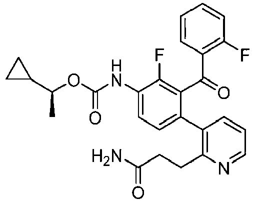 | ¹H-NMR (CDCl₃) δ ppm : 0. 23-0. 38 (1H, m), 0. 43-0. 67 (3H, m), 0.98-1.14 (1H, m), 1. 40 (3H, d, J=6. 3Hz), 2. 47-2. 76 (2H, m), 2. 80-3. 11 (2H, m), 4. 30-4. 43 (1H, m), 5. 24 (1H, br), 6. 50 (1H, br), 6.86-7.20 (5H, m), 7.34 (1H, dd, J=1. 6, 7. 7Hz), 7. 44-7. 61 (2H, m), 8. 25-8. 47 (2H, m) | A |
| | | MS m/z : 494 (M+H)⁺ | |
| E-6 | 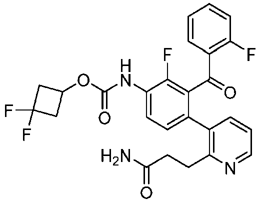 | ¹H-NMR (CDCl₃) δ ppm : 2.48-3.21 (8H, m), 4.98-5.13 (1H, m), 5.21 (1H, br), 6.43 (1H, br), 6. 91-7. 08 (3H, m), 7. 09-7.20 (2H, m), 7.34 (1H, dd, J=1.6, 7. 7Hz), 7. 44-7. 61 (2H, m), 8. 14-8. 35 (1H, m), 8. 37-8. 48 (1H, m) | B |
| | | MS m/z : 516 (M+H)⁺ | |
| E-7 | 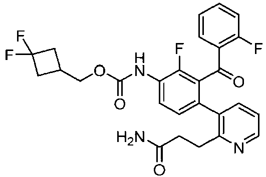 | ¹H-NMR (CDCl₃) δ ppm : 2. 31-3. 11 (9H, m), 4.28 (2H, d, J=6. 5Hz), 5.22 (1H, br), 6.45 (1H, br), 6. 89-7. 08 (3H, m), 7.09-7.20 (2H, m), 7.34 (1H, dd, J=1. 6, 7. 7Hz), 7. 44-7. 62 (2H, m), 8. 15-8. 50 (2H, m) | A |
| | | MS m/z : 530 (M+H)⁺ | |
| E-8 | 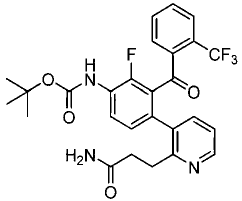 | MS m/z : 532 (M+H)⁺ | A |
| E-9 | 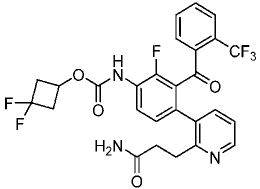 | MS m/z : 566 (M+H) ⁺ | B |

**[Table 31]**

| Ex. No. | Str. | Phys. data | IC₅₀ |
|---|---|---|---|
| F-1 | 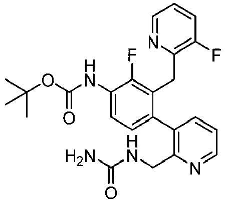 | MS m/z : 470 (M+H)⁺ | B |
| F-2 | 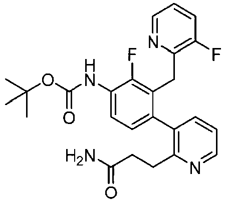 | MS m/z : 469 (M+H)⁺ | B |
| F-3 | 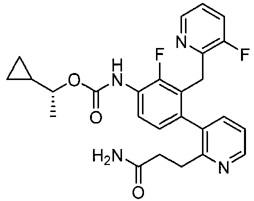 | ¹H-NMR (CDCl₃) δ ppm : 0.23-0. 36 (1H, m), 0.42-0. 68 (3H, m), 0.96-1. 11 (1H, m), 1. 33-1. 44 (3H, m), 2. 56 (2H, t, J=7.0Hz), 2.67-2.94 (2H, m), 3.90 (1H, d, J=17. 7Hz), 4.09 (1H, d, J=17. 7Hz), 4.26-4.43 (1H, m), 5.18 (1H, br), 6.58 (1H, br), 6.85-6.97 (2H, m), 7.04-7.13 (2H, m), 7.15-7.23 (1H, m), 7.34 (1H, dd, J=1. 6, 7. 7Hz), 8.04-8.17 (1H, m), 8.18-8.24 (1H, m), 8.48 (1H, dd, J=1.8, 4. 8Hz) | A |
| | | MS m/z : 481 (M+H)⁺ | |
| F-4 | 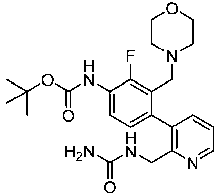 | MS m/z : 460 (M+H)⁺ | B |
| F-5 | 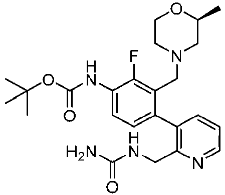 | MS m/z : 474 (M+H)⁺ | C |

**[Table 32]**

| Ex. No. | Str. | Phys. data | IC₅₀ |
|---|---|---|---|
| F-6 | 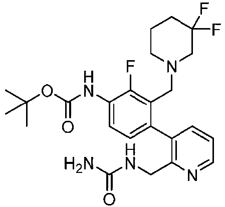 | MS m/z : 494 (M+H)⁺ | B |
| F-7 | 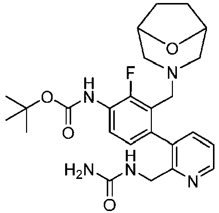 | MS m/z : 486 (M+H)⁺ | B |
| F-8 | 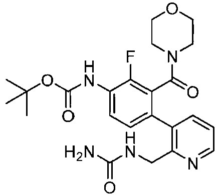 | MS m/z : 474 (M+H)⁺ | C |
| F-9 | 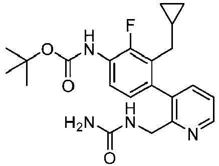 | MS m/z : 415 (M+H)⁺ | B |
| G-1 | 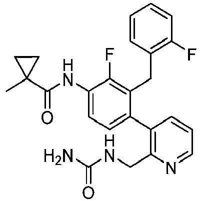 | MS m/z : 451 (M+H)⁺ | B |

**[Table 33]**

| Ex. No. | Str. | Phys. data | IC₅₀ |
|---|---|---|---|
| G-2 | 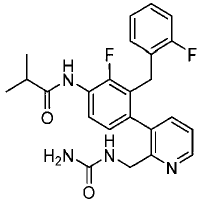 | MS m/z : 439 (M+H)⁺ | B |
| G-3 | 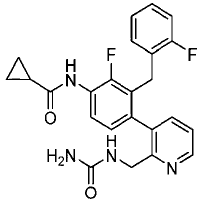 | MS m/z : 437 (M+H)⁺ | B |
| G-4 | 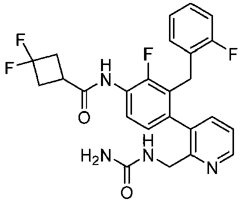 | MS m/z : 487 (M+H)⁺ | A |
| G-5 | 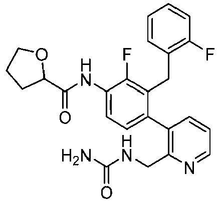 | MS m/z : 467 (M+H)⁺ | B |
| G-6 | 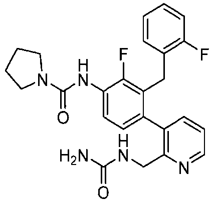 | MS m/z : 466 (M+H)⁺ | B |

**[Table 34]**

| Ex. No. | Str. | Phys. data | IC₅₀ |
|---|---|---|---|
| G-7 | 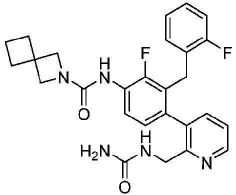 | MS m/z : 492 (M+H)⁺ | A |
| G-8 | 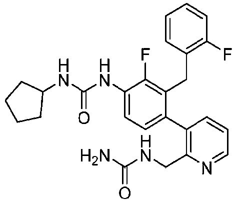 | MS m/z : 480 (M+H) ⁺ | A |
| G-9 | 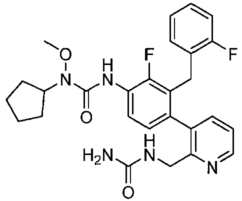 | MS m/z : 510 (M+H)⁺ | B |
| H-1 | 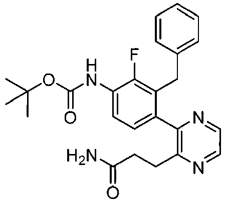 | MS m/z : 451 (M+H)⁺ | B |

### Test Example 1

### Measurement of antagonist activity using TSH-induced cAMP generation in human TSHR-stably expressing CHO cells as an indicator

The gene sequence (reference number: NM_000369.2) of a human TSHR was inserted into multiple cloning sites of pcDNA3.1 (+). Prepared plasmid vectors were introduced into CHO cells using a lipofection method, and human TSHR-stably expressing CHO cells were established. Obtained cells were seeded in a 96-well poly-D-lysine-coated plate at 5 × 10⁴ cells/well and cultured in an F12 culture medium containing 10%FBS, 400 µg/mL of G418, 50 U/mL of penicillin, and 50 µg/mL of streptomycin under conditions of 37°C and 5% CO₂ for one day. After the culture medium was removed, the cells were washed twice with 100 µL of an assay buffer solution (Hanks' Balanced Salt Solution containing 20 mM HEPES and 1 mM IBMX) per well. 30 µL of a test compound-containing assay buffer solution was added to the well and incubated at room temperature for 15 minutes. Next, 30 µL of an assay buffer solution containing human TSH (final concentration: 50 ng/mL, R&D Systems, Inc.) was added thereto and incubated at 37°C for one hour. A supernatant was removed, a Lysis and Detection Buffer 2(Cisbio) was added thereto, and the mixture was incubated at room temperature for one hour to prepare a cell lysate. According to the instruction manual of a cAMP Gs HiRange kit (Cisbio), the cell lysate, d2-labeled cAMP, and anti-cAMP Europium Cryptate labeled antibodies (Cisbio) were reacted together in a 384-well white microplate. Next, fluorescence intensity ratios (measurement wavelengths: 665 nm/620 nm) were measured using a multi-plate reader (PHERAstar FSX, BMG LABTECH, Japan). The fluorescence intensity ratio of each sample was converted to a cAMP content using a standard curve. The cAMP content was converted as a percentage of the control value to calculate a cAMP generation rate. The cAMP generation rates were plotted against the test compound concentrations using Prism (Graph Pad Software Inc.) to calculate IC₅₀ values. The IC₅₀'s of the individual test compounds are shown in the above tables. In the tables, IC₅₀ < 0.3 µM was denoted as A, and 0.3 µM ≤ IC₅₀ < 30 µM was denoted as B. Cases where the IC₅₀ values were not calculated for test compounds from which activity was recognized are denoted as C.

As shown in the above tables, it has been clarified that the compound of the present invention has human TSHR antagonist activity.

### [Industrial Applicability]

The compound of the present invention or a pharmacologically acceptable salt thereof has TSHR antagonist activity and is thus useful as a therapeutic agent for thyroid-related diseases.

## Claims

1. A compound represented by formula (I): 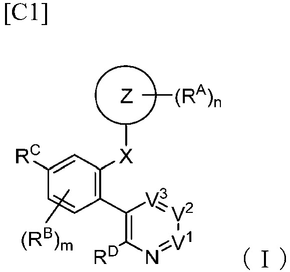 [wherein
a ring Z is a C₆₋₁₀ aryl, a 5- or 6-membered heteroaryl, a C₃₋₈ cycloalkyl, or a 3- to 8-membered heterocycloalkyl;
V¹ is =CR^{v1}-, or =N-;
V² is =CR^{v2}-, or =N-; and
V³ is =CR^{v3}-, or =N-;
wherein R^{v1}, R^{v2}, and R^{v3} are each independently a hydrogen atom, a C₁₋₆ alkyl, or a hydroxy C₁₋₆ alkyl;
X is -CR^{x}R^{x'}-, -O-, or -C(=O)-;
R^{x} and R^{x'} are each independently a hydrogen atom or a C₁₋₆ alkyl;
m and n are each independently an integer of 0 to 2;
R^{A} is a halogen atom, a cyano group, a hydroxyl group, a C₁₋₆ alkyl, a halo C₁₋₆ alkyl, a C₁₋₆ alkoxy, a halo C₁₋₆ alkoxy, a C₆₋₁₀ aryl C₁₋₆ alkoxy, or a C₁₋₆ alkylsulfonyl;
in a case where n is 2, the individual R^{A}'s are the same or different from each other;
R^{B} is a halogen atom or a C₁₋₆ alkyl;
in a case where m is 2, the individual R^{B}'s are the same or different from each other;
R^{C} is -NHC(O)OR^{c1}, -NHC(O)R^{c2}, -NHC(O)NR^{c2}R^{c2'}, or -NHC(O)NR^{c2}(OR^{c2'});
R^{c1} is a group selected from the group consisting of the following (a) to (g):
(a) a C₁₋₆ alkyl unsubstituted or substituted with 1 to 6 groups selected from a substituent group A,
(b) a C₃₋₈ cycloalkyl unsubstituted or substituted with 1 to 6 groups selected from the substituent group A,
(c) a C₃₋₈ cycloalkyl C₁₋₆ alkyl unsubstituted or substituted with 1 to 6 groups selected from the substituent group A,
(d) a C₆₋₁₀ aryl C₁₋₆ alkyl unsubstituted or substituted with 1 to 3 groups selected from the substituent group A,
(e) a 3- to 8-membered heterocycloalkyl unsubstituted or substituted with 1 to 3 groups selected from the substituent group A,
(f) a 3- to 8-membered heterocycloalkyl C₁₋₆ alkyl unsubstituted or substituted with 1 to 3 groups selected from the substituent group A, and
(g) a 5- or 6-membered heteroaryl C₁₋₆ alkyl unsubstituted or substituted with 1 to 3 groups selected from the substituent group A;
the substituent group A is the group consisting of a halogen atom, a cyano group, a hydroxyl group, -N(C₁₋₆ alkyl)₂, a C₁₋₆ alkyl, a halo C₁₋₆ alkyl, a C₁₋₆ alkoxy, a C₆₋₁₀ aryl C₁₋₆ alkoxy, and a C₃₋₈ cycloalkyl;
in the case of being substituted with 2 or more groups selected from the substituent group A, the individual groups may be the same or different from each other;
R^{c2} and R^{c2'} are each independently a group selected from the group consisting of the following (a) to (f):
(a) a hydrogen atom,
(b) a C₁₋₆ alkyl unsubstituted or substituted with 1 to 3 groups selected from a substituent group B,
(c) a C₃₋₈ cycloalkyl unsubstituted or substituted with 1 to 3 groups selected from the substituent group B,
(d) a C₃₋₈ cycloalkyl C₁₋₆ alkyl unsubstituted or substituted with 1 to 3 groups selected from the substituent group B,
(e) a 3- to 8-membered heterocycloalkyl unsubstituted or substituted with 1 to 3 groups selected from the substituent group B, and
(f) the following group unsubstituted or substituted with 1 to 3 groups selected from the substituent group B: 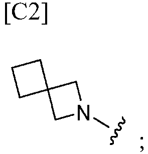
the substituent group B is the group consisting of a halogen atom, a cyano group, a C₁₋₆ alkyl, a halo C₁₋₆ alkyl, a C₁₋₆ alkoxy, and a C₃₋₈ cycloalkyl;
in the case of being substituted with 2 or 3 groups selected from the substituent group B, the individual groups may be the same or different from each other;
R^{D} is a group selected from the group consisting of the following (a) to (e):
(a) a C₁₋₆ alkyl,
(b) a hydroxy C₁₋₆ alkyl, 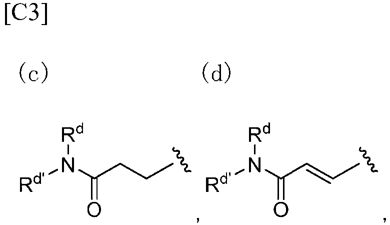 and 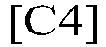 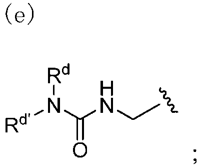 and
R^{d} and R^{d'} are each independently a hydrogen atom or a C₁₋₆ alkyl], or
a pharmacologically acceptable salt thereof.

2. The compound according to Claim 1, wherein
V² is =CR^{v2}-;
R^{v1}, R^{v2}, and R^{v3} are each independently a hydrogen atom or a C₁₋₆ alkyl;
R^{A} is a halogen atom, a cyano group, a hydroxyl group, a C₁₋₆ alkyl, a halo C₁₋₆ alkyl, a C₁₋₆ alkoxy, a C₆₋₁₀ aryl C₁₋₆ alkoxy, or a C₁₋₆ alkylsulfonyl;
R^{c1} is a group selected from the group consisting of the following (a) to (f):
(a) a C₁₋₆ alkyl unsubstituted or substituted with 1 to 6 groups selected from the substituent group A,
(b) a C₃₋₈ cycloalkyl unsubstituted or substituted with 1 to 6 groups selected from the substituent group A,
(c) a C₃₋₈ cycloalkyl C₁₋₆ alkyl unsubstituted or substituted with 1 to 6 groups selected from the substituent group A,
(d) a C₆₋₁₀ aryl C₁₋₆ alkyl unsubstituted or substituted with 1 to 3 groups selected from the substituent group A,
(e) a 3- to 8-membered heterocycloalkyl unsubstituted or substituted with 1 to 3 groups selected from the substituent group A, and
(f) a 3- to 8-membered heterocycloalkyl C₁₋₆ alkyl unsubstituted or substituted with 1 to 3 groups selected from the substituent group A;
the substituent group A has the same meaning as in Claim 1;
R^{c2} and R^{c2'} are each independently a group selected from the group consisting of the following (a) to (e):
(a) a hydrogen atom,
(b) a C₁₋₆ alkyl unsubstituted or substituted with 1 to 3 groups selected from a substituent group B,
(c) a C₃₋₈ cycloalkyl unsubstituted or substituted with 1 to 3 groups selected from the substituent group B,
(d) a 3- to 8-membered heterocycloalkyl unsubstituted or substituted with 1 to 3 groups selected from the substituent group B, and
(e) the following group unsubstituted or substituted with 1 to 3 groups selected from the substituent group B: 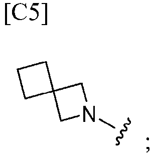
the substituent group B is the group consisting of a halogen atom and a C₁₋₆ alkyl; and
R^{d} and R^{d'} are hydrogen atoms, or a pharmacologically acceptable salt thereof.

3. The compound according to Claim 2, wherein
the ring Z is a C₆₋₁₀ aryl or a 5- or 6-membered heteroaryl;
V¹ is =CH- or =N-;
V² and V³ are =CH-;
R^{x} and R^{x'} are hydrogen atoms;
m is 1;
R^{B} is a halogen atom;
R^{C} is -NHC(O)OR^{c1};
R^{c1} is a group selected from the group consisting of the following (a) to (e):
(a) a C₁₋₆ alkyl unsubstituted or substituted with 1 to 3 groups selected from the substituent group A,
(b) a C₃₋₈ cycloalkyl unsubstituted or substituted with 1 to 3 groups selected from the substituent group A,
(c) a C₃₋₈ cycloalkyl C₁₋₆ alkyl unsubstituted or substituted with 1 to 3 groups selected from the substituent group A,
(d) a C₆₋₁₀ aryl C₁₋₆ alkyl unsubstituted or substituted with 1 to 3 groups selected from the substituent group A, and
(e) a 3- to 8-membered heterocycloalkyl unsubstituted or substituted with 1 to 3 groups selected from the substituent group A;
the substituent group A has the same meaning as in Claim 1;
R^{D} is a group represented by the following (a) or (b): 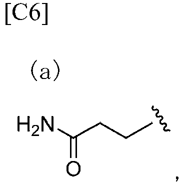 or 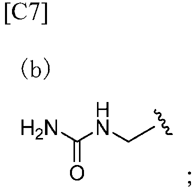
or a pharmacologically acceptable salt thereof.

4. A compound represented by formula (II): 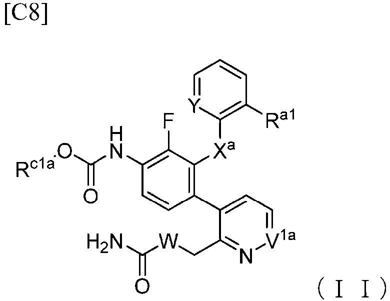 [wherein
V^{1a} is =CH- or =N-;
W is -CH₂- or -NH-;
X^{a} is -CH₂-, -O-, or -C(=O)-;
Y is =CR^{a2}- or =N-;
R^{a1} and R^{a2} are each independently a hydrogen atom, a halogen atom, a cyano group, a hydroxyl group, a C₁₋₆ alkyl, a halo C₁₋₆ alkyl, a C₁₋₆ alkoxy, a C₆₋₁₀ aryl C₁₋₆ alkoxy, or a C₁₋₆ alkylsulfonyl;
R^{c1a} is a group selected from the group consisting of the following (a) to (e):
(a) a C₁₋₆ alkyl unsubstituted or substituted with 1 to 3 groups selected from a substituent group A^{a},
(b) a C₃₋₈ cycloalkyl unsubstituted or substituted with 1 to 3 groups selected from the substituent group A^{a},
(c) a C₃₋₈ cycloalkyl C₁₋₆ alkyl unsubstituted or substituted with 1 to 3 groups selected from the substituent group A^{a},
(d) a C₆₋₁₀ aryl C₁₋₆ alkyl unsubstituted or substituted with 1 to 3 groups selected from the substituent group A^{a}, and
(e) a 3- to 8-membered heterocycloalkyl unsubstituted or substituted with 1 to 3 groups selected from the substituent group A^{a};
the substituent group A^{a} is a group consisting of a halogen atom, a cyano group, a hydroxyl group, -N(C₁₋₆ alkyl)₂, a C₁₋₆ alkyl, a halo C₁₋₆ alkyl, a C₁₋₆ alkoxy, a C₆₋₁₀ aryl C₁₋₆ alkoxy, and a C₃₋₈ cycloalkyl; and
in the case of being substituted with 2 or 3 groups selected from the substituent group A^{a}, the individual groups may be the same or different from each other], or
a pharmacologically acceptable salt thereof.

5. A compound represented by formula (III): 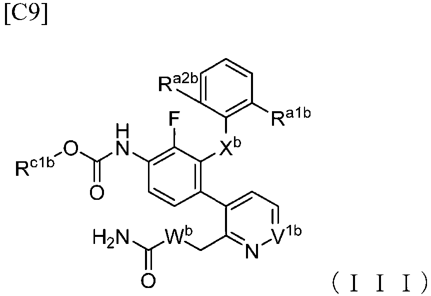 [wherein
V^{1b} is =CH- or =N-;
W^{b} is -CH₂- or -NH-;
X^{b} is -CH₂- or -O-;
R^{a1b} and R^{a2b} are each independently a hydrogen atom, a halogen atom, a cyano group, a hydroxyl group, a C₁₋₆ alkyl, a halo C₁₋₆ alkyl, a C₁₋₆ alkoxy, a C₆₋₁₀ aryl C₁₋₆ alkoxy, or a C₁₋₆ alkylsulfonyl;
R^{c1b} is a group selected from the group consisting of the following (a) to (e):
(a) a C₁₋₆ alkyl unsubstituted or substituted with 1 to 3 groups selected from a substituent group A^{b},
(b) a C₃₋₈ cycloalkyl unsubstituted or substituted with 1 to 3 groups selected from the substituent group A^{b},
(c) a C₃₋₈ cycloalkyl C₁₋₆ alkyl unsubstituted or substituted with 1 to 3 groups selected from the substituent group A^{b},
(d) a C₆₋₁₀ aryl C₁₋₆ alkyl unsubstituted or substituted with 1 to 3 groups selected from the substituent group A^{b}, and
(e) a 3- to 8-membered heterocycloalkyl unsubstituted or substituted with 1 to 3 groups selected from the substituent group A^{b};
the substituent group A^{b} is a group consisting of a halogen atom, a cyano group, a hydroxyl group, -N(C₁₋₆ alkyl)₂, a C₁₋₆ alkyl, a halo C₁₋₆ alkyl, a C₁₋₆ alkoxy, a C₆₋₁₀ aryl C₁₋₆ alkoxy, and a C₃₋₈ cycloalkyl; and
in the case of being substituted with 2 or 3 groups selected from the substituent group A^{b}, the individual groups may be the same or different from each other], or
a pharmacologically acceptable salt thereof.

6. The compound according to Claim 5, wherein
R^{a1b} and R^{a2b} are each independently a hydrogen atom, a halogen atom, or a cyano group;
R^{c1b} is a group selected from the group consisting of the following (a) to (c):
(a) a C₁₋₆ alkyl unsubstituted or substituted with 1 to 3 groups selected from the substituent group A^{b},
(b) a C₃₋₈ cycloalkyl unsubstituted or substituted with 1 to 3 groups selected from the substituent group A^{b}, and
(c) a C₃₋₈ cycloalkyl C₁₋₆ alkyl unsubstituted or substituted with 1 to 3 groups selected from the substituent group A^{b}; and
the substituent group A^{b} is a halogen atom, a cyano group, a C₁₋₆ alkyl, or a halo C₁₋₆ alkyl, or a pharmacologically acceptable salt thereof.

7. A compound selected from the group consisting of the following compounds: 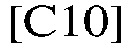 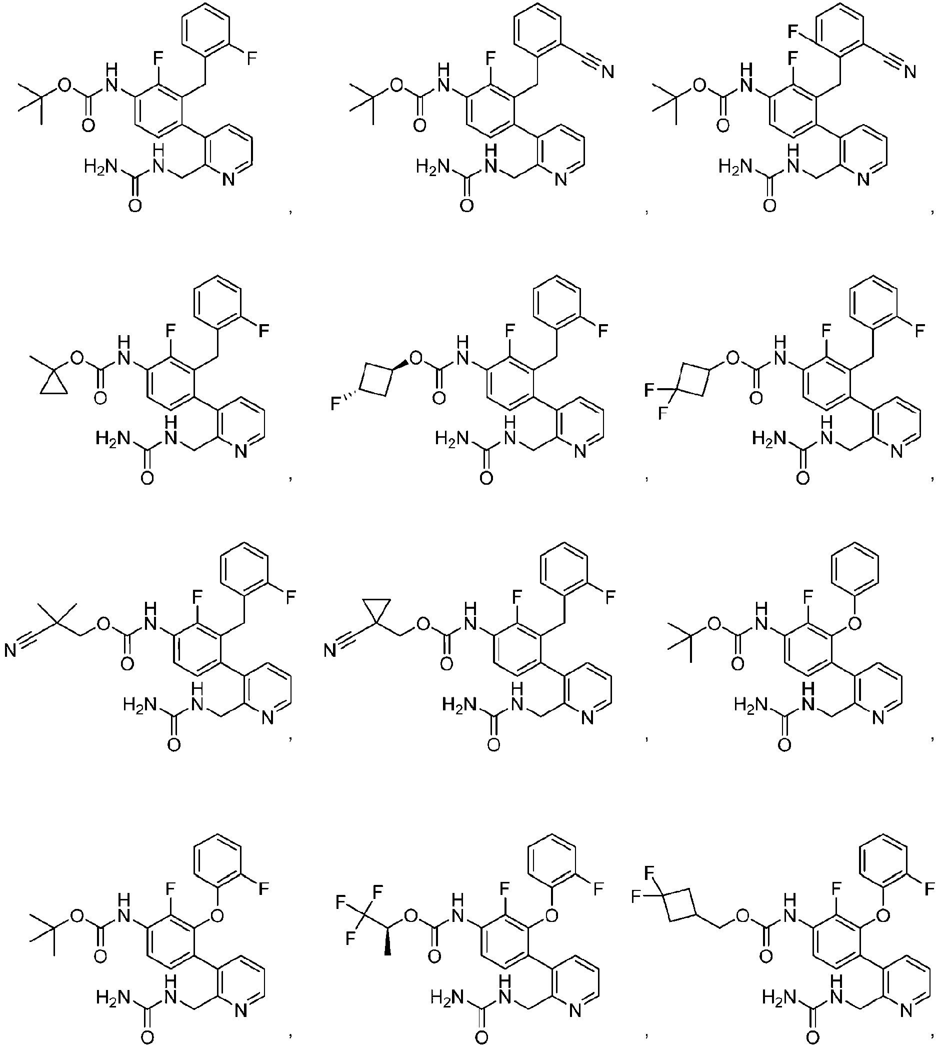 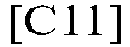 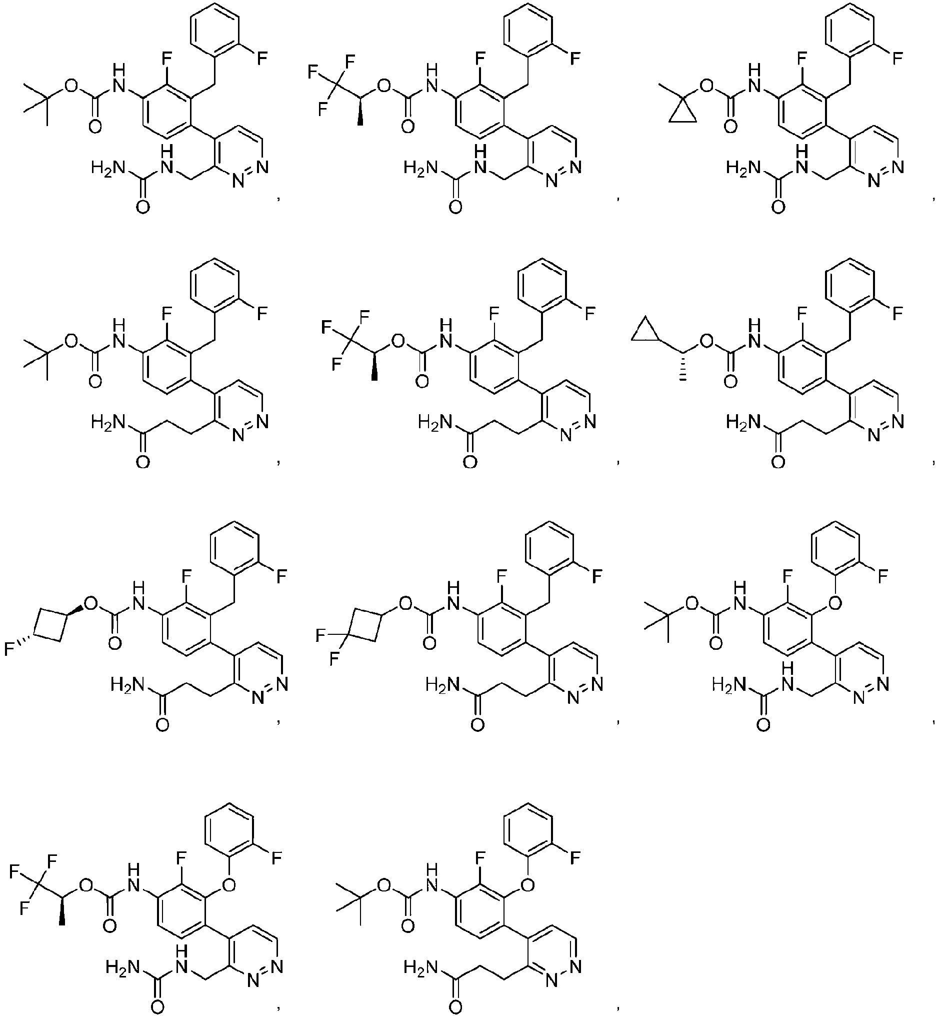 and 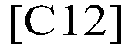 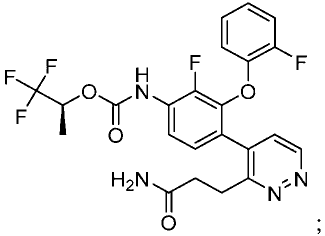 or a pharmacologically acceptable salt thereof.

8. A pharmaceutical composition comprising
the compound according to any one of Claims 1 to 7 or a pharmacologically acceptable salt thereof, and
a pharmaceutical additive.

9. The pharmaceutical composition according to Claim 8, wherein the composition is a pharmaceutical composition for treating thyroid-related diseases.

10. The pharmaceutical composition according to Claim 9, wherein the thyroid-related diseases are hyperthyroidism, Graves' disease, or thyroid eye disease.
